# FASCICULE DE BREVET EUROPEEN

(11) **EP 1 037 916 B1**
(45) Date de publication et mention de la délivrance du brevet: **26.05.2010**
(21) Numéro de dépôt: 98958322.4
(22) Date de dépôt: 03.12.1998
(51) Int. Cl.: C07K 14/015, C12N 15/35, C12N 15/64, C12N 15/11, C12Q 1/68, C07K 16/08, G01N 33/53, A61K 39/12

(54) **ERYTHROVIRUS ET SES APPLICATIONS**
ERYTHROVIRUS UND IHRE VERWENDUNG
ERYTHROVIRUS AND ITS APPLICATIONS

(30) Priorité: 03.12.1997 FR 9715197
(43) Date de publication de la demande: 27.09.2000
(73) Titulaire: ASSISTANCE PUBLIQUE, HOPITAUX DE PARIS, 75004 Paris (FR)
(72) Inventeur: NGUYEN, Quang, Tri, F-94200 Ivry sur Seine (FR); GARBARG-CHENON, Antoine, F-75017 Paris (FR); AUGUSTE, Véronique, F-75020 Paris (FR)
(74) Mandataire: Vialle-Presles, Marie José
(86) Numéro de dépôt international: PCT/FR1998/002615
(87) Numéro de publication internationale: WO 1999/028439

(56) Documents cités:
- WO-A-96/09391
- DE-A- 4 003 826
- R.O. SHADE ET AL.,: "Nucleotide sequence and genome organization of human Parvovirus B19 isolated from the serum of a child during aplastic crisis" JOURNAL OF VIROLOGY, vol. 58, no. 3, 1986, pages 921-936, XP002005737 Washington, DC, US cité dans la demande
- DATABASE EMBL NUCLEOTIDE AND PROTEIN SEQUENCE, - 8 octobre 1997 XP002079558 Hinxton, GB -& DATABASE WPI Week 9532 Derwent Publications Ltd., London, GB; AN 95-242756 XP002079561 & JP 07 147986 A (DENKA SEIKEN KK, DENKI KAGAKU KOGYO KK), 13 juin 1995
- A. HEMAUER ET AL.,: "Sequence variability among different parvovirus B19 isolates" JOURNAL OF GENERAL VIROLOGY, vol. 77, 1996, pages 1781-1785, XP002079559 Reading, GB
- K.E HICKS ET AL.,: "Sequence analysis of a parvovirus B19 isolate and baculovirus expression of the non-structural proteins" ARCHIVES OF VIROLOGY, vol. 141, 1996, pages 1319-1327, XP002079560
- NGUYEN QT ET AL., : "Detection of an erythrovirus sequence distinct from B19 in a child with acute anaemia (letter)" LANCET, vol. 352, no. 9139, 7 novembre 1998, page p1524 XP002102846

## Description

La présente invention est relative à des séquences nucléiques issues d'un érythrovirus humain, à leurs fragments ainsi qu'à leurs applications, en tant que réactif de diagnostic et en tant qu'agent immunogène.

Les études séro-épidémiologiques montrent que l'infection par le parvovirus B19, récemment renommé érythrovirus B19, est communément et largement répandue dans le monde entier.

En Europe, la séroprévalence pour l'érythrovirus B19 est d'environ 10 % chez les sujets de moins de 5 ans, d'environ 50 % pour les sujets de plus de 20 ans et supérieure à 90% chez les personnes âgées.

Le taux élevé de séroprévalence suggère que l'érythrovirus B19 est très contagieux. Lors d'épidémies, le taux de transmission à des sujets en contact rapproché est de 10 à 60 %, la voie de transmission étant principalement aérienne (sécrétions respiratoires).

L'érythrovirus B19 est un virus spécifiquement humain. L'infection aiguë entraîne communément une éruption cutanée maculo-papuleuse bénigne chez l'enfant (mégalérythème épidermique ou 5^{ème} maladie). Des arthralgies peuvent accompagner l'éruption, et exceptionnellement devenir chroniques.

Une crise érythroblastique aiguë transitoire survient habituellement, chez les patients déjà porteurs d'une anémie hémolytique chronique (drépanocytose, thalassémie, déficit en pyruvate kinase...), entraînant une anémie aiguë arégénérative transitoire.

La primo-infection aiguë par l'érythrovirus B19 est particulièrement dangereuse chez la femme enceinte avec un risque de transmission au foetus estimé à 30 %. Le risque de mort foetale est estimé entre 5 et 9 % par anémie, insuffisance hépatique, insuffisance cardiaque et anasarque foeto-placentaire.

Les infections chroniques à érythrovirus B19 se rencontrent essentiellement chez les sujets immunodéprimés (leucémie myéloïde chronique, déficit immunitaire humoral et cellulaire, greffés d'organe ou de moelle, malades du SIDA).

Chez les patients VIH-1 séropositifs, l'infection chronique par érythrovirus B19 est responsable d'anémie chronique, mais peut également agir sur les autres lignées (neutropénie et surtout thrombopénie). L'absence de réponse immunitaire humorale suffisante chez ces patients permet l'installation d'une érythrovirémie chronique et explique à la fois l'érythroblastopénie chronique et l'absence des autres symptômes tels que le rash ou les arthralgies.

L'érythrovirus B19 est un virus à génome ADN à simple brin d'environ 5,4 kbases ; c'est le seul érythrovirus répertorié à ce jour ; toutes les souches qui ont été séquencées et qui ont fait l'objet d'une publication dans les banques de séquences (GenBank ou EMBL) présentent une faible variabilité génétique, (98 % de similitude en séquence nucléotidique sur l'ensemble du génome et 96 % de similitude sur la région VP1) (R.O. SHADE, J. Virol., 1986, 58, 3, 921-936, B19-AU).

Le diagnostic virologique des infections à érythrovirus B19 repose essentiellement sur la détection du génome viral, dans la mesure où la culture n'est pas réalisable en routine.

Pour les infections aiguës, à érythrovirus B19 (primo-infections), cette détection peut se faire par amplification génique (PCR), mais aussi par hybridation (*dot*-*blot*), compte tenu du titre viral, habituellement très élevé lors des primo-infections (jusqu'à 10¹⁴/ml de sérum) ; toutefois, le titre viral est beaucoup plus faible lors des infections chroniques et seule une méthode de détection par amplification génique est envisageable.

Ces techniques de détection sont tributaires de la variabilité génétique du virus recherché ; les réactifs, préparés à partir des séquences d'érythrovirus B 19 connues, ne permettent pas de détecter les infections à érythrovirus variant, ni par amplification génique, ni par le sérodiagnostic B 19.

En effet, les tests sérodiagnostics existants sont spécifiques de l'érythrovirus B19 (Demande Internationale PCT WO 91/12269 ; Demande Internationale PCT WO 96/09391 (IDEIA^{®} Parvovirus B 19 IgG et IgM, DAKO ; Parvovirus B19 IgG et IgM *Enzyme Immunoassay,* BIOTRIN)).

En conséquence, les techniques de détection précisées ci-dessus risquent d'aboutir à des résultats négatifs tant au niveau nucléique qu'en ce qui concerne la réponse en anticorps.

La mise en évidence et la prise en compte de nouveaux variants sont importantes pour mettre au point :
- des réactifs de détection et de diagnostic des infections à érythrovirus humain (sérodiagnostic, PCR, hybridation), suffisamment sensibles et spécifiques, c'est-à-dire ne conduisant pas à des résultats faussement négatifs ou faussement positifs,
- des compositions aptes à protéger vis-à-vis de l'ensemble des infections à érythrovirus (vaccins) et
- des compositions aptes à traiter une infection à érythrovirus variant (sérothérapie, anticorps monoclonaux).

Les Inventeurs se sont donc donné pour but de pourvoir à des séquences issues d'érythrovirus, aptes à permettre la détection d'un érythrovirus variant (dénommé érythrovirus de type V9), c'est-à-dire génétiquement éloigné de l'érythrovirus B 19.

La présente invention a pour objet un érythrovirus variant par rapport à l'érythrovirus de type B19, dénommé érythrovirus de type V9, **caractérisé en ce qu**'il présente une divergence génétique < ou égale à 6 % avec la séquence SEQ ID NO:1, en ce que son génome s'hybride spécifiquement en conditions stringentes avec l'une des séquences SEQ ID NO:45 à 80, 108 et 110 et en ce que ledit érythrovirus V9 ne peut être reconnu moléculairement comme un génome d'érythrovirus B19, car présentant une divergence génétique supérieure ou égale à 10 % sur l'ensemble du génome par rapport aux séquences de l'érythrovirus B19.

Le génome viral dudit érythrovirus V9 est considéré comme génétiquement éloigné de l'érythrovirus B 19.

La présente invention a également pour objet un polynucléotide, **caractérisé en ce qu**'il est sélectionné dans le groupe constitué par :
a) le génome de l'érythrovirus de type V9 tel que défini ci-dessus, lequel génome présente une séquence sélectionnée dans le groupe constitué par :
   - les séquences présentant une divergence génétique ≥ 10 % sur l'ensemble du génome par rapport aux séquences de l'érythrovirus B19 et présentant une divergence génétique inférieure ou égale à 6 % par rapport à la SEQ ID NO: 1, et
   - les séquences capables de s'hybrider dans des conditions stringentes avec l'une des séquences SEQ ID NO: 45 à 80, 108 et 110, et
b) le polynucléotide de séquence SEQ ID NO : 1 représentant 95 % du génome de l'érythrovirus V9 tel que défini ci-dessus, incluant toutes les séquences codantes.

On entend par conditions stringentes, au sens de la présente invention, les conditions suivantes :
- hybridation pendant 3 à 24 h dans un tampon 1XSSC contenant 50 % de formamide, à 42°C et
- 3 lavages de 15 min dans un tampon 2XSSC, à 60°C.

La séquence SEQ ID NO:1, qui correspond à environ 95 % du génome d'un érythrovirus de type V9 et qui inclut toutes les séquences codantes, présente une carte de restriction différente de celle des érythrovirus B19, notamment pour ce qui concerne les sites BamHI (aucun site), HindIII (un seul site) et PvuII (cinq sites).

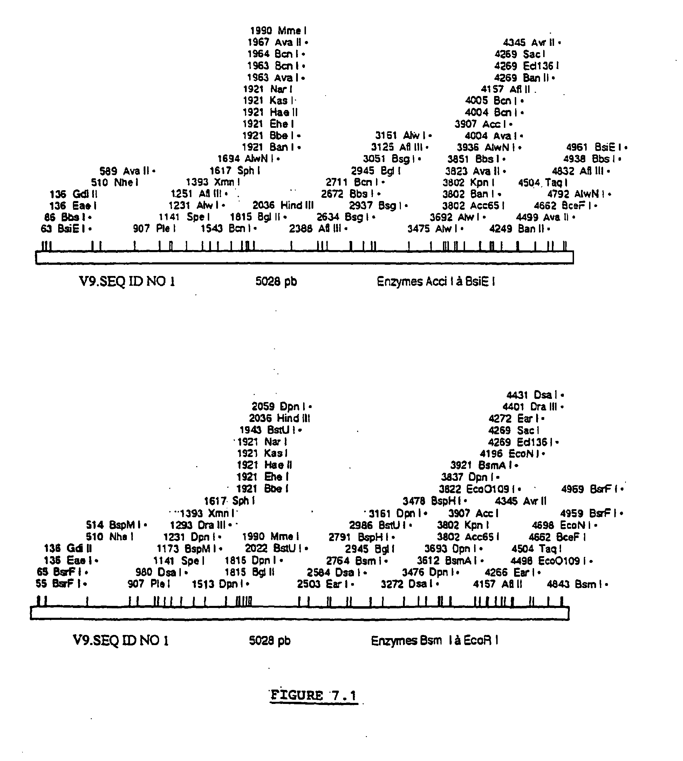

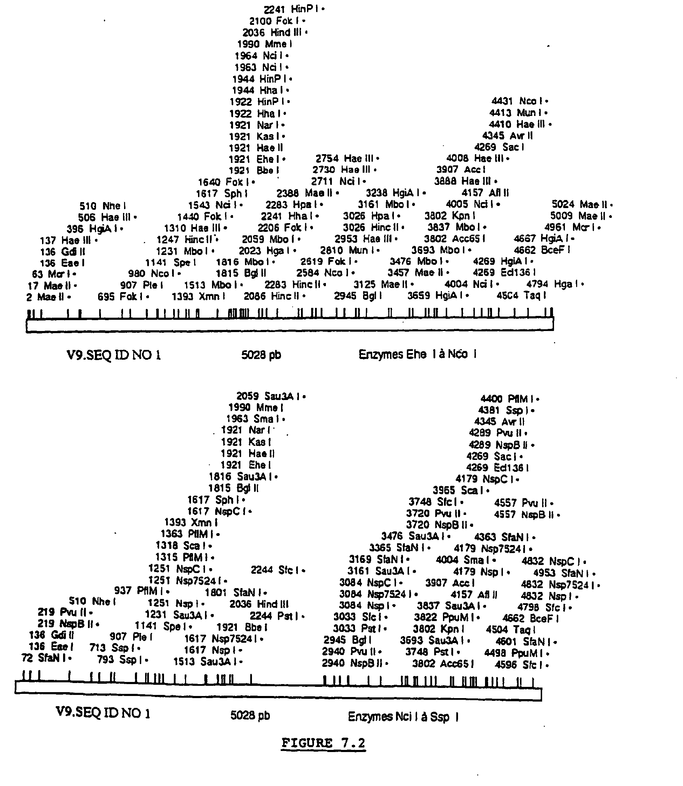

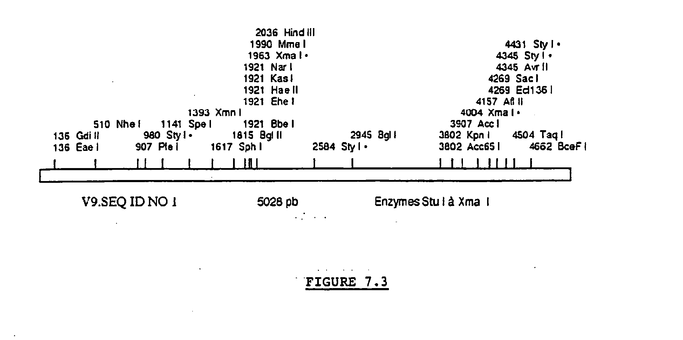

De manière plus précise, la séquence SEQ ID NO:1 présente un profil de restriction différent de celui de l'érythrovirus B19, notamment par les sites de restriction suivants : Acc I, Afl III, Alw I, AlwN I, **Apa I,** Ava I, Ava II, Avr II, **BamH I,** Ban I, Ban II, Bbe I, Bbs I, BceF I, Bcg I, Bcn I, **BgI II,** Bsg I, BsiE I, Bsm I, BsmA I, Bsp120 I, BspH I, BspM I, BsrF I, Bst1107 I, BstE II, BstU I, Bsu36 I, Dpn I, Dra III, Dsa I, Eae I, Eag I, Ear I, Ecl136 I, EcoN I, Eco109 I, EcoR I, Ehe I, Fok I, Hae I, Hae III, Hga I, HgiA I, Hha I, Hinc II, **Hind III,** HinP I, Hpa I, Kas I, Mae II, Mbo I, Mcr I, Msc I, **Mun I,** Nar I, Nci I, Nco I, Nsi I, Nsp I, Nsp7524 I, NspB II, NspC I, PflM I, Pme I, Ppu10 I, PpuM I, Pst I, **Pvu II**, Sac I, Sau3A I, Sca I, SfaN I, Sfc I, Sma I, Spe I, Sph I, Ssp I, Stu I, Sty I, Swa I, Tth11 I, Xba I, Xma I et leurs isoschizomères, conformément aux figures 7.1 à 7.3.

La présente invention a également pour objet un fragment de la séquence SEQ ID NO:1 telle que définie ci-dessus, **caractérisé en ce qu**'il représente une séquence nucléotidique codant pour une protéine dudit érythrovirus V9, sélectionnée dans le groupe constitué par les séquences :
- SEQ ID NO:81 correspondant aux positions 328-2340 de la SEQ ID NO:1, codant pour la protéine NS1,
- SEQ ID NO:83 correspondant aux positions 1796-2017 de la SEQ ID NO:1, codant pour la protéine de 7,5 kDa,
- SEQ ID NO:85 correspondant aux positions 2336-4678 de la SEQ ID NO:1, codant pour la protéine VP1,
- SEQ ID NO:87 correspondant aux positions 2336-3016 de la SEQ ID NO:1, codant pour la protéine VP1u,
- SEQ ID NO:89 correspondant aux positions 2523-2828 de la SEQ ID NO:1, codant pour la protéine X,
- SEQ ID NO: 91 correspondant aux positions 3017-4678 de la SEQ ID NO:1, codant pour la protéine VP2, et
- SEQ ID NO: 93 correspondant aux positions 4488-4883 de la SEQ ID NO:1, codant pour la protéine de 11 kDa.Selon un mode de réalisation avantageux dudit fragment, il est sélectionné dans le groupe constitué par les séquences SEQ ID NO:45-80, 108 et NO:110, leurs séquences complémentaires, les séquences d'au moins 17 nucléotides, issues de ces séquences et les séquences comprenant lesdites séquences et en ce qu'il est apte à servir de sonde ou d'amorce dans l'identification spécifique d'un érythrovirus de type V9 ou d'un érythrovirus apparenté.

Selon un autre mode de réalisation avantageux dudit fragment, il est sélectionné dans le groupe constitué par les séquences SEQ ID NO: 2 à 44, 105 à 107, 109, et 111 à 121 et les séquences complémentaires des séquences précédentes, et il est apte à servir de sonde ou d'amorce pour le diagnostic différentiel des érythrovirus.

Les séquences SEQ ID NO:105 à 121 sont respectivement également dénommées comme suit: SEQ ID NO:105 (E1905f), 106 (E1987r), 107 (E2076f), 108 (E2151r), 109 (E2406r), 110 (E2149rs), 111 (E2717f), 112 (E2901r), 113 (e1855f), 114 (e2960r), 115 (e1863f), 116 (e2953), 117 (e2435fStuI/BglII), 118 (e4813rEcoRI), 119 (e3115fBamHI), 120 (e4813rBamHI) et 121 (e1954fp).Les séquences complémentaires des séquences précédentes, les fragments issus des séquences précédentes d'au moins 17 nucléotides ou leurs séquences complémentaires sont également incluses dans la présente Demande.

Au sens de la présente invention, on entend par séquence nucléique ou séquence nucléotidique (séquence d'ADN ou d'ARN), l'une des séquences, telles que définies ci-dessus et leurs séquences complémentaires (séquences anti-sens) ainsi que les séquences comprenant une ou plusieurs desdites séquences ou fragments de celles-ci.

L'invention englobe également des fragments nucléotidiques complémentaires des précédents ainsi que des fragments modifiés par rapport aux précédents, par enlèvement ou addition de nucléotides dans une proportion d'environ 15 %, par rapport à la longueur des fragments ci-dessus et/ou modifiés au niveau de la nature des nucléotides, dès lors que les fragments nucléotidiques modifiés conservent une capacité d'hybridation avec la séquence d'ADN ou d'ARN d'érythrovirus V9, analogue à celle que présentent les fragments correspondants non modifiés.

Certains de ces fragments sont spécifiques et sont utilisés comme sonde ou amorce ; ils s'hybrident spécifiquement à un érythrovirus V9 ou un érythrovirus apparenté ; on entend par virus apparenté à l'érythrovirus V9, un érythrovirus présentant une divergence génétique < ou égale à 6 % ; ces fragments sont sélectionnés dans le groupe constitué par les séquences SEQ ID NO:45-80 et NO:108 et 110, ou leurs séquences complémentaires, les séquences issues de ces séquences d'au moins 17 nucléotides et les séquences comprenant lesdites séquences et trouvent application dans l'identification spécifique d'un érythrovirus V9 ou d'un érythrovirus apparenté.

D'autres de ces fragments sont utilisés en tant qu'amorces, pour l'amplification de séquences issues d'un érythrovirus de type V9 ou apparenté, telle que la séquence SEQ ID NO:1 ; ces amorces sont choisies dans le groupe constitué par les séquences SEQ ID NO:2-44 et les séquences SEQ ID NO:105-109 et 111-121 ou leurs séquences complémentaires et les séquences issues desdites séquences, d'au moins 17 nucléotides.

Lesdits fragments incluent également, lorsqu'il s'agit d'amorces, les séquences anti-sens.

De telles séquences trouvent application pour l'identification différentielle des érythrovirus (érythrovirus B19 et érythrovirus V9), associées avec des sondes telles que définies ci-dessus et/ou à des enzymes de restriction convenables.

Lesdites amorces comprennent de préférence entre 17 et 30 nucléotides ; des amorces préférées, sont les suivantes : la séquence SEQ ID NO:105 positions 1797-1815 de la séquence SEQ ID NO:1), qui correspond à un fragment de la séquence SEQ ID NO:10, la séquence SEQ ID NO:106 (positions 1899-1879 de la séquence SEQ ID NO:1), qui correspond à un fragment de la séquence anti-sens de la séquence SEQ ID NO:11, la séquence SEQ ID NO:107 (positions 1968-1987 de la séquence SEQ ID NO:1), qui correspond à un fragment de la séquence SEQ ID NO:13, la séquence SEQ ID NO:108 (positions 2061-2043 de la séquence SEQ ID NO:1), qui correspond à un fragment de la séquence anti-sens de la séquence SEQ ID NO:58, la séquence SEQ ID NO:109 (positions 2317-2298 de la séquence SEQ ID NO:1), qui correspond à un fragment de la séquence anti-sens de la séquence SEQ ID NO:16, la séquence SEQ ID NO:111 (positions 2609-2627 de la séquence SEQ ID NO:1), qui correspond à un fragment de la séquence SEQ ID NO:19 et la séquence SEQ ID NO:112 (positions 2812-2793 de la séquence SEQ ID NO:1), qui correspond à un fragment de la séquence anti-sens de la séquence SEQ ID NO:23.

Des paires d'amorces préférées sont les suivantes :
- paire A : amorces SEQ ID NO:111 et SEQ ID NO:112;
- paire B : amorces SEQ ID NO:105 et SEQ ID NO:106;
- paire C : l'une des séquences SEQ ID NO :2-44, 105, 106, 107, 109, 111 ou 112 et l'une des séquences SEQ ID NO:45-80, 108 ou 110 ;
- paire D : amorce SEQ ID NO:107 et amorce SEQ ID NO:109 ;
- paire E : deux amorces sélectionnées parmi les séquences SEQ ID NO:2-44, 105, 106, 107, 109, 111 ou 112 ;
- paire F : deux amorces sélectionnées parmi les séquences SEQ ID NO:45-80, 108 ou 110.

Ces différentes amorces peuvent être utilisées, selon le fragment amplifié, comme amorce sens ou comme amorce anti-sens.

La présente invention a également pour objet des sondes, **caractérisées en ce qu**'elles sont sélectionnées dans le groupe constitué par les séquences SEQ ID NO:45 à 80, 108, 110 et 121.

La présente invention a également pour objet un plasmide, **caractérisé en ce qu**'il comprend un polynucléotide tel que défini ci-dessus ou un fragment de la séquence SEQ ID NO:1 tel que défini ci-dessus.

Selon un mode de réalisation avantageux dudit plasmide, il inclut la séquence SEQ ID NO:1 (PCD.V9.C22).

La présente invention a également pour objet l'utilisation d'une molécule d'acide nucléique sélectionnée parmi les séquences SEQ ID NO:45-80, 108 et 110, leurs séquences complémentaires, les séquences d'au moins 17 nucléotides, issues de ces séquences, éventuellement marquées avec un marqueur approprié, pour la préparation d'un réactif de diagnostic pour la détection différentielle des érythrovirus de type V9.

Parmi les marqueurs appropriés, on peut citer, les isotopes radioactifs, les enzymes, les fluomchromes, des marqueurs chimiques (biotine...), les haptènes (digoxygénine...) et les anticorps ou analogues de bases appropriées.

La présente invention a également pour objet un procédé de diagnostic rapide et différentiel des érythrovirus, par hybridation et/ou amplification génique, réalisé à partir d'un échantillon biologique, lequel procédé est **caractérisé en ce qu**'il comprend :
(1) une étape dans laquelle l'on met en contact un échantillon biologique à analyser avec au moins une sonde de séquence SEQ ID NO:45-80, 108 ou 110 et
(2) une étape dans laquelle on détecte par tout moyen approprié le ou les produits résultant de l'interaction séquence nucléotidique d'érythrovirus-sonde.

De manière préférée, l'hybridation comprend une préhybridation, qui est réalisée dans un tampon qui comprend 5-60 % de formamide ; 1-5X SSC ; 2 % de réactif de blocage (*Blocking buffer*, Boehringer Mannheim, Meylan, France) ; 0,1 % de N-lauryl sarcosine ; 0,01-5 % de SDS, à 40-70°C pendant 90 minutes, puis l'hybridation est réalisée dans 3 ml d'un tampon de même composition avec 10 µl de sonde marquée à 40-70°C pendant 1-30 heures.

Conformément audit procédé, il peut comprendre, préalablement à l'étape (1) :
- une étape d'extraction de l'acide nucléique à détecter, appartenant au génome du virus, éventuellement présent dans l'échantillon biologique, et
- au moins un cycle d'amplification génique.

L'étape d'amplification génique est notamment réalisée à l'aide d'une des techniques d'amplification génique suivante : amplification par la Qβ-réplicase (I. Haruna et al., Proc. Nat. Acad. Sci. USA, 1965, 54, 579-587), PCR (réaction de polymérisation en chaîne) (R.K. Saiki et al., 1986, Nature, 324:163-6), LCR (*ligase chain reaction*) (F. Barany, Proc. Nat. Acad. Sci. USA, 1991, 88, 189-193), ERA (*end*-*run amplification*) (C. Adams et al., 1994, Novel amplification technologies for DNA/RNA-based diagnostics meeting, San Francisco, CA, Etats-Unis), CPR (*cycling probe reaction)* (P. Duck et al., Biotechniques, 1990, 9, 142-147) ou SDA (*strand displacement amplification*) (GT. Walker, 1994, SDA: novel amplification technologies for DNA/RNA-based diagnostics meeting, San Francisco, CA, Etats-Unis).

Selon un mode de mise en oeuvre avantageux dudit procédé, les cycles d'amplification sont réalisés à l'aide d'une paire d'amorces sélectionnée dans le groupe constitué par :
- la paire A : amorces SEQ ID NO: 111 et SEQ ID NO: 112 ;
- la paire B : amorces SEQ ID NO: 105 et SEQ ID NO: 106 ;
- la paire C : l'une des séquences SEQ ID NO: 2-44, 105, 106, 107, 109, 111 ou 112 et l'une des séquences SEQ ID NO:45-80, 108 ou 110 ;
- la paire D : amorce SEQ ID NO: 107 et amorce SEQ ID NO: 109 ;
- la paire E : deux amorces sélectionnées parmi les séquences SEQ ID NO: 2-44, 105, 106, 107, 109, 111 ou 112 ; et
- la paire F : deux amorces sélectionnées parmi les séquences SEQ ID NO: 45-80, 108 ou 110.

Lorsque l'on utilise la paire A, le produit d'amplification est avantageusement criblé par action de l'enzyme de restriction Apa I (GGGCCC) : le produit d'amplification d'un génome B 19 est clivé par Apa I (générant 2 fragments de 149 et 55 paires de bases (pb)) alors que le produit d'amplification d'un génome V9 n'est pas clivé par Apa I (un fragment de 204 pb) ; une électrophorèse sur gel d'agarose ou d'acrylamide permet de distinguer ces fragments de restriction.

Lorsque l'on utilise la paire B, le produit d'amplification est avantageusement criblé par action de l'une des enzymes de restrictions suivantes : BglII (AGATCT), ou MunI (CAATTG) ; on obtient ainsi différents fragments selon qu'il s'agit d'un érythrovirus V9 ou B19 ; un fragment qui comprend un site de restriction BglII est spécifique de l'érythrovirus V9 variant tel que défini ci-dessus, alors que les érythrovirus B 19 comprennent dans cette zone, un site MunI. Le produit d'amplification d'un génome B19 est clivé par MunI (générant 2 fragments de 36 et 67 pb) et n'est pas clivé par BglII (un fragment de 103 pb) alors que le produit d'amplification d'un génome V9 est clivé par BglII (2 fragments de 19 et 84 pb) et n'est pas clivé par MunI (un fragment de 103 pb) ; une électrophorèse sur gel d'agarose ou d'acrylamide permet de distinguer ces différents fragments de restriction.

Lorsque l'on utilise la paire C (une amorce apte à hybrider avec tous les érythrovirus et une amorce apte à hybrider de manière spécifique avec l'érythrovirus V9) ou la paire F (deux amorces aptes à hybrider de manière spécifique avec l'érythrovirus V9), le génome V9 est amplifié alors qu'il n'y a pas d'amplification spécifique avec le génome B 19.

Lorsque l'on utilise la paire D, le produit d'amplification est avantageusement criblé par hybridation avec une sonde spécifique marquée de l'érythrovirus V9, sélectionnée parmi les séquences SEQ ID NO:58-60 et 110, de préférence par hybridation avec la sonde de séquence SEQ ID NO:110 ; le produit d'amplification d'un génome V9 hybride de manière spécifique avec ces sondes et notamment la sonde de séquence SEQ ID NO:110, alors que le produit d'amplification d'un génome B19 n'hybride pas avec les sondes précitées.

Lorsque l'on utilise la paire E, le produit d'amplification est criblé par tout procédé d'hybridation avec une sonde spécifique de l'érythrovirus V9 sélectionnée parmi les séquences SEQ ID NO:45-80, 108 et 110 ; dans ce cas, le produit d'amplification d'un génome V9 hybride avec la sonde, mais pas le produit d'amplification d'un génome B 19.

L'invention a en outre pour objet un procédé de diagnostic rapide et différentiel des érythrovirus, réalisé à partir d'un échantillon biologique, lequel procédé est **caractérisé en ce qu**'il comprend :
- une étape d'extraction de l'acide nucléique à détecter, appartenant au génome du virus, éventuellement présent dans l'échantillon biologique,
- au moins un cycle d'amplification génique à l'aide de la paire d'amorces B telle que définie ci-dessus et
- la détection du produit amplifié d'une part par hybridation avec la sonde de séquence SEQ ID NO:121 et d'autre part par action de l'enzyme de restriction Mun I.

L'invention a également pour objet un procédé de diagnostic rapide et différentiel des érythrovirus, réalisé à partir d'un échantillon biologique, lequel procédé est **caractérisé en ce qu**'il comprend :
- une étape d'extraction de l'acide nucléique à détecter, appartenant au génome du virus, éventuellement présent dans l'échantillon biologique,
- au moins un cycle d'amplification génique à l'aide de la paire d'amorces A telle que définie ci-dessus et
- la détection du produit amplifié par action de l'enzyme ApaI.

L'invention a également pour objet un procédé de diagnostic rapide et différentiel des érythrovirus, réalisé à partir d'un échantillon biologique, lequel procédé est **caractérisé en ce qu**'il comprend :
- une étape d'extraction de l'acide nucléique à détecter, appartenant au génome du virus, éventuellement présent dans l'échantillon biologique,
- au moins un cycle d'amplification génique à l'aide de la paire d'amorces C telle que définie ci-dessus et
- la détection du produit amplifié.

L'invention a en outre pour objet un procédé de diagnostic rapide et différentiel des érythrovirus, réalisé à partir d'un échantillon biologique, lequel procédé est **caractérisé en ce qu**'il comprend :
- une étape d'extraction de l'acide nucléique à détecter, appartenant au génome du virus, éventuellement présent dans l'échantillon biologique,
- au moins un cycle d'amplification génique à l'aide de la paire d'amorces D telle que définie ci-dessus et
- la détection du produit amplifié par hybridation avec une sonde dont la séquence est sélectionnée dans le groupe constitué par les séquences SEQ ID NO:58-60 et 110.

L'invention a également pour objet un procédé de diagnostic rapide et différentiel des érythrovirus, réalisé à partir d'un échantillon biologique, lequel procédé est **caractérisé en ce qu**'il comprend :
- une étape d'extraction de l'acide nucléique à détecter, appartenant au génome du virus, éventuellement présent dans l'échantillon biologique,
- au moins un cycle d'amplification génique à l'aide de la paire d'amorces E telle que définie ci-dessus et
- la détection du produit amplifié par hybridation avec une sonde dont la séquence est sélectionnée dans le groupe constitué par les séquences SEQ ID NO:45-80, 108 et 110.

L'invention a en outre pour objet un procédé de diagnostic rapide et différentiel des érythrovirus, réalisé à partir d'un échantillon biologique, lequel procédé est **caractérisé en ce qu**'il comprend :
- une étape d'extraction de l'acide nucléique à détecter, appartenant au génome du virus, éventuellement présent dans l'échantillon biologique,
- au moins un cycle d'amplification génique à l'aide de la paire d'amorces F telle que définie ci-dessus et
- la détection du produit amplifié.

L'invention a également pour objet l'utilisation des séquences décrites ci-dessus, pour la mise en oeuvre d'un procédé d'hybridation ou d'amplification génique de séquences nucléiques d'érythrovirus, pour le diagnostic *in vitro* de l'infection potentielle d'un individu par un érythrovirus.

La présente invention a également pour objet un procédé de criblage et de typage d'un érythrovirus V9 ou apparenté, **caractérisé en ce qu**'il comprend la mise en contact d'une sonde sélectionnée dans le groupe constitué par les séquences SEQ ID NO:45-80, 108 et 110, leurs séquences complémentaires, les séquences d'au moins 17 nucléotides, issues de ces séquences et les séquences comprenant lesdites séquences, éventuellement marquée, avec l'acide nucléique du virus à typer et la détection de l'hybride acide nucléique-sonde obtenu.

La présente description présente une protéine, **caractérisée en ce qu**'elle est codée par un polynucléotide selon l'invention ou bien un fragment de séquence SEQ ID NO:1 selon l'invention.

Ladite protéine, présente une séquence sélectionnée dans le groupe constitué par les séquences SEQ ID NO : 82, 86, 88 et 92.

La présente description présente également un peptide, **caractérisé en ce qu**'il représente un fragment d'une protéine telle que définie ci-dessus, sélectionné dans le groupe constitué par les séquences SEQ ID NO : 95 à 104.

On entend par peptide, ci-après, aussi bien les protéines que les peptides, tels que définis ci-dessus.

De tels peptides sont notamment aptes à être reconnus par des anticorps induits par un érythrovirus V9 et/ou à induire la production d'anticorps anti-érythrovirus V9.

Lesdits peptides sont notamment sélectionnés parmi les séquences SEQ ID NO:82 (protéine NS1), SEQ ID NO:86 (protéine VP1), SEQ ID NO:88 (protéine VP1 unique), SEQ ID NO:92 (protéine VP2) et SEQ ID NO:95-104, à savoir des fragments de la protéine VP1 [peptide VP1a (SEQ ID NO:95) ; peptide VP1b (SEQ ID NO:96) ; peptide VP1c (SEQ ID NO:97) peptide VP1d (SEQ ID NO:98) ; peptide VP1e (SEQ ID NO:99 et peptide VP1f (SEQ ID NO:100)], ou des fragments de la protéine VP2 [peptide VP2a (SEQ ID NO:101) ; peptide VP2b (SEQ ID NO:102) ; peptide VP2c (SEQ ID NO:103) ; peptide VP2d (SEQ ID NO:104] ainsi que les peptides dérivés comprenant 7 à 50 aminoacides.

La description présente a également des compositions immunogènes comprenant une ou plusieurs protéines telles que définies ci-dessus.

La description présente également des anticorps, **caractérisés en ce qu**'ils reconnaissent parmi les protéines d'érythrovirus, uniquement celle(s) de l'érythrovirus variant, telles que définies ci-dessus.

La présente description présente également un procédé de détection immunologique d'une infection à érythrovirus V9, réalisé à partir d'un échantillon biologique, **caractérisé en ce qu**'il comprend :
- pour la détection des anticorps anti-érythrovirus V9, la mise en contact d'un échantillon biologique avec une protéine ou un peptide selon l'invention (sérodiagnostic),
- pour la détection des protéines virales d'érythrovirus V9, la mise en contact d'un échantillon biologique avec un anticorps selon l'invention ;
la lecture du résultat étant révélée par un moyen approprié, notamment EIA, ELISA, RIA, fluorescence.

A titre d'illustration, une telle méthode de diagnostic *in vitro* selon l'invention comprend la mise en contact d'un échantillon biologique prélevé chez un patient, avec des anticorps selon l'invention ou des peptides selon l'invention, et la détection à l'aide de tout procédé approprié, notamment à l'aide d'anti-immunoglobulines marquées, des complexes immunologiques formés entre les antigènes ou les anticorps des érythrovirus éventuellement présents dans l'échantillon biologique et lesdits anticorps ou lesdits peptides, respectivement.

Les réactifs selon l'invention sont notamment utiles pour la détection des érythrovirus V9 et apparentés chez les femmes enceintes, chez les patients VIH-positifs présentant une anémie et/ou une thrombopénie chronique, les greffés d'organe ou de moelle, et les patients présentant une anémie aiguë centrale et dont les tests de détection de l'érythrovirus B19 sont négatifs.

L'invention a en outre pour objet une trousse de diagnostic d'érythrovirus, **caractérisée en ce qu**'elle inclut au moins une paire d'amorces selon l'invention, et/ou une sonde selon l'invention.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- les figures 1, 2 et 3 illustrent les arbres phylogénétiques de l'érythrovirus V9: figure 1 : arbre phylogénétique de la séquence complète d'érythrovirus ; figure 2 : arbre phylogénétique des gènes NS1 d'érythrovirus ; figure 3 : arbre phylogénétique des gènes VP1 d'érythmvirus ;
- les figures 4, 5 et 6 représentent les distances génétiques des séquences complètes d'érythrovirus (figure 4), des gènes NS1 (figure 5) et des gènes VP1 (figure 6) d'érythrovirus.
- la figure 7 illustre la carte de restriction de la séquence ID NO:1.

Il doit être bien entendu, toutefois, que ces exemples sont donnés uniquement à titre d'illustration de l'objet de l'invention, dont ils ne constituent en aucune manière une limitation.

### EXEMPLE 1 : Obtention des séquences conformes à l'invention.

Un fragment de restriction Aat II/Aat II de 5 028 pb représentant la quasi totalité (95 %) du génome du variant V9 a été cloné dans le vecteur de séquençage pcDNA2.1 (Invitrogen, Netherlands) de la façon suivante.

L'ADN viral simple brin est extrait du sérum d'un patient présentant une crise érythroblastopénique aiguë à l'aide d'une colonne QIAamp Blood Kit (Qiagen S.A., France). Par une étape d'hybridation dans un tampon NaCl 50 mM à 56°C pendant 16 heures, l'ADN viral est transformé en ADN double brin. Puis 1,3 µg d'ADN viral double brin est soumis à l'enzyme de restriction Aat II (18 U) à 37°C pendant 2 heures, l'enzyme de restriction est ensuite inactivée à 65°C pendant 15 minutes. Le produit est dialysé sur membrane d'acétate et de nitrate de cellulose Millipore^{®} VSWPO13000 contre de l'eau pendant 2 heures. Le fragment de restriction Aat II/Aat II d'ADN viral double brin ainsi préparé est congelé à -20°C en attendant l'étape de ligation.

Le vecteur pcDNA2.1 est modifié pour recevoir le fragment Aat II par mutagénèse dirigée insertionnelle : le site de restriction Eag I du multisite de clonage a été supprimé et remplacé par un site Aat II. Le vecteur pcDNA2.1a, ainsi produit, est amplifié en culture bactérienne et purifié à l'aide d'un QIAfilter Plasmid Maxi Kit (Qiagen S.A., France). Puis 3 µg du vecteur pcDNA2.1a est soumis à une restriction par l'enzyme Aat II à 37°C pendant 1 heure, puis déphosphorylé par la phosphatase alcaline de crevette (Boehringer Mannheim, Meylan France). Les enzymes sont inactivées à 65°C pendant 15 minutes.

La ligation est réalisée avec un rapport molaire vecteur/ insert d'ADN viral de 1/l soit 50 ng de vecteur et 100 ng d'insert d'ADN viral, préparés comme décrits ci-dessus, à l'aide de 1 U de ligase T4 (Life Technologies, France) à 24 °C pendant 16 heures. Après dilution au 1/2, le produit de ligation est chauffé à 65°C pour inactiver la ligase T4 puis refroidi sur de la glace. Des bactéries électrocompétentes Sure® (Stratagene, Heidelberg Allemagne) sont électroporées avec 2 ou 4 µl de cette solution de ligation (1500 V, 50 µF, 200 Ω) puis mises à incuber avec 1 ml de milieu SOC (Life Technologies, France) pendant 1 heure avant d'être étalées sur un milieu gélosé Luria Broth (Life Technologies, France) contenant 100 µg/ml d'amoxycilline, 15 µg/ml de tétracycline, 100 µg/ml d'IPTG et 50 µg/ml de X-gal.

Vingt quatre colonies blanches (recombinantes) ont été sélectionnées ; leur plasmide est extrait par minipréparation d'ADN et une carte de restriction sommaire (Aat II, Aat II + BamH I, BamH I, BamH I + Bgl II, Hind II) a permis de sélectionner 2 clones recombinants avec un insert de taille et de carte de restriction compatibles avec un insert d'ADN viral V9.

Ces 2 clones (2 et 22) ont été séquencés à l'aide d'un séquenceur automatique ABI 377 (Perkin Elmer, France) : ils contiennent bien un insert de 5 028 pb, les 2 séquences sont identiques sauf à la position 1165 (A et G pour les clones 2 et 22 respectivement). La séquence directe de l'ADN viral V9 a permis de déterminer que c'est le G en position 1165 qui est correct, c'est donc le clone 22 qui a été sélectionné (PCD.V9.C22), dont la séquence correspond à SEQ ID NO:1.

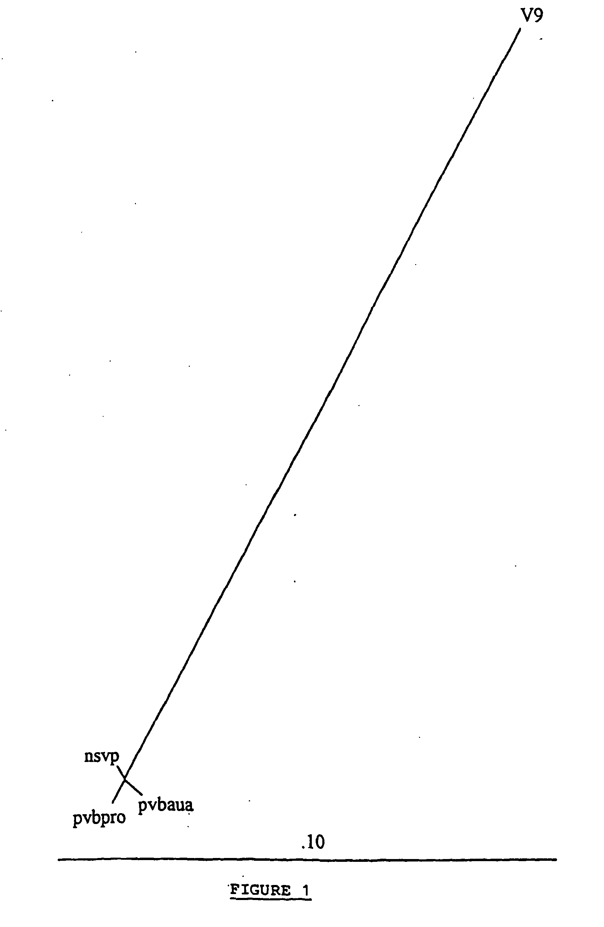

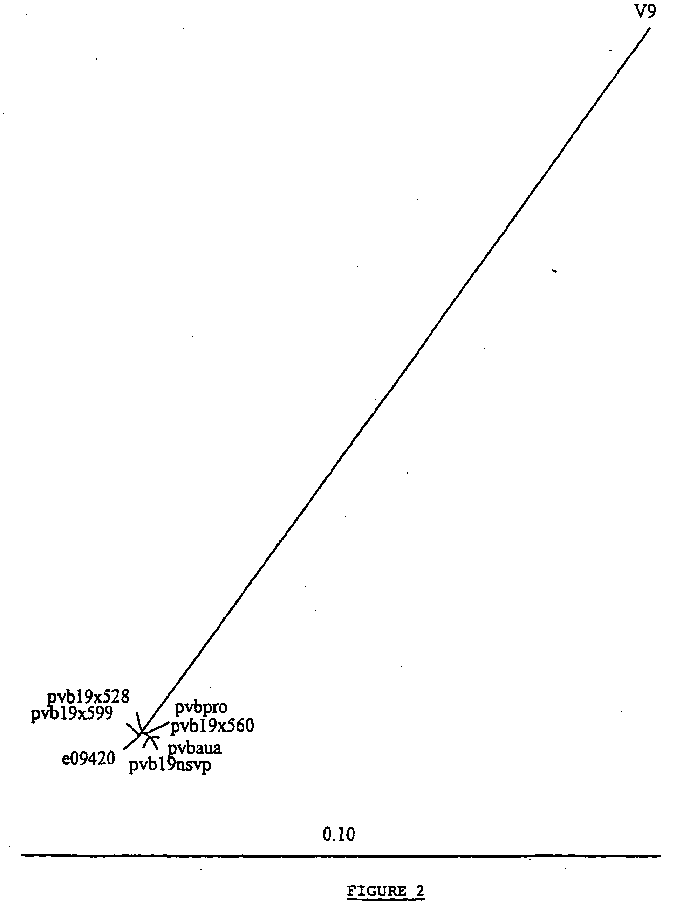

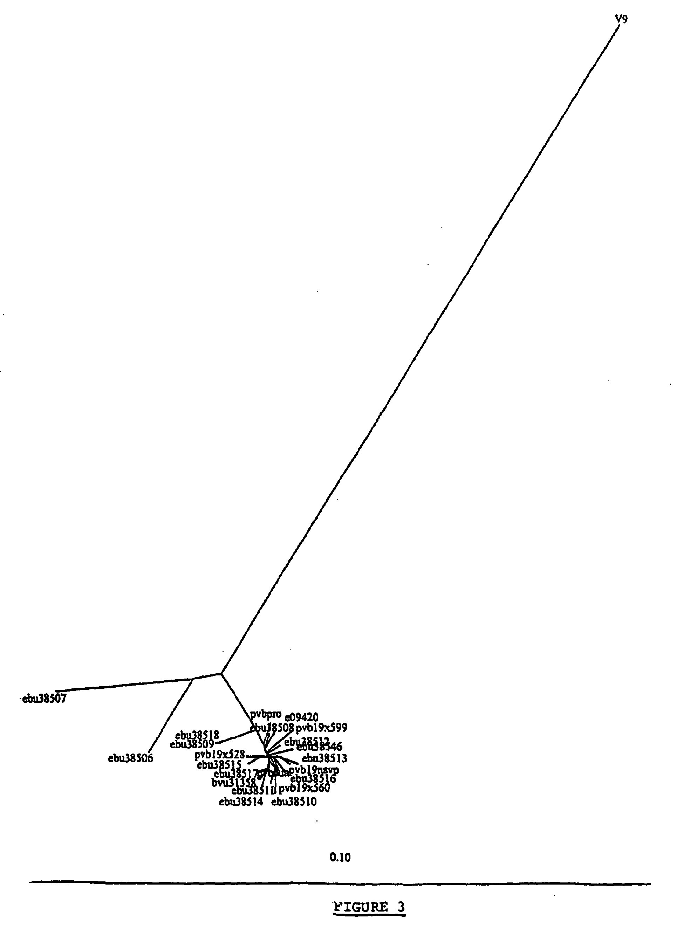

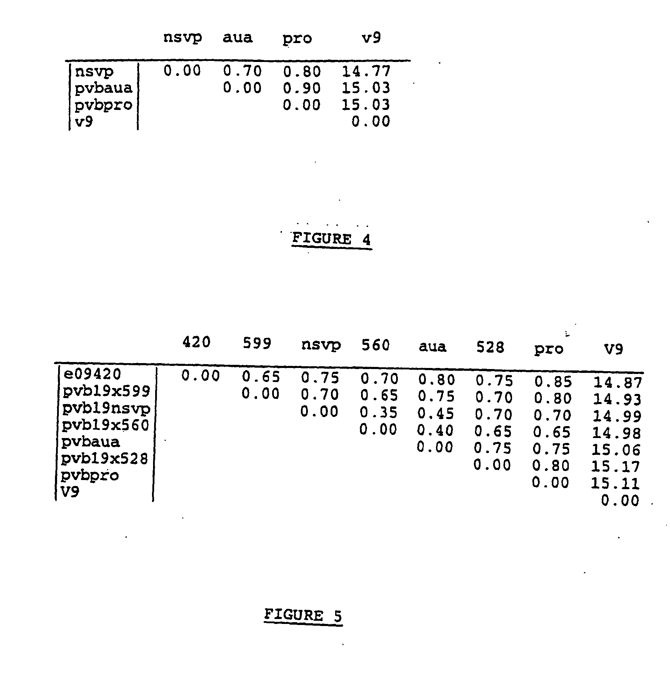

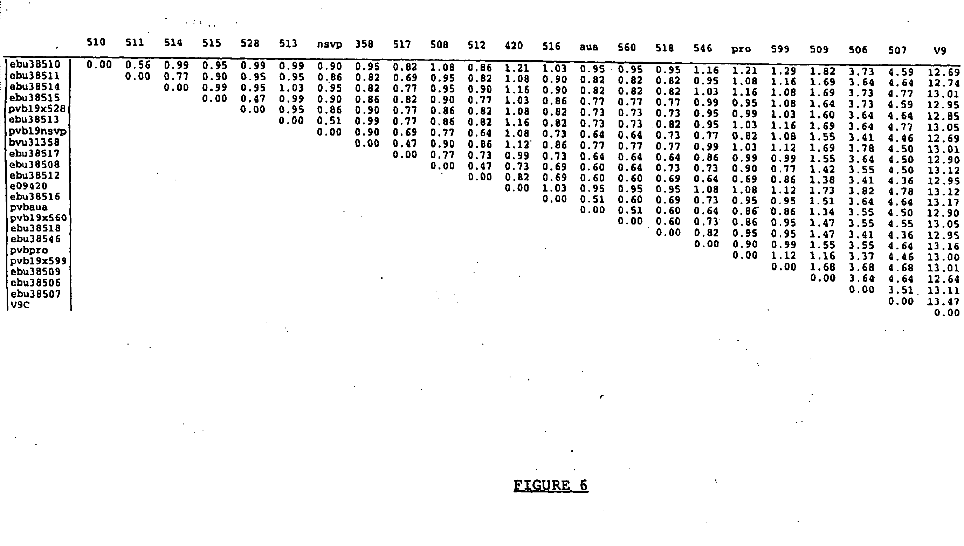

Les figures 1 à 6 montrent les distances génétiques qui existent entre l'érythrovirus V9 et l'érythrovirus B19. Dans ces figures, les différentes séquences d'érythrovirus sont représentées par leur mnémonique dans GeneBank (*release* 103.0 d'octobre 1997).

### EXEMPLE 2 : Diagnostic d'un érythrovirus de type V9 par hybridation ADN (dot blot ou slot blot ou microplaque) avec une sonde spécifique.

L'ADN viral est extrait par exemple à l'aide d'une colonne QIAamp Blood Kit (Qiagen S.A., France) ou de tout autre procédé d'extraction des acides nucléiques à partir d'un prélèvement biologique (sang, sérum, plasma, liquide amniotique, moelle osseuse, tissus). L'ADN en solution est dénaturé à 95°C pendant 2 minutes puis refroidi sur de la glace, transféré sur membrane de Nylon ou d'acétate de cellulose par filtration sous vide, puis fixé (chauffage de la membrane à 80°C pendant 1 heure). La membrane est ensuite hybridée en conditions stringentes avec une sonde ADN ou ARN spécifique de V9, telle que la séquence SEQ ID NO:1 ou son complémentaire ou un fragment de celle-ci, en particulier les séquences SEQ ID NO:45 à SEQ ID NO:80 et 110 ou leur complémentaire, ou un fragment de ces séquences marquées de manière appropriée. Ce marquage peut être un marquage avec un radio-élément (³²P, ³³P, ³⁵S, ³H, ¹⁴C ou un autre radio-isotope), un marquage froid (biotine, marqueur fluorescent, digoxygénine ou tout autre molécule pouvant être couplée ou incorporée dans un fragment d'ADN ou d'ARN et pouvant être détectée par un anticorps spécifique, ou par un chélate de ruthénium). Dans le cas d'un marquage par un élément radioactif, la révélation est effectuée par auto-radiographie ou tout autre procédé permettant la détection de l'émission du radio-isotope (tel que le Phosphorimager, Molecular Dynamics, Bondoufle, France). Dans le cas d'un marquage à la biotine, la révélation est effectuée à l'aide d'un conjugué enzyme/streptavidine et un substrat de révélation adapté. Dans le cas d'un marquage fluorescent, la révélation se fait à l'aide d'un fluoro-Imager (Molecular Dynamics, Bondoufle, France) ou de tout autre appareil capable de détecter l'émission de fluorescence. Dans le cas d'un marquage à la digoxygénine (ou avec un autre antigène) la révélation se fait à l'aide d'un anticorps anti-digoxygénine (ou spécifique de l'antigène utilisé pour le marquage), couplé directement à une enzyme (phosphatase alcaline, peroxydase ou toute autre enzyme), ou de manière indirecte par un anticorps anti-digoxygénine (ou spécifique de l'antigène utilisé pour le marquage) et un anti-anticorps couplé à une enzyme. Un substrat adapté à l'enzyme du conjugué est utilisé pour la révélation. Dans le cas d'un marquage par chélate de ruthénium (comme le TBR) la révélation s'effectue par une réaction d'électrochemiluminescence (G.F. Blackburn et al., Clin. Chem., 1991, 37:1534-1539).

Une variante de cette technique comprend la fixation de l'ADN viral sur micro-plaque ou un autre support solide et l'hybridation avec une sonde marquée comme précisé ci-dessus.

Une autre variante de cette technique comprend la fixation d'une sonde non marquée sur une micro-plaque ou un autre support solide et l'hybridation avec l'ADN viral de l'échantillon qui aura été préalablement marqué.

### EXEMPLE 3 : Diagnostic d'un érythrovirus de type V9 par amplification génique (PCR ou polymerase chain reaction) et hybridation :

On extrait de l'ADN viral à partir d'un échantillon biologique (sang, sérum, plasma, liquide amniotique, moelle osseuse, tissus), à l'aide d'une colonne QIAamp Blood Kit (Qiagen S.A., France) ou de tout autre procédé d'extraction des acides nucléiques.

La PCR est réalisée selon la méthode décrite par Saiki et al. (Nature, 1986, 324:163-66) avec 10 µl de solution d'ADN dans un volume final de 100 µl de mélange réactionnel (50 mM KCl ; 10 mM Tris-HCl pH 8,3 ; 2,5 mM MgCl₂ ; 200 µM dNTP; 25 pmoles d'oligonucléotides sens et anti-sens) avec 1,5 UI d'AmpliTaq Gold ™ (Perkin Elmer, France). Les amorces d'amplification sont des oligonucléotides de 20 à 25 mers choisis pour n'amplifier que l'ADN du variant V9 : soit les 2 amorces (sens et anti-sens) sont des fragments des séquences spécifiques du V9 (SEQ ID NO:45 à 80, 108 et 110) ou leurs complémentaires, soit l'une des amorces est choisie parmi ces séquences spécifiques du V9 (SEQ ID NO:45-80, 108 et 110) ou leurs complémentaires tandis que l'autre amorce est choisie parmi les séquences aptes à hybrider aussi bien les érythrovirus B19 que les érythrovirus V9 (SEQ ID NO:2 à 44, 105-107, 109 et 111-112) ou leurs complémentaires. Les cycles de températures sont imposés au mélange réactionnel par un thermocycleur (T9600, Perkin Elmer, France) selon le programme suivant :
1 cycle :
   - 6 minutes à 95°C
5 cycles :
   - 60 secondes à 95°C
   - 30 secondes à 60°C
   - 30 secondes à 72°C
45 cycles :
   - 30 secondes à 95°C
   - 30 secondes à 60°C
   - 30 secondes à 72°C
1 cycle :
   - 5 minutes à 72°C

Le produit d'amplification est déposé sur un gel d'agarose à 1,3 % pour subir une séparation électrophorétique et un transfert sur une membrane de Nylon chargée par capillarité selon une technique classique (Sambrook J. et al., 1989, Molecular Cloning: A Laboratory Manual. Cold Spring Harbor Laboratory Press, Cold Spring Harb*or*).

La sonde est un oligonucléotide de 20-30 mers, fragment d'une séquence spécifique du V9 (SEQ ID NO:45 à 80, 108 et 110) ou leurs complémentaires. Elle est marquée en 3' par du DIG-dUTP à l'aide du kit DIG *Oligonucleotide Tailing* (Boehringer Mannheim, Meylan, France).

La membrane de transfert est préhybridée dans un tampon comprenant (50 % formamide ; 5X SSC ; 2 % de réactif de blocage (Boehringer Mannheim, Meylan, France) ; 0,1 % de N-lauryl sarcosine ; 0,02 % de SDS), à 42°C pendant 90 minutes. L'hybridation est réalisée dans 3 ml d'un tampon de même composition avec 10 µl de sonde marquée à 42°C pendant 16 heures. La membrane est lavée 2 fois en tampon 2X SSC; 0,1 % SDS à 60°C pendant 10 minutes, puis 2 fois en tampon 1X SSC ; 0,1 % SDS à 60°C pendant 10 minutes. La membrane est ensuite révélée avec le DIG *Luminescent Detection Kit* (Boehringer Mannheim, Meylan, France) et une auto-radiographie.

### EXEMPLE 4 : Diagnostic de groupe et diagnostic différentiel des érythrovirus de type B19 et V9 par amplification génique et hybridation:

On extrait de l'ADN viral à partir d'un échantillon biologique (sang, sérum, plasma, liquide amniotique, moelle osseuse, tissus) à l'aide d'une colonne QIAamp Blood Kit (Qiagen S.A., France) ou de tout autre procédé d'extraction des acides nucléiques.

La PCR est réalisée selon la méthode décrite par Saiki et al. (Nature, 1986, précité) avec 10 µl de la solution d'ADN dans un volume final de 100 µl de mélange réactionnel (50 mM KCl ; 10 mM Tris-HCl pH 8,3 ; 2,5 mM MgCl₂ ; 200 µM dNTP ; 25 pmoles d'oligonucléotides sens et anti-sens) avec 1,5 UI d'AmpliTaq Gold™ (Perkin Elmer, France). Les amorces d'amplification sont des oligonucléotides de 20 à 25 mers choisis pour amplifier l'ADN du B19 et du variant V9 : les 2 amorces (sens et anti-sens) sont des fragments des séquences aptes à s'hybrider aussi bien avec les érythrovirus B19 qu'avec les érythrovirus V9 (SEQ ID NO:2 à 44, 105-107, 109, 111-112) ou de leurs complémentaires. Les cycles de températures sont imposés au mélange réactionnel par un thermocycleur (T9600, Perkin Elmer, France) selon le programme suivant :
1 cycle :
   - 6 minutes à 95°C
5 cycles :
   - 60 secondes à 95°C
   - 30 secondes à 60°C
   - 30 secondes à 72°C
45 cycles :
   - 30 secondes à 95°C
   - 30 secondes à 60°C
   - 30 secondes à 72°C
1 cycle :
   - 5 minutes à 72°C

Le produit d'amplification est déposé sur un gel d'agarose à 1,3% pour subir une séparation électrophorétique et un transfert sur une membrane de Nylon chargée par capillarité selon une technique classique (J. Sambrook et al., 1989, précité).

La sonde est un oligonucléotide de 20-30 mers, fragment d'une séquence spécifique du V9 (SEQ ID NO:45-80, 108 et 110) ou de leurs complémentaires, ou bien spécifique du B 19, ou enfin qui s'hybride aussi aux B 19 qu'aux V9 (SEQ ID NO:2 à 44 ou 105-107, 109, 111-112), si on cherche à réaliser un diagnostic de groupe. Elle est marquée en 3' par du DIG-dUTP à l'aide du kit DIG oligonucleotide tailing (Boehringer Mannheim, Meylan, France).

La membrane de transfert est préhybridée et hybridée dans les mêmes conditions que celles exposées à l'exemple 3.

### EXEMPLE 5 : Diagnostic de groupe et diagnostic différentiel des érythrovirus de type B19 et V9 par amplification génique et enzymes de restriction.

Extraction de l'ADN viral à partir d'un échantillon biologique (sang, sérum, plasma, liquide amniotique, moelle osseuse, tissus) à l'aide d'une colonne QIAamp Blood Kit (Qiagen S.A., France) ou de tout autre procédé d'extraction des acides nucléiques.

La PCR NS1a est réalisée selon la méthode décrite par Saiki et al. avec 5 µl de la solution d'ADN dans un volume final de 50 µl de mélange réactionnel (50 mM KCl; 10 mM Tris-HCl pH 8,3; 2,5 mM MgCl₂; 200 µM dNTP; 12,5 pmoles d'oligonucléotides sens et anti-sens) avec 1,5 UI d'AmpliTaq Gold™ (Perkin Elmer, France) et la paire d'amorce B (amorce sens e1905f, SEQ ID NO:105; et amorce antisens e1987r, SEQ ID NO:106) en utilisant les cycles de températures suivants (sur un thermocycleur T9700, Perkin Elmer, France) :
- 1 cycle :
   6 minutes à 94°C
- 5 cycles :
   30 secondes à 94°C
   1 minute à 55°C
   1 minute à 72°C
- 45 cycles :
   30 secondes à 94°C
   30 secondes à 60°C
   30 secondes à 72°C
- 1 cycle :
   7 minutes à 72°C

Un aliquot du produit d'amplification (10 µl) a été déposé sur un gel d'agarose à 2% pour subir une séparation électrophorétique et un transfert sur une membrane de nylon chargée par capillarité selon une technique classique (J. Sambrook et al., 1989, précité). La membrane a été hybridée avec une sonde oligonucléotidique de 36 mer, e1954fp (SEQ ID NO:121) : ACCAGTATCAGCAGCAGTGGTGGTGAAAGCTCTGAA, fragment de la séquence SEQ ID NO:11. Cette sonde permet une détection des érythrovirus de type B19 et V9.

Un aliquot du produit d'amplification (10 µl) a été soumis à l'action de l'enzyme de restriction Mun I pendant 2 heures puis soumis à une séparation électrophorétique sur un gel d'agarose à 2%. Comme précédemment décrit le type d'érythrovirus est B19 s'il y a clivage, et V9 s'il n'y a pas clivage.

### Résultats de la PCR NS1a :

79 échantillons retrouvés indéterminés ou positifs faibles avec l'ancienne PCR B 19 (Lefrere, et al., Transfusion, 1995, 35:389-391) ont été criblés à l'aide de la nouvelle PCR NS1a (érythrovirus consensus, séquences selon l'invention). Sur les 79 échantillons criblés, 31 sont positifs et ont été typés à l'aide de l'enzyme de restriction Mun I : 18 (58%) ont été retrouvés de type B 19 et 13 (42%) de type V9.

Les échantillons positifs en PCR NS1a ont pu être amplifiés sur 1110 pb par une PCR nichée (PCR S1S2) à l'aide de la paire d'amorces e1855f (SEQ ID NO:113) et e2960r (SEQ ID NO:114) pour la première étape d'amplification de 30 cycles (PCRS1), et de la paire d'amorces e1863f (SEQ ID NO:115) et e2953r (SEQ ID NO:116) pour la deuxième étape d'amplification de 50 cycles (PCRS2). 15 échantillons ont été retrouvés positifs en PCR S1S2 et séquencés sur 1110 pb (13 de type B19 en PCR NS1A et 2 de type variant). L'analyse des séquences a montré que :
- les amorces B (amorce sens e1905f, SEQ ID NO:105; et amorce antisens e1987r, SEQ ID NO:106) sont parfaitement conservées pour toutes les 15 séquences (de type B19 et variant) ainsi que pour toutes les séquences de B19 connues, confirmant leur intérêt pour une utilisation pour un test de diagnostic consensus pour le B19 et le V9.
- la sonde e1954fp (SEQ ID NO:121), fragment de la séquence SEQ ID NO:11 est elle aussi bien conservée pour les 15 séquences ainsi que pour toutes les séquences de B19 connues,
- les séquences B 19 forment un groupe bien homogène avec moins de 1,2% de divergence entre elles (7 séquences B19 de GenBank et les 13 séquences B 19 de cette étude),
- enfin pour les 2 séquences typées érythrovirus variant par la PCR NS1a avec la digestion Mun I, moins de 4,5 %b de divergence avec V9 est observée.

### EXEMPLE 6 : Clonage des gènes de capside VP1 et VP2 de V9 dans un vecteur d'expression baculovirus:

Première étape :
- clonage du gène VP 1 dans un plasmide bactérien

Le gène VP1 de V9 est amplifié par PCR selon la méthode décrite par Saiki et al. (Nature, 1986, 324:163-166) avec 10 µl d'une dilution 10⁻² d'ADN viral V9 dans un volume final de 100 µl de mélange réactionnel (20 mM Tris-HCl pH 8,8 ; 10 mM KCl, 10 mM (NH₄)₂SO₄ ; 2 mM MgSO₄ ; 0,1% Triton X-100 ; 0,1 mg/ml de BSA; 0,2 mM dNTP ; 25 pmoles d'amorces sens (e2435fStuI/BglII: AAAGGCCTAGATCTTGTAGATTATGAGTAAAAC, SEQ ID NO:117) et antisens (e4813rEcoRI: CGGAATTCGGTGGGTGACGGTTCCTG, SEQ ID NO:118)) avec 2,5 U de Pfu Turbo™ (Stratagene, France). Les amorces d'amplification ont été choisies sur la séquence V9 de part et d'autre du gène VP 1, leur extrémité 5' a été modifiée par addition de site(s) de restriction (indiqués dans leur dénomination) pour faciliter le clonage. Les cycles de températures imposés au mélange réactionnel sont les suivants :
1 cycle :
   - 1 minute à 94°C
20 cycles :
   - 1 minute à 94°C
   - 1 minute à 55°C
   - 2,5 minute à 72°C
1 cycle :
   - 10 minute à 72°C

Le produit d'amplification du gène VP1 a été purifié à l'aide d'une colonne de silice (QIAquick PCR Purification Kit, Qiagen, France), puis soumis à l'action des enzymes de restriction Stu I et EcoR I. Après inactivation des enzymes de restriction par la chaleur (20 min à 65°C), le fragment de gène VP1 a été purifié par dialyse contre H₂O sur filtre de 0,025 µm (VSWP01300, Millipore).

Le plasmide pBacPAK8 (Clontech, France) est soumis à l'action des enzymes de restriction Stu I et EcoR I, le vecteur est ensuite déphosphorylé par la phosphatase alcaline de crevette (Boehringer, France). Après inactivation des enzymes de restriction par la chaleur (20 min à 65°C), le plasmide a été purifié par QIAquick PCR Purification Kit (Qiagen).

La ligation est réalisée avec 50 ng de plasmide pBacPAK8 et 100 ng de fragment VP1 (préparés comme précédemment décrit) avec la ligase de T4 (Life Technologies, France). Après inactivation de la ligase de T4 par la chaleur (10 min à 65°C), 2 µl de produit de ligation dilué au 1/2 avec de l'eau sont électroporés avec 25 µl de bactéries électrocompétentes (Epicurian Coli Sure Electroporation-Competent cells, Stratagene). Les bactéries électroporées sont immédiatement reprises dans 1 ml de milieu SOC (2% de tryptone, 0,5% d'extraits de levures, 10 mM NaCl, 2,5 mM KCl, 10 mM MgCl₂, 10 mM MgSO₄ et 20 mM de glucose), incubées 1 h à 37°C sous agitation. Puis 10 µl, 100 µl et 890 µl des bactéries transformées sont étalés sur boites de gélose Lennox (10 g/l de peptone, 5 g/l d'extraits de levures, 5 g/l NaCl, et 13 g/l agar) contenant 50 µg/ml d'ampicilline. Après 24h d'incubation à 37°C, 24 colonies par constructions sont repiquées dans 5 ml de milieu Lennox avec 50 µg/ml d'ampicilline et incubées 24 h à 37°C sous agitation.

L'ADN plasmidique est extrait par minilyse alcaline à l'aide du kit QIAprep 8 Turbo miniprep (Qiagen) et analysé par restriction Stu I/EcoR I et Kpn I/Hind III afin de déterminer la présence de l'insert et son orientation. Le clone pB8-VP1.C5 a été sélectionné et le plasmide recombinant a été vérifiée par séquençage.
- clonage du gène VP2 dans un plasmide bactérien

Le gène VP2 de V9 est amplifié par PCR selon la méthode décrite par Saiki et al. (Nature, 1986, 324:163-166) avec 10 µl d'une dilution 10⁻² d'ADN viral V9 dans un volume final de 100 µl de mélange réactionnel (20 mM Tris-HCl pH 8,8; 10 mM KCl, 10 mM (NH₄)₂SO₄; 2 mM MgSO₄; 0,1% Triton X-100; 0,1 mg/ml de BSA; 0,2 mM dNTP; 25 pmoles d'amorces sens (e3115fBamHI : CACGGATCCATACCCCAGCATGACTTCAG, SEQ ID NO:119) et anti-sens (e4813rBamHI: CACGGATCCGGTGGGTGACGGTTCCTG, SEQ ID NO:120)) avec 2,5 U de Pfu Turbo™ (Stratagene, France). Les amorces d'amplification ont été choisies sur la séquence V9 de part et d'autre du gène VP2, leur extrémité 5' a été modifiée par addition de site(s) de restriction (indiqués dans leur dénomination) pour faciliter le clonage. Les cycles de températures imposés au mélange réactionnel sont les suivants :
1 cycle :
   - 1 minute à 94°C
20 cycles :
   - 1 minute à 94°C
   - 1 minute à 60°C
   - 2,5 minute à 72°C
1 cycle :
   - 10 minute à 72°C

Le produit d'amplification du gène VP2 a été purifié à l'aide d'une colonne de silice (QlAquick PCR Purification Kit, Qiagen, France), puis soumis à l'action de l'enzymes de restriction BamH I. Le fragment de gène VP2 a été purifié par QIAquick PCR Purification Kit (Qiagen).

Le plasmide pBacPAK8 (Clontech, France) est soumis à l'action de l'enzymes de restriction BamH I, le vecteur est ensuite déphosphorylé par la phosphatase alcaline de crevette (Boehringer, France). Après inactivation de la phosphatase alcaline de crevette par la chaleur (20 min à 65°C), le plasmide a été purifié par extraction au phénol/chloroforme et précipité à l'éthanol.

La ligation est réalisée avec 50 ng de plasmide pBacPAK8 et 100 ng de fragment VP2 (préparés comme précédemment décrit) avec la ligase de T4 (Life Technologies, France). Après inactivation de la ligase de T4 par la chaleur (10 min à 65°C), 2 µl de produit de ligation dilué au 1/2 avec de l'eau sont électroporés avec 25 µl de bactéries électrocompétentes (Epicurian Coli Sure Electroporation-Competent cells, Stratagene). Les bactéries électroporées sont immédiatement reprises dans 1 ml de milieu SOC, incubées 1h à 37°C sous agitation. Puis 10 µl, 100 µl et 890 µl des bactéries transformées sont étalés sur boites de gélose Lennox contenant 50 µg/ml d'ampicilline. Après 24h d'incubation à 37°C, 24 colonies par constructions sont repiquées dans 5 ml de milieu Lennox avec 50 µg/ml d'ampicilline et incubées 24 h à 37°C sous agitation.

L'ADN plasmidique est extrait par minilyse alcaline à l'aide du kit QIAprep 8 Turbo miniprep (Qiagen) et analysé par restriction BamH I et Sac I afin de déterminer la présence de l'insert et son orientation. Le clone pB8-VP2.C20 a été sélectionné et le plasmide recombinant a été vérifiée par séquençage: on note une base A délétée juste en amont de l'ATG initiateur de VP2, mais cette mutation peut être négligée: elle ne générera pas l'expression de VP2.

Deuxième étape :
- Construction du baculovirus recombinant exprimant VP1

Le plasmide pB8-VP1.C5 est cotransfecté avec le baculovirus BacPAk6 linéarisé par Bsu361 (BacPAK™ Baculovirus Expression System, Clontech) dans des cellules d'insecte SF9 par la lipofectine. 2 isolements sont effectués par la méthode des plages de lyse, les plages isolées sont transférées sur une membrane de nitro-cellulose, la membrane est ensuite hybridée avec une sonde ADN spécifique du gène VP1 du V9.

Le baculovirus recombinant BacPAK6-pB8-VP1.C4.2 a ainsi été sélectionné. Par *Western-blot* sur un culot cellulaire de cellules SF9 infectées par ce baculovirus recombinant, l'expression de la protéine VP1 a été vérifiée. On observe une bande à la taille attendue de VP1 (environ 80 kDa) mais qui n'est pas reconnue avec l'anticorps monoclonal anti-VP1-B19 (Argène, France). Il est possible que cet anticorps monoclonal ne donne pas de réaction croisée avec la protéine VP1 du V9.

Le clonage en baculovirus a été vérifié par séquençage après PCR par les amorces Bac1 et Bac2 (Clontech).

### - Construction du baculovirus recombinant exprimant VP2

Le plasmide pB8-VP2.C20 est cotransfecté avec le baculovirus BacPAk6 linéarisé par Bsu361 (BacPAK™ Baculovirus Expression System, Clontech) dans des cellules d'insecte SF9 par la lipofectine. 2 isolements sont effectués par la méthode des plages de lyse, les plages isolées sont transférées sur une membrane de nitro-cellulose, la membrane est ensuite hybridée avec une sonde ADN spécifique du gène VP2 du V9.

Le baculovirus recombinant BacPAK6-pB8-VP2.C1.3 est sélectionné. Par *Western*-*blot* sur un culot cellulaire de cellules SF9 infectées par ce baculovirus recombinant, l'expression de la protéine VP2 a été vérifiée. L'anticorps monoclonal anti-VP2-B19 (Argène, France) détecte bien une protéine d'environ 58 kDa de poids moléculaire apparent qui est également bien visible sur le gel d'acrylamide. On observe en microscopie électronique des pseudoparticules virales d'environ 20 à 30 nm de diamètre dans les surnageants de culture des cellules SF9 après infection par un baculovirus recombinant exprimant la protéine VP2 du V9. La taille et l'aspect des pseudoparticules virales obtenues sont en tout point conformes à ceux décrits pour le B19. Cette observation confirme que la protéine VP2 du V9 est produite sous forme native par le baculovirus, car capable de former des capsides vides par auto-assemblage.

Le clonage en baculovirus a été vérifié par séquençage après PCR par les amorces Bac1 et Bac2 (Clontech).

Troisième étape :
Les protéines VP1 et VP2 de V9 exprimées en baculovirus seront purifiées afin d'être utilisées comme antigène cible pour de nouveaux tests sérologiques pour le diagnostic des infections à érythrovirus V9.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Érythrovirus variant par rapport à l'érythrovirus de type B 19, dénommé érythrovirus de type V9, **caractérisé en ce qu'**il présente une divergence génétique < ou égale à 6 % avec la séquence SEQ ID NO :1, **en ce que** son génome s'hybride spécifiquement en conditions stringentes avec l'une des séquences SEQ ID NO:45 à 80, 108 et 110 et **en ce que** ledit érythrovirus V9 ne peut être reconnu moléculairement comme un génome d'érythrovirus B19, car présentant une divergence génétique supérieure ou égale à 10 % sur l'ensemble du génome par rapport aux séquences de l'érythrovirus B19.

2. Polynucléotide, **caractérisé en ce qu'**il est sélectionné dans le groupe constitué par :
a) le génome de l'érythrovirus de type V9 tel que défini à la revendication 1, lequel génome présente une séquence sélectionnée dans le groupe constitué par :
- les séquences présentant une divergence génétique ≥ 10 % sur l'ensemble du génome par rapport aux séquences de l'érythrovirus B19 et présentant une divergence génétique inférieure ou égale à 6 % par rapport à la SEQ ID NO: 1, et
- les séquences capables de s'hybrider dans des conditions stringentes avec l'une des séquences SEQ ID NO: 45 à 80, 108 et 110, et
b) le polynucléotide de séquence SEQ ID NO : 1 représentant 95 % du génome de l'érythrovirus V9 tel que défini à la revendication 1, incluant toutes les séquences codantes.

3. Polynucléotide selon la revendication 2, **caractérisé en ce qu'**il présente un profil de restriction selon les figures 7.1 à 7.3.

4. Fragment de la séquence SEQ ID NO: 1, **caractérisé en ce qu'**il représente une séquence nucléotidique codant pour une protéine dudit érythrovirus V9, sélectionnée dans le groupe constitué par les séquences SEQ ID NO: 81, SEQ ID NO: 83, SEQ ID NO: 85, SEQ ID NO: 87, SEQ ID NO: 89, SEQ ID NO: 91 et SEQ ID NO: 93.

5. Fragment de la séquence SEQ ID NO:1, **caractérisé en ce qu'**il est sélectionné dans le groupe constitué par les séquences SEQ ID NO:45-80, 108 et NO:110, leurs séquences complémentaires, les séquences d'au moins 17 nucléotides, issues de ces séquences et les séquences comprenant lesdites séquences et **en ce qu'**il est apte à servir de sonde ou d'amorce dans l'identification spécifique d'un érythrovirus de type V9 ou d'un érythrovirus présentant une divergence génétique inférieure ou égale à 6% avec la séquence SEQ ID NO: 1.

6. Fragment de la séquence SEQ ID NO:1, **caractérisé en ce qu'**il est sélectionné dans le groupe constitué par les séquences SEQ ID NO: 2 à 44, 105 à 107, 109, et 111 à 121 et les séquences complémentaires des séquences précédentes, et **en ce qu'**il est apte à servir de sonde ou d'amorce pour le diagnostic différentiel des érythrovirus.

7. Paires d'amorces, **caractérisées en ce qu'**elles sont sélectionnées dans le groupe constitué par :
- la paire A : amorces SEQ ID NO: 111 et SEQ ID NO: 112 ;
- la paire B : amorces SEQ ID NO: 105 et SEQ ID NO: 106 ;
- la paire C : l'une des séquences SEQ ID NO: 2-44, 105, 106, 107, 109, 111 ou 112 et l'une des séquences SEQ ID NO:45-80, 108 ou 110 ;
- la paire D : amorce SEQ ID NO: 107 et amorce SEQ ID NO: 109 ;
- la paire E : deux amorces sélectionnées parmi les séquences SEQ ID NO: 2-44, 105, 106, 107, 109, 111 ou 112 ; et
- la paire F : deux amorces sélectionnées parmi les séquences SEQ ID NO: 45-80, 108 ou 110.

8. Sondes, **caractérisées en ce qu'**elles sont sélectionnées dans le groupe constitué par les séquences SEQ ID NO : 45 à 80, 108, 110 et 121.

9. Plasmide, **caractérisé en ce qu'**il comprend un polynucléotide selon la revendication 2 ou la revendication 3 ou un fragment selon l'une quelconque des revendications 4 à 6.

10. Plasmide selon la revendication 9, **caractérisé en ce qu'**il inclut la séquence SEQ ID NO:1.

11. Utilisation d'une molécule d'acide nucléique sélectionnée parmi les séquences SEQ ID NO:45-80, 108 et 110, leurs séquences complémentaires, les séquences d'au moins 17 nucléotides, issues de ces séquences, éventuellement marquées avec un marqueur approprié, pour la préparation d'un réactif de diagnostic pour la détection différentielle des érythrovirus de type V9.

12. Procédé de diagnostic rapide et différentiel des érythrovirus, par hybridation et/ou amplification génique, réalisé à partir d'un échantillon biologique, lequel procédé est **caractérisé en ce qu'**il comprend :
(1) une étape dans laquelle l'on met en contact un échantillon biologique à analyser avec au moins une sonde de séquence SEQ ID NO:45-80, 108 ou 110 et
(2) une étape dans laquelle on détecte par tout moyen approprié le ou les produits résultant de l'interaction séquence nucléotidique d'érythrovirus-sonde.

13. Procédé selon la revendication 12, **caractérisé en ce qu'**il comprend, préalablement à l'étape (1) :
. une étape d'extraction de l'acide nucléique à détecter, appartenant au génome du virus, éventuellement présent dans l'échantillon biologique, et
. au moins un cycle d'amplification génique.

14. Procédé selon la revendication 13, **caractérisé en ce que** les cycles d'amplification sont réalisés à l'aide d'une paire d'amorces selon la revendication 7.

15. Procédé de diagnostic rapide et différentiel des érythrovirus, réalisé à partir d'un échantillon biologique, lequel procédé est **caractérisé en ce qu'**il comprend :
. une étape d'extraction de l'acide nucléique à détecter, appartenant au génome du virus, éventuellement présent dans l'échantillon biologique,
. au moins un cycle d'amplification génique à l'aide de la paire d'amorces B telle que définie à la revendication 7 et
. la détection du produit amplifié d'une part par hybridation avec la sonde de séquence SEQ ID NO:121 et d'autre part par action de l'enzyme de restriction Mun I.

16. Procédé de diagnostic rapide et différentiel des érythrovirus, réalisé à partir d'un échantillon biologique, lequel procédé est **caractérisé en ce qu'**il comprend :
. une étape d'extraction de l'acide nucléique à détecter, appartenant au génome du virus, éventuellement présent dans l'échantillon biologique,
. au moins un cycle d'amplification génique à l'aide de la paire d'amorces A telle que définie à la revendication 7 et
. la détection du produit amplifié par action de l'enzyme ApaI.

17. Procédé de diagnostic rapide et différentiel des érythrovirus, réalisé à partir d'un échantillon biologique, lequel procédé est **caractérisé en ce qu'**il comprend :
. une étape d'extraction de l'acide nucléique à détecter, appartenant au génome du virus, éventuellement présent dans l'échantillon biologique,
. au moins un cycle d'amplification génique à l'aide de la paire d'amorces C telle que définie à la revendication 7 et
. la détection du produit amplifié.

18. Procédé de diagnostic rapide et différentiel des érythrovirus, réalisé à partir d'un échantillon biologique, lequel procédé est **caractérisé en ce qu'**il comprend :
. une étape d'extraction de l'acide nucléique à détecter, appartenant au génome du virus, éventuellement présent dans l'échantillon biologique,
. au moins un cycle d'amplification génique à l'aide de la paire d'amorces D telle que définie à la revendication 7 et
. la détection du produit amplifié par hybridation avec une sonde dont la séquence est sélectionnée dans le groupe constitué par les séquences SEQ ID NO:58-60 et 110.

19. Procédé de diagnostic rapide et différentiel des érythrovirus, réalisé à partir d'un échantillon biologique, lequel procédé est **caractérisé en ce qu'**il comprend :
. une étape d'extraction de l'acide nucléique à détecter, appartenant au génome du virus, éventuellement présent dans l'échantillon biologique,
. au moins un cycle d'amplification génique à l'aide de la paire d'amorces E telle que définie à la revendication 7 et
. la détection du produit amplifié par hybridation avec une sonde dont la séquence est sélectionnée dans le groupe constitué par les séquences SEQ ID NO:45-80, 108 et 110.

20. Procédé de diagnostic rapide et différentiel des érythrovirus, réalisé à partir d'un échantillon biologique, lequel procédé est **caractérisé en ce qu'**il comprend :
. une étape d'extraction de l'acide nucléique à détecter, appartenant au génome du virus, éventuellement présent dans l'échantillon biologique,
. au moins un cycle d'amplification génique à l'aide de la paire d'amorces F telle que définie à la revendication 7 et
. la détection du produit amplifié.

21. Utilisation des séquences telles que définies à l'une quelconque des revendications 2 à 6 pour la mise en oeuvre d'un procédé d'hybridation ou d'amplification génique de séquences nucléiques d'érythrovirus pour le diagnostic *in vitro* de l'infection potentielle d'un individu par un érythrovirus.

22. Procédé de criblage et de typage d'un érythrovirus V9 ou apparenté, **caractérisé en ce qu'**il comprend la mise en contact d'une sonde sélectionnée dans le groupe constitué par les fragments selon la revendication 5, éventuellement marquée, avec l'acide nucléique du virus à typer, éventuellement marqué, et la détection de l'hybride acide nucléique-sonde obtenu.

23. Trousse de diagnostic d'érythrovirus, **caractérisée en ce qu'**elle inclut au moins une paire d'amorces selon la revendication 7, et/ou une sonde selon la revendication 8.

## Claims

1. Variant erythrovirus in relation to erythrovirus type B 19, known as erythrovirus type V9, **characterised in that** it presents a genetic divergence < or equal to 6% from the sequence SEQ ID NO: 1, **in that** its genome hybridises specifically under stringent conditions with one of the sequences SEQ ID NO: 45 to 80, 108 and 110 and **in that** said erythrovirus V9 cannot be recognised in molecular terms as a genome of the erythrovirus B19, since it presents a genetic divergence greater than or equal to 10% over the whole of the genome compared to the sequences of the erythrovirus B19.

2. Polynucleotide, **characterised in that** it is selected from the group constituted of:
a) the genome of the erythrovirus of type V9 as defined in claim 1, this genome presenting a sequence selected from the group constituted of:
- sequences presenting a genetic divergence ≥ 10% over the whole of the genome compared to the sequences of the erythrovirus B 19 and presenting a genetic divergence less than or equal to 6% compared to SEQ ID NO: 1, and
- sequences capable of hybridising under stringent conditions with one of the sequences SEQ ID NO: 45 to 80, 108 and 110, and
b) the polynucleotide of sequence SEQ ID NO: 1 representing 95% of the genome of the erythrovirus V9 as defined in claim 1, including all the coding sequences.

3. Polynucleotide according to claim 2, **characterised in that** it presents a restriction profile according to figures 7.1 to 7.3.

4. Fragment of the sequence SEQ ID NO: 1, **characterised in that** it represents a nucleotide coding for a protein of said erythrovirus V9, selected from the group constituted of sequences SEQ ID NO: 81, SEQ ID NO: 83, SEQ ID NO: 85, SEQ ID NO: 87, SEQ ID NO: 89, SEQ ID NO: 91 or SEQ ID NO: 93.

5. Fragment of the sequence SEQ ID NO: 1, **characterised in that** it is selected from the group constituted of sequences SEQ ID NO: 45-80, 108 and NO: 110, their complementary sequences, sequences of at least 17 nucleotides, resulting from these sequences, and sequences comprising said sequences, and **in that** it is suitable for use as a probe or primer in the specific identification of an erythrovirus of type V9 or an erythrovirus presenting a genetic divergence less than or equal to 6% compared to SEQ ID NO: 1.

6. Fragment of the sequence SEQ ID NO: 1, **characterised in that** it is selected from the group constituted of sequences SEQ ID NO: 2 to 44, 105 to 107, 109, and 111 to 121 and the complementary sequences to the preceding sequences, and **in that** it is suitable for use as a primer or probe for the differential diagnosis of erythroviruses.

7. Pairs of primers, **characterised in that** they are selected from the group constituted of:
- pair A: primers SEQ ID NO: 111 and SEQ ID NO: 112;
- pair B: primers SEQ ID NO: 105 and SEQ ID NO: 106;
- pair C: one of sequences SEQ ID NO: 2-44, 105, 106, 107, 109, 111 or 112 and one of sequences SEQ ID NO: 45-80, 108 or 110;
- pair D: primer SEQ ID NO: 107 and primer SEQ ID NO: 109;
- pair E: two primers selected from among sequences SEQ ID NO: 2-44, 105, 106, 107, 109, 111 or 112; and
- pair F: two primers selected from among sequences SEQ ID NO: 45-80, 108 or 110.

8. Probes, **characterised in that** they are selected from the group constituted of the sequences SEQ ID NO: 45 to 80, 108, 110 and 121.

9. Plasmid, **characterised in that** it comprises a polynucleotide according to claim 2 or claim 3 or a fragment according to any one of claims 4-6.

10. Plasmid according to claim 9, **characterised in that** it includes the sequence SEQ ID NO: 1.

11. Use of a nucleic acid molecule selected from among sequences SEQ ID NO: 45-80, 108 and 110, their complementary sequences, sequences of at least 17 nucleotides, resulting from these sequences, possibly marked with an appropriate marker, for the preparation of a diagnostic reagent for the differential detection of erythroviruses of type V9.

12. Method for the rapid and differential diagnosis of erythroviruses, by means of hybridisation and/or gene amplification, carried out on a biological sample, said method being **characterised in that** it comprises:
(1) a step in which the biological sample to be analysed is placed in contact with at least one probe of sequence SEQ ID NO: 45-80, 108 or 110 and
(2) a step in which the product(s) resulting from the interaction between the nucleotide sequence of the erythrovirus and the probe is detected by any appropriate means.

13. Method according to claim 12, **characterised in that** it comprises, prior to step (1):
. a step of extracting the nucleic acid to be detected, belonging to the virus genome, possibly present in the biological sample, and
. at least one gene amplification cycle.

14. Method according to claim 13, **characterised in that** the amplification cycles are carried out using a pair of primers according to claim 7.

15. Method for the rapid and differential diagnosis of erythroviruses, carried out on a biological sample, said method being **characterised in that** it comprises:
. a step of extracting the nucleic acid to be detected, belonging to the virus genome, possibly present in the biological sample,
. at least one gene amplification cycle using the pair of primers B as defined in claim 7, and
. the detection of the amplified product, partly through hybridisation with the probe of SEQ ID NO: 121, and partly through the action of the restriction enzyme Mun I.

16. Method for the rapid and differential diagnosis of erythroviruses, carried out on a biological sample, said method being **characterised in that** it comprises:
. a step of extracting the nucleic acid to be detected, belonging to the virus genome, possibly present in the biological sample,
. at least one gene amplification cycle using the pair of primers A as defined in claim 7, and
. the detection of the amplified product through the action of the enzyme A pal.

17. Method for the rapid and differential diagnosis of erythroviruses, carried out on a biological sample, said method being **characterised in that** it comprises:
. a step of extracting the nucleic acid to be detected, belonging to the virus genome, possibly present in the biological sample,
. at least one gene amplification cycle using the pair of primers C as defined in claim 7, and
. the detection of the amplified product.

18. Method for the rapid and differential diagnosis of erythroviruses, carried out on a biological sample, said method being **characterised in that** it comprises:
. a step of extracting the nucleic acid to be detected, belonging to the virus genome, possibly present in the biological sample,
. at least one gene amplification cycle using the pair of primers D as defined in claim 7, and
. the detection of the amplified product through hybridisation with a probe, the sequence of which is selected from the group constituted of the sequences SEQ ID NO: 5 8-60 and 110.

19. Method for the rapid and differential diagnosis of erythroviruses, carried out on a biological sample, said method being **characterised in that** it comprises:
. a step of extracting the nucleic acid to be detected, belonging to the virus genome, possibly present in the biological sample,
. at least one gene amplification cycle using the pair of primers E as defined in claim 7, and
. the detection of the amplified product through hybridisation with a probe, the sequence of which is selected from the group constituted of the sequences SEQ ID NO: 45-80, 108 and 110.

20. Method for the rapid and differential diagnosis of erythroviruses, carried out on a biological sample, said method being **characterised in that** it comprises:
. a step of extracting the nucleic acid to be detected, belonging to the virus genome, possibly present in the biological sample,
. at least one gene amplification cycle using the pair of primers F as defined in claim 7, and
. the detection of the amplified product.

21. Use of the sequences as defined in any one of claims 2 to 6 for the implementation of a hybridisation or gene amplification process of nucleotide sequences of erythroviruses for the *in vitro* diagnosis of an individual's potential infection with an erythrovirus.

22. Method for the screening and typing of an erythrovirus V9 or related erythrovirus, **characterised in that** it comprises the placing of a probe selected from a group constituted of the fragments according to claim 5, possibly marked, in contact with the nucleic acid of the virus to be typed, possibly marked, and the detection of the nucleic acid-probe hybrid obtained.

23. Erythrovirus diagnostic kit, **characterised in that** it includes at least one pair of primers according to claim 7, and/or a probe according to claim 8.

## Patentansprüche

1. Erythrovirusvariante bezüglich des Erythrovirus vom Typ B 19, das als Erythrovirus vom Typ V9 bezeichnet wird, **dadurch gekennzeichnet, dass** sie eine genetische Divergenz von < oder gleich 6 % mit der Sequenz SEQ ID NO:1 aufweist, **dadurch**, dass ihr Genom unter Stringenzbedingungen spezifisch mit einer der Sequenzen SEQ ID NO:45 bis 80, 108 und 110 hybridisiert, und **dadurch**, dass das Erythrovirus V9 molekular nicht als ein Erythrovirus B19-Genom erkannt werden kann, da es eine genetische Divergenz von größer oder gleich 10 % der Gesamtheit des Genoms bezüglich der Sequenzen des Erythrovirus B19 aufweist.

2. Polynucleotid, **dadurch gekennzeichnet, dass** es aus der Gruppe ausgewählt ist, die besteht aus:
a) dem Genom des Erythrovirus vom Typ V9, wie in Anspruch 1 definiert, wobei das Genom eine Sequenz aufweist, die aus der Gruppe ausgewählt ist, die besteht aus:
- den Sequenzen, die eine genetische Divergenz von ≥ 10 % der Gesamtheit des Genoms bezüglich der Sequenzen des Erythrovirus B 19 und eine genetische Divergenz von kleiner oder gleich 6 % bezüglich der SEQ ID NO:1 aufweisen, und
- den Sequenzen, die in der Lage sind, unter Stringenzbedingungen mit einer der Sequenzen SEQ ID NO:45 bis 80, 108 und 110 zu hybridisieren, und
b) dem Polynucleotid von Sequenz SEQ ID NO:1, das 95 % des Genoms des Erythrovirus V9, wie in Anspruch 1 definiert, darstellt, das sämtliche codierenden Sequenzen umfasst.

3. Polynucleotid nach Anspruch 2, **dadurch gekennzeichnet, dass** es ein Restriktionsprofil gemäß Figuren 7.1 bis 7.3 aufweist.

4. Fragment der Sequenz SEQ ID NO:1, **dadurch gekennzeichnet, dass** es eine Nucleotidsequenz darstellt, die ein Protein des Erythrovirus V9 codiert, die aus der Gruppe ausgewählt ist, die aus den Sequenzen SEQ ID NO:81, SEQ ID NO:83, SEQ ID NO: 85, SEQ ID NO:87, SEQ ID NO:89, SEQ ID NO: 91 und SEQ ID NO:93 besteht.

5. Fragment der Sequenz von SEQ ID NO:1, **dadurch gekennzeichnet, dass** es aus der Gruppe ausgewählt ist, die aus den Sequenzen SEQ ID NO:45 bis 80, 108 und NO:110, ihren komplementären Sequenzen, den Sequenzen von mindestens 17 Nucleotiden, die sich von diesen Sequenzen ableiten, und den Sequenzen, die diese Sequenzen einschließen, besteht, und **dadurch**, dass es dazu geeignet ist, als Sonde oder als Primer bei der spezifischen Identifizierung eines Erythrovirus vom Typ V9 oder eines Erythrovirus, der eine genetische Divergenz von kleiner oder gleich 6 % mit der SEQ ID NO: 1 aufweist, zu dienen.

6. Fragment der Sequenz SEQ ID NO:1, wie in Anspruch 2 definiert, **dadurch gekennzeichnet, dass** es aus der Gruppe ausgewählt ist, die aus den Sequenzen SEQ ID NO: 2 bis 44, 105 bis 107, 109 und 111 bis 121 und den komplementären Sequenzen der vorangegangenen Sequenzen besteht, und **dadurch**, dass es dazu geeignet ist, als Sonde oder als Primer zur differentiellen Diagnostik der Erythroviren zu dienen.

7. Primerpaare, **dadurch gekennzeichnet, dass** sie aus der Gruppe ausgewählt sind, die besteht aus
- dem Paar A: Primer SEQ ID NO:111 und SEQ ID NO:112;
- dem Paar B: Primer SEQ ID NO:105 und SEQ ID NO:106;
- dem Paar C: eine der Sequenzen SEQ ID NO:2-44, 105, 106, 107, 109, 111 oder 112 und eine der Sequenzen SEQ ID NO:45-80, 108 oder 110;
- dem Paar D: Primer SEQ ID NO:107 und Primer SEQ ID NO:109;
- dem Paar E: zwei Primer, die aus den Sequenzen SEQ ID NO:2-44, 105, 106, 107, 109, 111 oder 112 ausgewählt sind; und
- dem Paar F: zwei Primer, die aus den Sequenzen SEQ ID NO:45-80, 108 oder 110 ausgewählt sind.

8. Sonden, die **dadurch gekennzeichnet sind, dass** sie aus der Gruppe ausgewählt sind, die aus den Sequenzen SEQ ID NO:45 bis 80, 108, 110 und 121 besteht.

9. Plasmid, **dadurch gekennzeichnet, dass** es ein Polynucleotid nach Anspruch 2 oder Anspruch 3 oder ein Fragment nach einem der Ansprüche 4 bis 6 einschließt.

10. Plasmid nach Anspruch 9, **dadurch gekennzeichnet, dass** es die Sequenz SEQ ID NO:1 einschließt.

11. Verwendung eines Nukleinsäuremoleküls, das ausgewählt ist aus den Sequenzen SEQ ID NO:45 bis 80, 108 und 110, ihren komplementären Sequenzen, den Sequenzen von mindestens 17 Nucleotiden, die von diesen Sequenzen abgeleitet sind, gegebenenfalls markiert mit einem entsprechenden Marker, zur Herstellung eines diagnostischen Reagenz zum differentiellen Nachweis des Erythrovirus vom Typ V9.

12. Schnelles und differentielles Verfahren zur Diagnose der Erythroviren durch Gen-Hybridisierung und/oder Gen-Amplifikation, das mit einer biologischen Probe durchgeführt wird, wobei das Verfahren **dadurch gekennzeichnet ist, dass** es umfasst:
(1) einen Schritt, wobei eine biologische, zu analysierende Probe mit mindestens einer Sonde der Sequenz SEQ ID NO:45 bis 80, 108 oder 110 in Kontakt gebracht wird, und
(2) einen Schritt, wobei durch jedes geeignete Mittel das oder die Produkte nachgewiesen werden, die aus der Wechselwirkung Erythrovirusnucleotidsequenz-Sonde hervorgehen.

13. Verfahren nach Anspruch 12, **dadurch gekennzeichnet, dass** es vor Schritt (1) umfasst:
einen Schritt der Extraktion der nachzuweisenden Nucleinsäure, die dem Genom des Virus gehört, das gegebenenfalls in der biologischen Probe vorhanden ist;
und
mindestens einen Gen-Amplifikationszyklus.

14. Verfahren nach Anspruch 13, **dadurch gekennzeichnet, dass** die Amplifikationszyklen mit Hilfe eines Paares von Primem nach Anspruch 7 durchgeführt werden.

15. Schnelles und differentielles Verfahren zur Diagnose der Erythroviren, das mit einer biologischen Probe durchgeführt wird, wobei das Verfahren **dadurch gekennzeichnet ist, dass** es umfasst:
· einen Schritt der Extraktion der nachzuweisenden Nucleinsäure, die dem Genom des Virus gehört, das gegebenenfalls in der biologischen Probe vorhanden ist,
· mindestens einen Gen-Amplifikationszyklus mit Hilfe des Paares von Primem B, wie in Anspruch 7 definiert, und
· den Nachweis des amplifizierten Produkts einerseits durch Hybridisierung mit der Sonde von Sequenz SEQ ID NO:121 und andererseits durch Wirkung des Restriktionsenzyms Mun I.

16. Schnelles und differentielles Verfahren zur Diagnose der Erythroviren, das mit einer biologischen Probe durchgeführt wird, wobei das Verfahren **dadurch gekennzeichnet ist, dass** es umfasst:
· einen Schritt der Extraktion der nachzuweisenden Nucleinsäure, die dem Genom des Virus gehört, das gegebenenfalls in der biologischen Probe vorhanden ist,
· mindestens einen Gen-Amplifikationszyklus mit Hilfe des Paares von Primem A, wie in Anspruch 7 definiert, und
· den Nachweis des amplifizierten Produkts durch Wirkung des Restriktionsenzyms Apa I.

17. Schnelles und differentielles Verfahren zur Diagnose der Erythroviren, das mit einer biologischen Probe durchgeführt wird, wobei das Verfahren **dadurch gekennzeichnet ist, dass** es umfasst:
· einen Schritt der Extraktion der nachzuweisenden Nucleinsäure, die dem Genom des Virus gehört, das gegebenenfalls in der biologischen Probe vorhanden ist,
· mindestens einen Gen-Amplifikationszyklus mit Hilfe des Paares von Primem C, wie in Anspruch 7 definiert, und
· den Nachweis des amplifizierten Produkts.

18. Schnelles und differentielles Verfahren zur Diagnose der Erythroviren, das mit einer biologischen Probe durchgeführt wird, wobei das Verfahren **dadurch gekennzeichnet ist, dass** es umfasst:
· einen Schritt der Extraktion der nachzuweisenden Nucleinsäure, die dem Genom des Virus gehört, das gegebenenfalls in der biologischen Probe vorhanden ist,
· mindestens einen Gen-Amplifikationszyklus mit Hilfe des Paares von Primem D, wie in Anspruch 7 definiert, und
· den Nachweis des amplifizierten Produkts durch Hybridisierung mit einer Sonde, deren Sequenz aus der Gruppe ausgewählt ist, die aus den Sequenzen SEQ ID NO:58-60 und 110 besteht.

19. Schnelles und differentielles Verfahren zur Diagnose der Erythroviren, das mit einer biologischen Probe durchgeführt wird, wobei das Verfahren **dadurch gekennzeichnet ist, dass** es umfasst:
· einen Schritt der Extraktion der nachzuweisenden Nucleinsäure, die dem Genom des Virus gehört, das gegebenenfalls in der biologischen Probe vorhanden ist,
· mindestens ein Gen-Amplifikationszyklus mit Hilfe des Paares von Primem E, wie in Anspruch 7 definiert, und
· den Nachweis des amplifizierten Produkts durch Hybridisierung mit einer Sonde, deren Sequenz aus der Gruppe ausgewählt ist, die aus den Sequenzen SEQ ID NO:45-80, 108 und 110 besteht.

20. Schnelles und differentielles Verfahren zur Diagnose der Erythroviren, das mit einer biologischen Probe durchgeführt wird, wobei das Verfahren **dadurch gekennzeichnet ist, dass** es umfasst:
· einen Schritt der Extraktion der nachzuweisenden Nucleinsäure, die dem Genom des Virus gehört, das gegebenenfalls in der biologischen Probe vorhanden ist,
· mindestens einen Gen-Amplifikationszyklus mit Hilfe des Paares von Primern F, wie in Anspruch 7 definiert, und
· den Nachweis des amplifizierten Produkts.

21. Verwendung der Sequenzen, definiert nach einem der Ansprüche 2-6, zur Durchführung eines Gen-Hybridisierungs- oder -Amplifikationsverfahrens von Erythrovirus-Nucleinsäuresequenzen zur *in vitro* Diagnose der potentiellen Infektion eines Individuums durch ein Erythrovirus.

22. Scan- und Charakterisierungsverfahren eines Erythrovirus V9 oder eines verwandten Virus, **dadurch gekennzeichnet, dass** es das Zusammenbringen einer Sonde, die aus der Gruppe ausgewählt ist, die aus den Fragmenten nach Anspruch 5, gegebenenfalls markiert, besteht, mit der Nucleinsäure des zu charakterisierenden Virus, gegebenenfalls markiert, und den Nachweis des erhaltenen Hybrids Nucleinsäure-Sonde umfasst.

23. Diagnostischer Erythrovirus-Testsatz, **dadurch gekennzeichnet, dass** er mindestens ein Paar von Primem nach Anspruch 7 und/oder eine Sonde nach Anspruch 8 einschließt.


### LISTAGE DES SEQUENCES

<110> NGUYEN, Quang Tri
   GARBARG-CHENON, Antoine
   AUGUSTE, Véronique
   ASSISTANCE PUBLIQUE-HOPITAUX DE PARIS
<120> ERYTHROVIRUS HUMAIN, FRAGMENTS DUDIT VIRUS AINSI QUE LEURS APPLICATIONS
<130> BLOcp1020/3P
<140>
   <141>
<150> FR9715197
   <151> 1997-12-03
<160> 121
<170> PatentIn Vers. 2.0
<210> 1
   <211> 5028
   <212> ADN
   <213> erythrovirus
<400> 1
   GACGTCACAG GAAATGACGT AACTGTCCGC CATCTTGTAC CGGAAGTCCC GCCTACCGGC    60
   GGCGACCGGC GGCATCTGAT TTGGTGTCTT CTTTTTGAAA TTTTGGCGGG CTTTTTCCCG   120
   CCTTATGCAA ATAAGCGGCC ATGTTTAATG TTATATTTTA ATTTAATTGG ACAAACGCCT   180
   AACGGTTACT AGGGGCGGAG TTACGGGCGG TATATAAGCA GCTGCGTTCC CTGACACTTT   240
   CTTTTCTGGT TGCTTTTGAC TGGAACTCAC TTGCTGTTCT TTGCCTGCTA AGTAACAGGT   300
   ATTTATACTA ACTTTTAATT TACTAACATG GAGCTATTTC GGGGTGTCTT GCACATTTCC   360
   TCTAACATTC TGGACTGTGC TAATGATAAC TGGTGGTGCT CTATGCTAGA CTTAGATACT   420
   TCTGACTGGG AACCACTAAC CCATTCTAAC AGATTAATGG CAATATATTT AAGCAGTGTT   480
   GCTTCTAAAC TTGATTTTAC TGGGGGGCCG CTAGCAGGTT GCTTATACTT TTTTCAGGTG   540
   GAATGTAACA AATTTGAGGA AGGCTATCAT ATCCATGTAG TTATTGGTGG TCCAGGACTA   600
   AATGCTAGAA ACTTAACTGT GTGCGTAGAA GGTTTATTTA ATAATGTTCT TTACCATCTT   660
   GTAACTGAAA GTGTTAAACT TAAATTTTTG CCAGGGATGA CTACCAAAGG AAAATATTTT   720
   AGAGATGGAG AGCAGTTTAT AGAAAATTAC TTAATGAAAA AAATTCCTTT AAATGTTGTG   780
   TGGTGTGTAA CAAATATTGA CGGGTATATA GACACCTGTA TTTCCGCCTC TTTTCGGCGA   840
   GGAGCTTGTC ATGCTAAAAG ACCCCGCATT ACTGCAAATA CAGACAGTGC TACTAATGAA   900
   ACTGGGGAGT CTAGCTGTGG AGGGGGAGAT GTTGTGCCAT TCGCTGGAAA GGGAACAAAA   960
   GCGGGGTTAA AGTTTCAAAC CATGGTAAAT TGGCTATGTG AAAACAGAGT ATTTACTGAA  1020
   GATAAATGGA AATTAGTGGA TTTTAACCAA TATACTTTAT TAAGTAGCAG TCACAGTGGC  1080
   AGCTTTCAAA TTCAAAGTGC CTTAAAGTTA GCTATTTATA AAGCTACTAA CTTAGTACCC  1140
   ACTAGTACAT TCTTGTTACA TTCAGACTTT GAGCAGGTTA CTTGCATTAA AGAAAATAAA  1200
   ATAGTAAAAT TATTATTGTG TCAAAACTAT GATCCTCTTT TAGTGGGTCA ACATGTGTTA 1260
   AGGTGGATTG ACAAAAAATG TGGTAAAAAA AACACCCTGT GGTTTTACGG GCCACCAAGT 1320
   ACTGGAAAAA CAAATTTGGC AATGGCTATT GCTAAAACTG TACCAGTGTA TGGAATGGTG 1380
   AATTGGAATA ATGAAAACTT TCCATTTAAT GATGTAGCGG GGAAAAGTTT GGTGGTCTGG 1440
   GATGAAGGCA TTATTAAGTC CACTATTGTG GAAGCTGCAA AAGCCATTTT AGGTGGTCAG 1500
   CCAACCAGGG TAGATCAGAA AATGCGTGGC AGTGTGGCAG TGCCCGGTGT GCCTGTGGTT 1560
   ATAACCAGCA ATGGTGACAT TACATTTGTT GTGAGTGGTA ATACCACTAC AACTGTGCAT 1620
   GCTAAAGCCT TAAAGGAACG GATGGTAAAG CTAAACTTTA CCATAAGATG TAGCCCTGAC 1680
   ATGGGTTTAC TTACAGAGGC TGATGTACAA CAATGGCTAA CTTGGTGTAA TGCACAAAGC 1740
   TGGAGCCACT ATGAAAACTG GGCAATAAAC TACACATTTG ATTTCCCTGG AATAAATGCA 1800
   GATGCCCTCC ACCCAGATCT CCAAACCACC CCCATTGTCC CAGACACCAG TATCAGCAGC 1860
   AGTGGTGGTG AAAGCTCTGA AGAACTCAGT GAAAGCAGCT TTTTCAACCT CATCACTCCA 1920
   GGCGCCTGGA ACAGTGAAAC CCCGCGCTCT AGTACGCCCG TCCCCGGGAC CAGTTCAGGA 1980
   GAATCATTTG TCGGAAGCCC AGTTTCCTCC GAAGTGGTAG CCGCGTCGTG GGAGGAAGCT 2040
   TTTTACACGC CGCTTGCCGA TCAGTTTCGT GAACTGTTAG TAGGGGTTGA CTTTGTATGG 2100
   GATGGTGTGA GGGGATTGCC TGTTTGCTGT GTGGAACATA TAAACAACAG TGGGGGAGGG 2160
   TTGGGGCTTT GCCCTCATTG TATTAATGTG GGAGCTTGGT ATAATGGATG GAAATTTAGA 2220
   GAGTTTACTC CAGACTTAGT GCGCTGCAGT TGTCATGTAG GAGCCTCTAA CCCATTTTCT 2280
   GTGTTAACTT GTAAAAAATG TGCTTACCTG TCTGGATTAC AAAGTTTTGT AGATTATGAG 2340
   TAAAACCACT AACAAATGGT GGGAAAGCAG TGACAAATTT GCCCAGGACG TGTATAAGCA 2400
   GTTTGTGCAA TTTTATGAAA AAGCTACTGG AACAGACTTA GAGCTTATTC AAATTTTAAA 2460
   AGACCATTAC AACATTTCTT TAGATAATCC TTTAGAAAAC CCCTCTTCTT TATTTGACTT 2520
   AGTTGCTCGC ATTAAAAGTA ATCTTAAAAA CTCTCCAGAC CTATATAGTC ATCATTTTCA 2580
   GAGCCATGGA CAGTTATCTG ACCACCCCCA TGCCTTATCA TCCAGTAACA GTAGTGCAGA 2640
   ACCTAGAGGA GAAAATGCAG TATTATCTAG TGAAGACTTA CACAAGCCTG GGCAAGTTAG 2700
   CATACAATTA CCCGGTACTA ACTATGTTGG GCCTGGCAAT GAGCTACAAG CTGGGCCTCC 2760
   GCAGAATGCT GTGGACAGTG CTGCAAGGAT TCATGACTTT AGGTATAGCC AATTGGCTAA 2820
   GTTGGGAATA AATCCTTATA CACATTGGAC GGTAGCAGAT GAAGAATTGT TAAAAAATAT 2880
   AAAAAATGAA ACAGGGTTTC AAGCACAAGC AGTAAAAGAT TACTTTACTT TAAAAGGTGC 2940
   AGCTGCCCCT GTGGCCCATT TTCAAGGAAG TTTACCGGAA GTGCCCGCGT ACAACGCCTC 3000
   AGAAAAATAC CCCAGCATGA CTTCAGTTAA CTCTGCAGAA GCCAGCACTG GTGCAGGCGG 3060
   GGGAGGTAGC AACCCTACAA AAAGCATGTG GAGTGAAGGG GCTACATTTA CTGCTAATTC 3120
   TGTAACGTGT ACATTCTCTA GGCAATTTTT AATTCCATAT GATCCAGAGC ATCATTATAA 3180
   AGTGTTCTCT CCAGCAGCTA GTAGCTGCCA CAATGCTAGT GGGAAAGAGG CAAAAGTGTG 3240
   CACTATTAGT CCCATTATGG GGTACTCTAC TCCGTGGAGA TACTTAGATT TTAATGCTTT 3300
   AAATTTGTTT TTCTCACCAT TAGAGTTTCA GCACTTAATT GAAAATTATG GTAGTATAGC 3360
   TCCAGATGCT TTAACTGTAA CTATTTCAGA AATTGCTGTA AAAGATGTCA CAGACAAAAC 3420
   AGGAGGAGGT GTGCAAGTTA CTGACAGCAC CACAGGACGT TTGTGTATGT TAGTGGATCA 3480
   TGAGTATAAA TACCCATATG TGCTAGGTCA GGGACAAGAC ACACTAGCTC CAGAACTGCC 3540
   CATTTGGGTT TACTTTCCCC CCCAGTATGC TTACTTAACA GTAGGTGAAG TAAACACACA 3600
   AGGAATTTCA GGAGACAGCA AAAAATTGGC TAGTGAAGAA TCAGCTTTTT ATGTGTTAGA 3660
   GCACAGTTCA TTTGAACTTT TGGGTACAGG GGGATCTGCC ACTATGTCCT ACAAATTTCC 3720
   AGCTGTGCCC CCAGAAAACC TAGAAGGCTG CAGCCAACAT TTTTATGAAA TGTACAACCC 3780
   TTTGTACGGT TCTCGTTTAG GGGTACCTGA CACATTAGGA GGGGACCCTA AATTTAGATC 3840
   ATTGACACAC GAAGACCACG CAATTCAGCC ACAAAACTTT ATGCCTGGGC CACTAATAAA 3900
   TTCAGTGTCT ACCAAAGAAG GAGACAATTC TAATACAGGT GCTGGAAAAG CCCTTACGGG 3960
   GCTTAGTACT GGCACTAGCC AAAACACCAG AATTTCCCTA CGCCCCGGGC CAGTATCTCA 4020
   GCCATACCAT CACTGGGACA CTGATAAATA TGTTACAGGA ATAAATGCCA TTTCACATGG 4080
   ACAAACCACT TATGGAAATG CTGAGGACAA AGAGTATCAG CAAGGGGTAG GAAGATTTCC 4140
   AAATGAAAAA GAACAGCTTA AGCAGTTACA AGGTCTTAAC ATGCACACAT ACTTCCCTAA 4200
   TAAAGGAACC CAACAATACA CAGACCAAAT TGAACGCCCT CTTATGGTGG GCTCTGTTTG 4260
   GAACAGAAGA GCTCTTCACT ATGAAAGTCA GCTGTGGAGT AAAATCCCTA ACTTAGATGA 4320
   CAGTTTTAAA ACTCAATTTG CAGCCCTAGG CGGGTGGGGT TTGCATCAAC CACCCCCTCA 4380
   AATATTTTTA AAAATACTAC CACAAAGTGG GCCAATTGGA GGTATTAAAT CCATGGGAAT 4440
   TACTACTTTA GTTCAATATG CTGTGGGAAT AATGACAGTT ACCATGACCT TTAAATTGGG 4500
   ACCTCGAAAG GCTACTGGAA GGTGGAATCC CCAGCCTGGC GTTTATCCTC CTCATGCAGC 4560
   TGGTCATTTA CCATATGTAC TGTATGACCC CACAGCTACA GATGCAAAGC AACACCACAG 4620
   ACACGGATAT GAAAAGCCTG AAGAATTGTG GACTGCCAAA AGCCGTGTGC ACCCATTGTA 4680
   AACATTCCCC ACCGTGTCCT CAGCCAGGAA CCGTCACCCA CCGCCCACCT GTGCCGCCCA 4740
   GATTATATGT GCCCCCTCCA ATACCCCGTA GGCAACCATC TATAAAAGAT ACAGACGCTG 4800
   TAGAATATAA ATTATTAACT AGATATGAAC AACATGTAAT TAGAATGCTA AGATTATGTA 4860
   ATATGTACAC AAGTTTGGAA AAATAAAAGC CTTAAATAAA TAATTCATAG TGTATGGTTC 4920
   TTTAAAAATT TCAAAAAGAA GACACCAAAT CAGATGCCGC CGGTCGCCGC CGGTAGGCGG 4980
   GACTTCCGGT ACAAGATGGC GGACAGTTAC GTCATTTCCT GTGACGTC 5028
<210> 2
   <211> 23
   <212> ADN
   <213> erythrovirus
<400> 2
   ACTTCTGACT GGGAACCACT AAC 23
<210> 3
   <211> 29
   <212> ADN
   <213> erythrovirus
<400> 3
   TTTAGAGATG GAGAGCAGTT TATAGAAAA 29
<210> 4
   <211> 35
   <212> ADN
   <213> erythrovirus
<400> 4
   TGGAATAATG AAAACTTTCC ATTTAATGAT GTAGC 35
<210> 5
   <211> 20
   <212> ADN
   <213> erythrovirus
<400> 5
   TTGGTGGTCT GGGATGAAGG 20
<210> 6
   <211> 20
   <212> ADN
   <213> erythrovirus
<400> 6
   ACAGAGGCTG ATGTACAACA 20
<210> 7
   <211> 21
   <212> ADN
   <213> erythrovirus
<400> 7
   TGGTGTAATG CACAAAGCTG G 21
<210> 8
   <211> 31
   <212> ADN
   <213> erythrovirus
<400> 8
   CCACTATGAA AACTGGGCAA TAAACTACAC A 31
<210> 9
   <211> 17
   <212> ADN
   <213> erythrovirus
<400> 9
   TTTGATTTCC CTGGAAT 17
<210> 10
   <211> 23
   <212> ADN
   <213> erythrovirus
<400> 10
   AATGCAGATG CCCTCCACCC AGA 23
<210> 11
   <211> 64
   <212> ADN
   <213> erythrovirus
<400> 11
   CAGACACCAG TATCAGCAGC AGTGGTGGTG AAAGCTCTGA AGAACTCAGT GAAAGCAGCT 60
   TTTT 64
<210> 12
   <211> 25
   <212> ADN
   <213> erythrovirus
<400> 12
   TGAAACCCCG CGCTCTAGTA CGCCC 25
<210> 13
   <211> 55
   <212> ADN
   <213> erythrovirus
<400> 13
   TCCCCGGGAC CAGTTCAGGA GAATCATTTG TCGGAAGCCC AGTTTCCTCC GAAGT 55
<210> 14
   <211> 20
   <212> ADN
   <213> erythrovirus
<400> 14
   CAGTTTCGTG AACTGTTAGT 20
<210> 15
   <211> 24
   <212> ADN
   <213> erythrovirus
<400> 15
   GCTTGGTATA ATGGATGGAA ATTT 24
<210> 16
   <211> 26
   <212> ADN
   <213> erythrovirus
<400> 16
   AAAAAATGTG CTTACCTGTC TGGATT 26
<210> 17
   <211> 18
   <212> ADN
   <213> erythrovirus
<400> 17
   CTTAAAAACT CTCCAGAC 18
<210> 18
   <211> 19
   <212> ADN
   <213> erythrovirus
<400> 18
   TATATAGTCA TCATTTTCA 19
<210> 19
   <211> 43
   <212> ADN
   <213> erythrovirus
<400> 19
   CATGGACAGT TATCTGACCA CCCCCATGCC TTATCATCCA GTA 43
<210> 20
   <211> 19
   <212> ADN
   <213> erythrovirus
<400> 20
   TGCAGAACCT AGAGGAGAA 19
<210> 21
   <211> 47
   <212> ADN
   <213> erythrovirus
<400> 21
   ATGCAGTATT ATCTAGTGAA GACTTACACA AGCCTGGGCA AGTTAGC 47
<210> 22
   <211> 49
   <212> ADN
   <213> erythrovirus
<400> 22
   TACCCGGTAC TAACTATGTT GGGCCTGGCA ATGAGCTACA AGCTGGGCC 49
<210> 23
   <211> 39
   <212> ADN
   <213> erythrovirus
<400> 23
   GACAGTGCTG CAAGGATTCA TGACTTTAGG TATAGCCAA 39
<210> 24
   <211> 22
   <212> ADN
   <213> erythrovirus
<400> 24
   TGGCTAAGTT GGGAATAAAT CC 22
<210> 25
   <211> 23
   <212> ADN
   <213> erythrovirus
<400> 25
   TTAAAAAATA TAAAAAATGA AAC 23
<210> 26
   <211> 53
   <212> ADN
   <213> erythrovirus
<400> 26
   TACTTTACTT TAAAAGGTGC AGCTGCCCCT GTGGCCCATT TTCAAGGAAG TTT 53
<210> 27
   <211> 23
   <212> ADN
   <213> erythrovirus
<400> 27
   TACAACGCCT CAGAAAAATA CCC 23
<210> 28
   <211> 26
   <212> ADN
   <213> erythrovirus
<400> 28
   TCTGCAGAAG CCAGCACTGG TGCAGG 26
<210> 29
   <211> 23
   <212> ADN
   <213> erythrovirus
<400> 29
   TTAGATTTTA ATGCTTTAAA TTT 23
<210> 30
   <211> 32
   <212> ADN
   <213> erythrovirus
<400> 30
   TTAGAGTTTC AGCACTTAAT TGAAAATTAT GG 32
<210> 31
   <211> 20
   <212> ADN
   <213> erythrovirus
<400> 31
   ACAGGAATAA ATGCCATTTC 20
<210> 32
   <211> 24
   <212> ADN
   <213> erythrovirus
<400> 32
   GACAAAGAGT ATCAGCAAGG GGTA 24
<210> 33
   <211> 26
   <212> ADN
   <213> erythrovirus
<400> 33
   AGATTTCCAA ATGAAAAAGA ACAGCT 26
<210> 34
   <211> 18
   <212> ADN
   <213> erythrovirus
<400> 34
   TCAGCTGTGG AGTAAAAT 18
<210> 35
   <211> 23
   <212> ADN
   <213> erythrovirus
<400> 35
   TTAGATGACA GTTTTAAAAC TCA 23
<210> 36
   <211> 21
   <212> ADN
   <213> erythrovirus
<400> 36
   CCTCAAATAT TTTTAAAAAT A 21
<210> 37
   <211> 34
   <212> ADN
   <213> erythrovirus
<400> 37
   TACCACAAAG TGGGCCAATT GGAGGTATTA AATC 34
<210> 38
   <211> 23
   <212> ADN
   <213> erythrovirus
<400> 38
   ATGGGAATTA CTACTTTAGT TCA 23
<210> 39
   <211> 62
   <212> ADN
   <213> erythrovirus
<400> 39
   GGTCATTTAC CATATGTACT GTATGACCCC ACAGCTACAG ATGCAAAGCA ACACCACAGA 60
   CA 62
<210> 40
   <211> 29
   <212> ADN
   <213> erythrovirus
<400> 40
   GGATATGAAA AGCCTGAAGA ATTGTGGAC 29
<210> 41
   <211> 30
   <212> ADN
   <213> erythrovirus
<400> 41
   GCCAAAAGCC GTGTGCACCC ATTGTAAACA 30
<210> 42
   <211> 21
   <212> ADN
   <213> erythrovirus
<400> 42
   TCCCCACCGT GTCCTCAGCC A 21
<210> 43
   <211> 109
   <212> ADN
   <213> erythrovirus
<400> 43
   CTTTAAAAAT TTCAAAAAGA AGACACCAAA TCAGATGCCG CCGGTCGCCG CCGGTAGGCG 60
   GGACTTCCGG TACAAGATGG CGGACAGTTA CGTCATTTCC TGTGACGTC 109
<210> 44
   <211> 103
   <212> ADN
   <213> erythrovirus
<400> 44
   GACGTCACAG GAAATGACGT AACTGTCCGC CATCTTGTAC CGGAAGTCCC GCCTACCGGC 60
   GGCGACCGGC GGCATCTGAT TTGGTGTCTT CTTTTTGAAA TTT 103
<210> 45
   <211> 210
   <212> ADN
   <213> erythrovirus
<400> 45
   CTTTTTGAAA TTTTGGCGGG CTTTTTCCCG CCTTATGCAA ATAAGCGGCC ATGTTTAATG 60
   TTATATTTTA ATTTAATTGG ACAAACGCCT AACGGTTACT AGGGGCGGAG TTACGGGCGG 120
   TATATAAGCA GCTGCGTTCC CTGACACTTT CTTTTCTGGT TGCTTTTGAC TGGAACTCAC 180
   TTGCTGTTCT TTGCCTGCTA AGTAACAGGT 210
<210> 46
   <211> 30
   <212> ADN
   <213> erythrovirus
<400> 46
   ATTTATACTA ACTTTTAATT TACTAACATG 30
<210> 47
   <211> 100
   <212> ADN
   <213> erythrovirus
<400> 47
   GAGCTATTTC GGGGTGTCTT GCACATTTCC TCTAACATTC TGGACTGTGC TAATGATAAC 60
   TGGTGGTGCT CTATGCTAGA CTTAGATACT TCTGACTGGG 100
<210> 48
   <211> 117
   <212> ADN
   <213> erythrovirus
<400> 48
   AACCACTAAC CCATTCTAAC AGATTAATGG CAATATATTT AAGCAGTGTT GCTTCTAAAC 60
   TTGATTTTAC TGGGGGGCCG CTAGCAGGTT GCTTATACTT TTTTCAGGTG GAATGTA 117
<210> 49
   <211> 183
   <212> ADN
   <213> erythrovirus
<400> 49
   ACAAATTTGA GGAAGGCTAT CATATCCATG TAGTTATTGG TGGTCCAGGA CTAAATGCTA 60
   GAAACTTAAC TGTGTGCGTA GAAGGTTTAT TTAATAATGT TCTTTACCAT CTTGTAACTG 120
   AAAGTGTTAA ACTTAAATTT TTGCCAGGGA TGACTACCAA AGGAAAATAT TTTAGAGATG 180
   GAG 183
<210> 50
   <211> 670
   <212> ADN
   <213> erythrovirus
<400> 50
   AGCAGTTTAT AGAAAATTAC TTAATGAAAA AAATTCCTTT AAATGTTGTG TGGTGTGTAA 60
   CAAATATTGA CGGGTATATA GACACCTGTA TTTCCGCCTC TTTTCGGCGA GGAGCTTGTC 120
   ATGCTAAAAG ACCCCGCATT ACTGCAAATA CAGACAGTGC TACTAATGAA ACTGGGGAGT 180
   CTAGCTGTGG AGGGGGAGAT GTTGTGCCAT TCGCTGGAAA GGGAACAAAA GCGGGGTTAA 240
   AGTTTCAAAC CATGGTAAAT TGGCTATGTG AAAACAGAGT ATTTACTGAA GATAAATGGA 300
   AATTAGTGGA TTTTAACCAA TATACTTTAT TAAGTAGCAG TCACAGTGGC AGCTTTCAAA 360
   TTCAAAGTGC CTTAAAGTTA GCTATTTATA AAGCTACTAA CTTAGTACCC ACTAGTACAT 420
   TCTTGTTACA TTCAGACTTT GAGCAGGTTA CTTGCATTAA AGAAAATAAA ATAGTAAAAT 480
   TATTATTGTG TCAAAACTAT GATCCTCTTT TAGTGGGTCA ACATGTGTTA AGGTGGATTG 540
   ACAAAAAATG TGGTAAAAAA AACACCCTGT GGTTTTACGG GCCACCAAGT ACTGGAAAAA 600
   CAAATTTGGC AATGGCTATT GCTAAAACTG TACCAGTGTA TGGAATGGTG AATTGGAATA 660
   ATGAAAACTT 670
<210> 51
   <211> 36
   <212> ADN
   <213> erythrovirus
<400> 51
   TCCATTTAAT GATGTAGCGG GGAAAAGTTT GGTGGT 36
<210> 52
   <211> 46
   <212> ADN
   <213> erythrovirus
<400> 52
   CTGGGATGAA GGCATTATTA AGTCCACTAT TGTGGAAGCT GCAAAA 46
<210> 53
   <211> 84
   <212> ADN
   <213> erythrovirus
<400> 53
   GCCATTTTAG GTGGTCAGCC AACCAGGGTA GATCAGAAAA TGCGTGGCAG TGTGGCAGTG 60
   CCCGGTGTGC CTGTGGTTAT AACC 84
<210> 54
   <211> 60
   <212> ADN
   <213> erythrovirus
<400> 54
   AGCAATGGTG ACATTACATT TGTTGTGAGT GGTAATACCA CTACAACTGT GCATGCTAAA 60
<210> 55
   <211> 76
   <212> ADN
   <213> erythrovirus
<400> 55
   GCCTTAAAGG AACGGATGGT AAAGCTAAAC TTTACCATAA GATGTAGCCC TGACATGGGT 60
   TTACTTACAG AGGCTG 76
<210> 56
   <211> 30
   <212> ADN
   <213> erythrovirus
<400> 56
   ATGTACAACA ATGGCTAACT TGGTGTAATG 30
<210> 57
   <211> 28
   <212> ADN
   <213> erythrovirus
<400> 57
   CACAAAGCTG GAGCCACTAT GAAAACTG 28
<210> 58
   <211> 98
   <212> ADN
   <213> erythrovirus
<400> 58
   TTCAGGAGAA TCATTTGTCG GAAGCCCAGT TTCCTCCGAA GTGGTAGCCG CGTCGTGGGA 60
   GGAAGCTTTT TACACGCCGC TTGCCGATCA GTTTCGTG 98
<210> 59
   <211> 134
   <212> ADN
   <213> erythrovirus
<400> 59
   AACTGTTAGT AGGGGTTGAC TTTGTATGGG ATGGTGTGAG GGGATTGCCT GTTTGCTGTG 60
   TGGAACATAT AAACAACAGT GGGGGAGGGT TGGGGCTTTG CCCTCATTGT ATTAATGTGG 120
   GAGCTTGGTA TAAT 134
<210> 60
   <211> 100
   <212> ADN
   <213> erythrovirus
<400> 60
   GGATGGAAAT TTAGAGAGTT TACTCCAGAC TTAGTGCGCT GCAGTTGTCA TGTAGGAGCC 60
   TCTAACCCAT TTTCTGTGTT AACTTGTAAA AAATGTGCTT 100
<210> 61
   <211> 30
   <212> ADN
   <213> erythrovirus
<400> 61
   ACCTGTCTGG ATTACAAAGT TTTGTAGATT 30
<210> 62
   <211> 102
   <212> ADN
   <213> erythrovirus
<400> 62
   ATGAGTAAAA CCACTAACAA ATGGTGGGAA AGCAGTGACA AATTTGCCCA GGACGTGTAT 60
   AAGCAGTTTG TGCAATTTTA TGAAAAAGCT ACTGGAACAG AC 102
<210> 63
   <211> 114
   <212> ADN
   <213> erythrovirus
<400> 63
   TTAGAGCTTA TTCAAATTTT AAAAGACCAT TACAACATTT CTTTAGATAA TCCTTTAGAA 60
   AACCCCTCTT CTTTATTTGA CTTAGTTGCT CGCATTAAAA GTAATCTTAA AAAC 114
<210> 64
   <211> 22
   <212> ADN
   <213> erythrovirus
<400> 64
   TCTCCAGACC TATATAGTCA TC 22
<210> 65
   <211> 23
   <212> ADN
   <213> erythrovirus
<400> 65
   ATTTTCAGAG CCATGGACAG TTA 23
<210> 66
   <211> 30
   <212> ADN
   <213> erythrovirus
<400> 66
   ATCATCCAGT AACAGTAGTG CAGAACCTAG 30
<210> 67
   <211> 39
   <212> ADN
   <213> erythrovirus .
<400> 67
   CAAGCTGGGC CTCCGCAGAA TGCTGTGGAC AGTGCTGCA 39
<210> 68
   <211> 56
   <212> ADN
   <213> erythrovirus
<400> 68
   GGAATAAATC CTTATACACA TTGGACGGTA GCAGATGAAG AATTGTTAAA AAATAT 56
<210> 69
   <211> 51
   <212> ADN
   <213> erythrovirus
<400> 69
   AAAAAATGAA ACAGGGTTTC AAGCACAAGC AGTAAAAGAT TACTTTACTT T 51
<210> 70
   <211> 37
   <212> ADN
   <213> erythrovirus
<400> 70
   AAGGAAGTTT ACCGGAAGTG CCCGCGTACA ACGCCTC 37
<210> 71
   <211> 42
   <212> ADN
   <213> erythrovirus
<400> 71
   AGAAAAATAC CCCAGCATGA CTTCAGTTAA CTCTGCAGAA GC 42
<210> 72
   <211> 255
   <212> ADN
   <213> erythrovirus
<400> 72
   CAGCACTGGT GCAGGCGGGG GAGGTAGCAA CCCTACAAAA AGCATGTGGA GTGAAGGGGC 60
   TACATTTACT GCTAATTCTG TAACGTGTAC ATTCTCTAGG CAATTTTTAA TTCCATATGA 120
   TCCAGAGCAT CATTATAAAG TGTTCTCTCC AGCAGCTAGT AGCTGCCACA ATGCTAGTGG 180
   GAAAGAGGCA AAAGTGTGCA CTATTAGTCC CATTATGGGG TACTCTACTC CGTGGAGATA 240
   CTTAGATTTT AATGC 255
<210> 73
   <211> 33
   <212> ADN
   <213> erythrovirus
<400> 73
   TTTAAATTTG TTTTTCTCAC CATTAGAGTT TCA 33
<210> 74
   <211> 725
   <212> ADN
   <213> erythrovirus
<400> 74
   GAAAATTATG GTAGTATAGC TCCAGATGCT TTAACTGTAA CTATTTCAGA AATTGCTGTA 60
   AAAGATGTCA CAGACAAAAC AGGAGGAGGT GTGCAAGTTA CTGACAGCAC CACAGGACGT 120
   TTGTGTATGT TAGTGGATCA TGAGTATAAA TACCCATATG TGCTAGGTCA GGGACAAGAC 180
   ACACTAGCTC CAGAACTGCC CATTTGGGTT TACTTTCCCC CCCAGTATGC TTACTTAACA 240
   GTAGGTGAAG TAAACACACA AGGAATTTCA GGAGACAGCA AAAAATTGGC TAGTGAAGAA 300
   TCAGCTTTTT ATGTGTTAGA GCACAGTTCA TTTGAACTTT TGGGTACAGG GGGATCTGCC 360
   ACTATGTCCT ACAAATTTCC AGCTGTGCCC CCAGAAAACC TAGAAGGCTG CAGCCAACAT 420
   TTTTATGAAA TGTACAACCC TTTGTACGGT TCTCGTTTAG GGGTACCTGA CACATTAGGA 480
   GGGGACCCTA AATTTAGATC ATTGACACAC GAAGACCACG CAATTCAGCC ACAAAACTTT 540
   ATGCCTGGGC CACTAATAAA TTCAGTGTCT ACCAAAGAAG GAGACAATTC TAATACAGGT 600
   GCTGGAAAAG CCCTTACGGG GCTTAGTACT GGCACTAGCC AAAACACCAG AATTTCCCTA 660
   CGCCCCGGGC CAGTATCTCA GCCATACCAT CACTGGGACA CTGATAAATA TGTTACAGGA 720
   ATAAA 725
<210> 75
   <211> 49
   <212> ADN
   <213> erythrovirus
<400> 75
   TGCCATTTCA CATGGACAAA CCACTTATGG AAATGCTGAG GACAAAGAG 49
<210> 76
   <211> 30
   <212> ADN
   <213> erythrovirus
<400> 76
   TATCAGCAAG GGGTAGGAAG ATTTCCAAAT 30
<210> 77
   <211> 180
   <212> ADN
   <213> erythrovirus
<400> 77
   GAAAAAGAAC AGCTTAAGCA GTTACAAGGT CTTAACATGC ACACATACTT CCCTAATAAA 60
   GGAACCCAAC AATACACAGA CCAAATTGAA CGCCCTCTTA TGGTGGGCTC TGTTTGGAAC 120
   AGAAGAGCTC TTCACTATGA AAGTCAGCTG TGGAGTAAAA TCCCTAACTT AGATGACAGT 180
<210> 78
   <211> 64
   <212> ADN
   <213> erythrovirus
<400> 78
   TTTAAAACTC AATTTGCAGC CCTAGGCGGG TGGGGTTTGC ATCAACCACC CCCTCAAATA 60
   TTTT 64
<210> 79
   <211> 152
   <212> ADN
   <213> erythrovirus
<400> 79
   AGGTATTAAA TCCATGGGAA TTACTACTTT AGTTCAATAT GCTGTGGGAA TAATGACAGT      60
   TACCATGACC TTTAAATTGG GACCTCGAAA GGCTACTGGA AGGTGGAATC CCCAGCCTGG     120
   CGTTTATCCT CCTCATGCAG CTGGTCATTT AC                                   152
<210> 80
   <211> 260
   <212> ADN
   <213> erythrovirus
<400> 80
   CCCATTGTAA ACATTCCCCA CCGTGTCCTC AGCCAGGAAC CGTCACCCAC CGCCCACCTG      60
   TGCCGCCCAG ATTATATGTG CCCCCTCCAA TACCCCGTAG GCAACCATCT ATAAAAGATA     120
   CAGACGCTGT AGAATATAAA TTATTAACTA GATATGAACA ACATGTAATT AGAATGCTAA     180
   GATTATGTAA TATGTACACA AGTTTGGAAA AATAAAAGCC TTAAATAAAT AATTCATAGT     240
   GTATGGTTCT TTAAAAATTT                                                 260
<210> 81
   <211> 2013
   <212> ADN
   <213> erythrovirus
<400> 81
   ATG GAG CTA TTT CGG GGT GTC TTG CAC ATT TCC TCT AAC ATT CTG GAC          48
   Met Glu Leu Phe Arg Gly Val Leu His Ile Ser Ser Asn Ile Leu Asp
    1               5                  10                  15
   TGT GCT AAT GAT AAC TGG TGG TGC TCT ATG CTA GAC TTA GAT ACT TCT          96
   Cys Ala Asn Asp Asn Trp Trp Cys Ser Met Leu Asp Leu Asp Thr Ser
               20                  25                  30
   GAC TGG GAA CCA CTA ACC CAT TCT AAC AGA TTA ATG GCA ATA TAT TTA         144
   Asp Trp Glu Pro Leu Thr His Ser Asn Arg Leu Met Ala Ile Tyr Leu
           35                  40                  45
   AGC AGT GTT GCT TCT AAA CTT GAT TTT ACT GGG GGG CCG CTA GCA GGT         192
   Ser Ser Val Ala Ser Lys Leu Asp Phe Thr Gly Gly Pro Leu Ala Gly
       50                  55                  60
   TGC TTA TAC TTT TTT CAG GTG GAA TGT AAC AAA TTT GAG GAA GGC TAT         240
   Cys Leu Tyr Phe Phe Gln Val Glu Cys Asn Lys Phe Glu Glu Gly Tyr
   65                  70                  75                  80
   CAT ATC CAT GTA GTT ATT GGT GGT CCA GGA CTA AAT GCT AGA AAC TTA         288
   His Ile His Val Val Ile Gly Gly Pro Gly Leu Asn Ala Arg Asn Leu
                   85                  90                  95
   ACT GTG TGC GTA GAA GGT TTA TTT AAT AAT GTT CTT TAC CAT CTT GTA         336
   Thr Val Cys Val Glu Gly Leu Phe Asn Asn Val Leu Tyr His Leu Val
               100                 105                 110
   ACT GAA AGT GTT AAA CTT AAA TTT TTG CCA GGG ATG ACT ACC AAA GGA         384
   Thr Glu Ser Val Lys Leu Lys Phe Leu Pro Gly Met Thr Thr Lys Gly
           115                 120                 125
   AAA TAT TTT AGA GAT GGA GAG CAG TTT ATA GAA AAT TAC TTA ATG AAA         432
   Lys Tyr Phe Arg Asp Gly Glu Gln Phe Ile Glu Asn Tyr Leu Met Lys
       130                 135                 140
   AAA ATT CCT TTA AAT GTT GTG TGG TGT GTA ACA AAT ATT GAC GGG TAT         480
   Lys Ile Pro Leu Asn Val Val Trp Cys Val Thr Asn Ile Asp Gly Tyr
   145                 150                 155                 160
   ATA GAC ACC TGT ATT TCC GCC TCT TTT CGG CGA GGA GCT TGT CAT GCT      528
   Ile Asp Thr Cys Ile Ser Ala Ser Phe Arg Arg Gly Ala Cys His Ala
                   165                 170                 175
   AAA AGA CCC CGC ATT ACT GCA AAT ACA GAC AGT GCT ACT AAT GAA ACT      576
   Lys Arg Pro Arg Ile Thr Ala Asn Thr Asp Ser Ala Thr Asn Glu Thr
               180                 185                 190
   GGG GAG TCT AGC TGT GGA GGG GGA GAT GTT GTG CCA TTC GCT GGA AAG      624
   Gly Glu Ser Ser Cys Gly Gly Gly Asp Val Val Pro Phe Ala Gly Lys
           195                 200                 205
   GGA ACA AAA GCG GGG TTA AAG TTT CAA ACC ATG GTA AAT TGG CTA TGT      672
   Gly Thr Lys Ala Gly Leu Lys Phe Gln Thr Met Val Asn Trp Leu Cys
       210                 215                 220
   GAA AAC AGA GTA TTT ACT GAA GAT AAA TGG AAA TTA GTG GAT TTT AAC      720
   Glu Asn Arg Val Phe Thr Glu Asp Lys Trp Lys Leu Val Asp Phe Asn
   225                 230                 235                 240
   CAA TAT ACT TTA TTA AGT AGC AGT CAC AGT GGC AGC TTT CAA ATT CAA      768
   Gln Tyr Thr Leu Leu Ser Ser Ser His Ser Gly Ser Phe Gln Ile Gln
                   245                 250                 255
   AGT GCC TTA AAG TTA GCT ATT TAT AAA GCT ACT AAC TTA GTA CCC ACT      816
   Ser Ala Leu Lys Leu Ala Ile Tyr Lys Ala Thr Asn Leu Val Pro Thr
               260                 265                 270
   AGT ACA TTC TTG TTA CAT TCA GAC TTT GAG CAG GTT ACT TGC ATT AAA      864
   Ser Thr Phe Leu Leu His Ser Asp Phe Glu Gln Val Thr Cys Ile Lys
           275                 280                 285
   GAA AAT AAA ATA GTA AAA TTA TTA TTG TGT CAA AAC TAT GAT CCT CTT      912
   Glu Asn Lys Ile Val Lys Leu Leu Leu Cys Gln Asn Tyr Asp Pro Leu
       290                 295                 300
   TTA GTG GGT CAA CAT GTG TTA AGG TGG ATT GAC AAA AAA TGT GGT AAA      960
   Leu Val Gly Gln His Val Leu Arg Trp Ile Asp Lys Lys Cys Gly Lys
   305                 310                 315                 320
   AAA AAC ACC CTG TGG TTT TAC GGG CCA CCA AGT ACT GGA AAA ACA AAT     1008
   Lys Asn Thr Leu Trp Phe Tyr Gly Pro Pro Ser Thr Gly Lys Thr Asn
                   325                 330                 335
   TTG GCA ATG GCT ATT GCT AAA ACT GTA CCA GTG TAT GGA ATG GTG AAT     1056
   Leu Ala Met Ala Ile Ala Lys Thr Val Pro Val Tyr Gly Met Val Asn
               340                 345                 350
   TGG AAT AAT GAA AAC TTT CCA TTT AAT GAT GTA GCG GGG AAA AGT TTG     1104
   Trp Asn Asn Glu Asn Phe Pro Phe Asn Asp Val Ala Gly Lys Ser Leu
           355                 360                 365
   GTG GTC TGG GAT GAA GGC ATT ATT AAG TCC ACT ATT GTG GAA GCT GCA     1152
   Val Val Trp Asp Glu Gly Ile Ile Lys Ser Thr Ile Val Glu Ala Ala
       370                 375                 380
   AAA GCC ATT TTA GGT GGT CAG CCA ACC AGG GTA GAT CAG AAA ATG CGT     1200
   Lys Ala Ile Leu Gly Gly Gln Pro Thr Arg Val Asp Gln Lys Met Arg
   385                 390                 395                 400
   GGC AGT GTG GCA GTG CCC GGT GTG CCT GTG GTT ATA ACC AGC AAT GGT     1248
   Gly Ser Val Ala Val Pro Gly Val Pro Val Val Ile Thr Ser Asn Gly
                   405                 410                 415
   GAC ATT ACA TTT GTT GTG AGT GGT AAT ACC ACT ACA ACT GTG CAT GCT     1296
   Asp Ile Thr Phe Val Val Ser Gly Asn Thr Thr Thr Thr Val His Ala
               420                 425                 430
   AAA GCC TTA AAG GAA CGG ATG GTA AAG CTA AAC TTT ACC ATA AGA TGT     1344
   Lys Ala Leu Lys Glu Arg Met Val Lys Leu Asn Phe Thr Ile Arg Cys
           435                 440                 445
   AGC CCT GAC ATG GGT TTA CTT ACA GAG GCT GAT GTA CAA CAA TGG CTA     1392
   Ser Pro Asp Met Gly Leu Leu Thr Glu Ala Asp Val Gln Gln Trp Leu
       450                 455                 460
   ACT TGG TGT AAT GCA CAA AGC TGG AGC CAC TAT GAA AAC TGG GCA ATA     1440
   Thr Trp Cys Asn Ala Gln Ser Trp Ser His Tyr Glu Asn Trp Ala Ile
   465                 470                 475                 480
   AAC TAC ACA TTT GAT TTC CCT GGA ATA AAT GCA GAT GCC CTC CAC CCA     1488
   Asn Tyr Thr Phe Asp Phe Pro Gly Ile Asn Ala Asp Ala Leu His Pro
                   485                 490                 495
   GAT CTC CAA ACC ACC CCC ATT GTC CCA GAC ACC AGT ATC AGC AGC AGT     1536
   Asp Leu Gln Thr Thr Pro Ile Val Pro Asp Thr Ser Ile Ser Ser Ser
               500                 505                 510
   GGT GGT GAA AGC TCT GAA GAA CTC AGT GAA AGC AGC TTT TTC AAC CTC     1584
   Gly Gly Glu Ser Ser Glu Glu Leu Ser Glu Ser Ser Phe Phe Asn Leu
           515                 520                 525
   ATC ACT CCA GGC GCC TGG AAC AGT GAA ACC CCG CGC TCT AGT ACG CCC     1632
   Ile Thr Pro Gly Ala Trp Asn Ser Glu Thr Pro Arg Ser Ser Thr Pro
       530                 535                 540
   GTC CCC GGG ACC AGT TCA GGA GAA TCA TTT GTC GGA AGC CCA GTT TCC     1680
   Val Pro Gly Thr Ser Ser Gly Glu Ser Phe Val Gly Ser Pro Val Ser
   545                 550                 555                 560
   TCC GAA GTG GTA GCC GCG TCG TGG GAG GAA GCT TTT TAC ACG CCG CTT     1728
   Ser Glu Val Val Ala Ala Ser Trp Glu Glu Ala Phe Tyr Thr Pro Leu
                   565                 570                 575
   GCC GAT CAG TTT CGT GAA CTG TTA GTA GGG GTT GAC TTT GTA TGG GAT     1776
   Ala Asp Gln Phe Arg Glu Leu Leu Val Gly Val Asp Phe Val Trp Asp
               580                 585                 590
   GGT GTG AGG GGA TTG CCT GTT TGC TGT GTG GAA CAT ATA AAC AAC AGT     1824
   Gly Val Arg Gly Leu Pro Val Cys Cys Val Glu His Ile Asn Asn Ser
           595                 600                 605
   GGG GGA GGG TTG GGG CTT TGC CCT CAT TGT ATT AAT GTG GGA GCT TGG     1872
   Gly Gly Gly Leu Gly Leu Cys Pro His Cys Ile Asn Val Gly Ala Trp
       610                 615                 620
   TAT AAT GGA TGG AAA TTT AGA GAG TTT ACT CCA GAC TTA GTG CGC TGC     1920
   Tyr Asn Gly Trp Lys Phe Arg Glu Phe Thr Pro Asp Leu Val Arg Cys
   625                 630                 635                 640
   AGT TGT CAT GTA GGA GCC TCT AAC CCA TTT TCT GTG TTA ACT TGT AAA     1968
   Ser Cys His Val Gly Ala Ser Asn Pro Phe Ser Val Leu Thr Cys Lys
                   645                 650                 655
   AAA TGT GCT TAC CTG TCT GGA TTA CAA AGT TTT GTA GAT TAT GAG         2013
   Lys Cys Ala Tyr Leu Ser Gly Leu Gln Ser Phe Val Asp Tyr Glu
               660                 665                 670
<210> 82
   <211> 671
   <212> PRT
   <213> erythrovirus
<400> 82
   Met Glu Leu Phe Arg Gly Val Leu His Ile Ser Ser Asn Ile Leu Asp
    1               5                  10                  15
   Cys Ala Asn Asp Asn Trp Trp Cys Ser Met Leu Asp Leu Asp Thr Ser
               20                  25                  30
   Asp Trp Glu Pro Leu Thr His Ser Asn Arg Leu Met Ala Ile Tyr Leu
           35                  40                  45
   Ser Ser Val Ala Ser Lys Leu Asp Phe Thr Gly Gly Pro Leu Ala Gly
       50                  55                  60
   Cys Leu Tyr Phe Phe Gln Val Glu Cys Asn Lys Phe Glu Glu Gly Tyr
   65                  70                  75                  80
   His Ile His Val Val Ile Gly Gly Pro Gly Leu Asn Ala Arg Asn Leu
                   85                  90                  95
   Thr Val Cys Val Glu Gly Leu Phe Asn Asn Val Leu Tyr His Leu Val
               100                 105                 110
   Thr Glu Ser Val Lys Leu Lys Phe Leu Pro Gly Met Thr Thr Lys Gly
           115                 120                 125
   Lys Tyr Phe Arg Asp Gly Glu Gln Phe Ile Glu Asn Tyr Leu Met Lys
       130                 135                 140
   Lys Ile Pro Leu Asn Val Val Trp Cys Val Thr Asn Ile Asp Gly Tyr
   145                 150                 155                 160
   Ile Asp Thr Cys Ile Ser Ala Ser Phe Arg Arg Gly Ala Cys His Ala
                   165                 170                 175
   Lys Arg Pro Arg Ile Thr Ala Asn Thr Asp Ser Ala Thr Asn Glu Thr
               180                 185                 190
   Gly Glu Ser Ser Cys Gly Gly Gly Asp Val Val Pro Phe Ala Gly Lys
           195                 200                 205
   Gly Thr Lys Ala Gly Leu Lys Phe Gln Thr Met Val Asn Trp Leu Cys
       210                 215                 220
   Glu Asn Arg Val Phe Thr Glu Asp Lys Trp Lys Leu Val Asp Phe Asn
   225                 230                 235                 240
   Gln Tyr Thr Leu Leu Ser Ser Ser His Ser Gly Ser Phe Gln Ile Gln
                   245                 250                 255
   Ser Ala Leu Lys Leu Ala Ile Tyr Lys Ala Thr Asn Leu Val Pro Thr
               260                 265                 270
   Ser Thr Phe Leu Leu His Ser Asp Phe Glu Gln Val Thr Cys Ile Lys
           275                 280                 285
   Glu Asn Lys Ile Val Lys Leu Leu Leu Cys Gln Asn Tyr Asp Pro Leu
       290                 295                 300
   Leu Val Gly Gln His Val Leu Arg Trp Ile Asp Lys Lys Cys Gly Lys
   305                 310                 315                 320
   Lys Asn Thr Leu Trp Phe Tyr Gly Pro Pro Ser Thr Gly Lys Thr Asn
   325 330 335
   Leu Ala Met Ala Ile Ala Lys Thr Val Pro Val Tyr Gly Met Val Asn
   340 345 350
   Trp Asn Asn Glu Asn Phe Pro Phe Asn Asp Val Ala Gly Lys Ser Leu
   355 360 365
   Val Val Trp Asp Glu Gly Ile Ile Lys Ser Thr Ile Val Glu Ala Ala
   370 375 380
   Lys Ala Ile Leu Gly Gly Gln Pro Thr Arg Val Asp Gln Lys Met Arg
   385 390 395 400
   Gly Ser Val Ala Val Pro Gly Val Pro Val Val Ile Thr Ser Asn Gly
   405 410 415
   Asp Ile Thr Phe Val Val Ser Gly Asn Thr Thr Thr Thr Val His Ala
   420 425 430
   Lys Ala Leu Lys Glu Arg Met Val Lys Leu Asn Phe Thr Ile Arg Cys
   435 440 445
   Ser Pro Asp Met Gly Leu Leu Thr Glu Ala Asp Val Gln Gln Trp Leu
   450 455 460
   Thr Trp Cys Asn Ala Gln Ser Trp Ser His Tyr Glu Asn Trp Ala Ile
   465 470 475 480
   Asn Tyr Thr Phe Asp Phe Pro Gly Ile Asn Ala Asp Ala Leu His Pro
   485 490 495
   Asp Leu Gln Thr Thr Pro Ile Val Pro Asp Thr Ser Ile Ser Ser Ser
   500 505 510
   Gly Gly Glu Ser Ser Glu Glu Leu Ser Glu Ser Ser Phe Phe Asn Leu
   515 520 525
   Ile Thr Pro Gly Ala Trp Asn Ser Glu Thr Pro Arg Ser Ser Thr Pro
   530 535 540
   Val Pro Gly Thr Ser Ser Gly Glu Ser Phe Val Gly Ser Pro Val Ser
   545 550 555 560
   Ser Glu Val Val Ala Ala Ser Trp Glu Glu Ala Phe Tyr Thr Pro Leu
   565 570 575
   Ala Asp Gln Phe Arg Glu Leu Leu Val Gly Val Asp Phe Val Trp Asp
   580 585 590
   Gly Val Arg Gly Leu Pro Val Cys Cys Val Glu His Ile Asn Asn Ser
   595 600 605
   Gly Gly Gly Leu Gly Leu Cys Pro His Cys Ile Asn Val Gly Ala Trp
   610 615 620
   Tyr Asn Gly Trp Lys Phe Arg Glu Phe Thr Pro Asp Leu Val Arg Cys
   625 630 635 640
   Ser Cys His Val Gly Ala Ser Asn Pro Phe Ser Val Leu Thr Cys Lys
   645 650 655
   Lys Cys Ala Tyr Leu Ser Gly Leu Gln Ser Phe Val Asp Tyr Glu
   660 665 670
<210> 83
   <211> 222
   <212> ADN
   <213> erythrovirus
<400> 83
   ATG CAG ATG CCC TCC ACC CAG ATC TCC AAA CCA CCC CCA TTG TCC CAG       48
   Met Gln Met Pro Ser Thr Gln Ile Ser Lys Pro Pro Pro Leu Ser Gln
               675                 680                 685
   ACA CCA GTA TCA GCA GCA GTG GTG GTG AAA GCT CTG AAG AAC TCA GTG       96
   Thr Pro Val Ser Ala Ala Val Val Val Lys Ala Leu Lys Asn Ser Val
           690                 695                 700
   AAA GCA GCT TTT TCA ACC TCA TCA CTC CAG GCG CCT GGA ACA GTG AAA      144
   Lys Ala Ala Phe Ser Thr Ser Ser Leu Gln Ala Pro Gly Thr Val Lys
       705                 710                 715
   CCC CGC GCT CTA GTA CGC CCG TCC CCG GGA CCA GTT CAG GAG AAT CAT      192
   Pro Arg Ala Leu Val Arg Pro Ser Pro Gly Pro Val Gln Glu Asn His
   720                 725                 730                 735
   TTG TCG GAA GCC CAG TTT CCT CCG AAG TGG                              222
   Leu Ser Glu Ala Gln Phe Pro Pro Lys Trp
                   740                 745
<210> 84
   <211> 74
   <212> PRT
   <213> erythrovirus
<400> 84
   Met Gln Met Pro Ser Thr Gln Ile Ser Lys Pro Pro Pro Leu Ser Gln
   1               5                   10                  15
   Thr Pro Val Ser Ala Ala Val Val Val Lys Ala Leu Lys Asn Ser Val
               20                  25                  30
   Lys Ala Ala Phe Ser Thr Ser Ser Leu Gln Ala Pro Gly Thr Val Lys
           35                  40                  45
   Pro Arg Ala Leu Val Arg Pro Ser Pro Gly Pro Val Gln Glu Asn His
       50                  55                  60
   Leu Ser Glu Ala Gln Phe Pro Pro Lys Trp
   65                  70
<210> 85
   <211> 2343
   <212> ADN
   <213> erythrovirus
<400> 85
   ATG AGT AAA ACC ACT AAC AAA TGG TGG GAA AGC AGT GAC AAA TTT GCC      48
   Met Ser Lys Thr Thr Asn Lys Trp Trp Glu Ser Ser Asp Lys Phe Ala
    75                 80                  85                  90
   CAG GAC GTG TAT AAG CAG TTT GTG CAA TTT TAT GAA AAA GCT ACT GGA      96
   Gln Asp Val Tyr Lys Gln Phe Val Gln Phe Tyr Glu Lys Ala Thr Gly
                    95                 100                 105
   ACA GAC TTA GAG CTT ATT CAA ATT TTA AAA GAC CAT TAC AAC ATT TCT     144
   Thr Asp Leu Glu Leu Ile Gln Ile Leu Lys Asp His Tyr Asn Ile Ser
               110                 115                 120
   TTA GAT AAT CCT TTA GAA AAC CCC TCT TCT TTA TTT GAC TTA GTT GCT      192
   Leu Asp Asn Pro Leu Glu Asn Pro Ser Ser Leu Phe Asp Leu Val Ala
           125                 130                 135
   CGC ATT AAA AGT AAT CTT AAA AAC TCT CCA GAC CTA TAT AGT CAT CAT      240
   Arg Ile Lys Ser Asn Leu Lys Asn Ser Pro Asp Leu Tyr Ser His His
       140                 145                 150
   TTT CAG AGC CAT GGA CAG TTA TCT GAC CAC CCC CAT GCC TTA TCA TCC      288
   Phe Gln Ser His Gly Gln Leu Ser Asp His Pro His Ala Leu Ser Ser
   155                 160                 165                 170
   AGT AAC AGT AGT GCA GAA CCT AGA GGA GAA AAT GCA GTA TTA TCT AGT      336
   Ser Asn Ser Ser Ala Glu Pro Arg Gly Glu Asn Ala Val Leu Ser Ser
                   175                 180                 185
   GAA GAC TTA CAC AAG CCT GGG CAA GTT AGC ATA CAA TTA CCC GGT ACT      384
   Glu Asp Leu His Lys Pro Gly Gln Val Ser Ile Gln Leu Pro Gly Thr
               190                 195                 200
   AAC TAT GTT GGG CCT GGC AAT GAG CTA CAA GCT GGG CCT CCG CAG AAT      432
   Asn Tyr Val Gly Pro Gly Asn Glu Leu Gln Ala Gly Pro Pro Gln Asn
           205                 210                 215
   GCT GTG GAC AGT GCT GCA AGG ATT CAT GAC TTT AGG TAT AGC CAA TTG      480
   Ala Val Asp Ser Ala Ala Arg Ile His Asp Phe Arg Tyr Ser Gln Leu
       220                 225                 230
   GCT AAG TTG GGA ATA AAT CCT TAT ACA CAT TGG ACG GTA GCA GAT GAA      528
   Ala Lys Leu Gly Ile Asn Pro Tyr Thr His Trp Thr Val Ala Asp Glu
   235                 240                 245                 250
   GAA TTG TTA AAA AAT ATA AAA AAT GAA ACA GGG TTT CAA GCA CAA GCA      576
   Glu Leu Leu Lys Asn Ile Lys Asn Glu Thr Gly Phe Gln Ala Gln Ala
                   255                 260                 265
   GTA AAA GAT TAC TTT ACT TTA AAA GGT GCA GCT GCC CCT GTG GCC CAT      624
   Val Lys Asp Tyr Phe Thr Leu Lys Gly Ala Ala Ala Pro Val Ala His
               270                 275                 280
   TTT CAA GGA AGT TTA CCG GAA GTG CCC GCG TAC AAC GCC TCA GAA AAA      672
   Phe Gln Gly Ser Leu Pro Glu Val Pro Ala Tyr Asn Ala Ser Glu Lys
           285                 290                 295
   TAC CCC AGC ATG ACT TCA GTT AAC TCT GCA GAA GCC AGC ACT GGT GCA      720
   Tyr Pro Ser Met Thr Ser Val Asn Ser Ala Glu Ala Ser Thr Gly Ala
       300                 305                 310
   GGC GGG GGA GGT AGC AAC CCT ACA AAA AGC ATG TGG AGT GAA GGG GCT      768
   Gly Gly Gly Gly Ser Asn Pro Thr Lys Ser Met Trp Ser Glu Gly Ala
   315                 320                 325                 330
   ACA TTT ACT GCT AAT TCT GTA ACG TGT ACA TTC TCT AGG CAA TTT TTA      816
   Thr Phe Thr Ala Asn Ser Val Thr Cys Thr Phe Ser Arg Gln Phe Leu
                   335                 340                 345
   ATT CCA TAT GAT CCA GAG CAT CAT TAT AAA GTG TTC TCT CCA GCA GCT      864
   Ile Pro Tyr Asp Pro Glu His His Tyr Lys Val Phe Ser Pro Ala Ala
               350                 355                 360
   AGT AGC TGC CAC AAT GCT AGT GGG AAA GAG GCA AAA GTG TGC ACT ATT      912
   Ser Ser Cys His Asn Ala Ser Gly Lys Glu Ala Lys Val Cys Thr Ile
           365                 370                 375
   AGT CCC ATT ATG GGG TAC TCT ACT CCG TGG AGA TAC TTA GAT TTT AAT      960
   Ser Pro Ile Met Gly Tyr Ser Thr Pro Trp Arg Tyr Leu Asp Phe Asn
        380                 385                 390
   GCT TTA AAT TTG TTT TTC TCA CCA TTA GAG TTT CAG CAC TTA ATT GAA      1008
   Ala Leu Asn Leu Phe Phe Ser Pro Leu Glu Phe Gln His Leu Ile Glu
   395                 400                 405                 410
   AAT TAT GGT AGT ATA GCT CCA GAT GCT TTA ACT GTA ACT ATT TCA GAA      1056
   Asn Tyr Gly Ser Ile Ala Pro Asp Ala Leu Thr Val Thr Ile Ser Glu
                   415                 420                 425
   ATT GCT GTA AAA GAT GTC ACA GAC AAA ACA GGA GGA GGT GTG CAA GTT      1104
   Ile Ala Val Lys Asp Val Thr Asp Lys Thr Gly Gly Gly Val Gln Val
               430                 435                 440
   ACT GAC AGC ACC ACA GGA CGT TTG TGT ATG TTA GTG GAT CAT GAG TAT      1152
   Thr Asp Ser Thr Thr Gly Arg Leu Cys Met Leu Val Asp His Glu Tyr
           445                 450                 455
   AAA TAC CCA TAT GTG CTA GGT CAG GGA CAA GAC ACA CTA GCT CCA GAA      1200
   Lys Tyr Pro Tyr Val Leu Gly Gln Gly Gln Asp Thr Leu Ala Pro Glu
       460                 465                 470
   CTG CCC ATT TGG GTT TAC TTT CCC CCC CAG TAT GCT TAC TTA ACA GTA      1248
   Leu Pro Ile Trp Val Tyr Phe Pro Pro Gln Tyr Ala Tyr Leu Thr Val
   475                 480                 485                 490
   GGT GAA GTA AAC ACA CAA GGA ATT TCA GGA GAC AGC AAA AAA TTG GCT      1296
   Gly Glu Val Asn Thr Gln Gly Ile Ser Gly Asp Ser Lys Lys Leu Ala
                   495                 500                 505
   AGT GAA GAA TCA GCT TTT TAT GTG TTA GAG CAC AGT TCA TTT GAA CTT      1344
   Ser Glu Glu Ser Ala Phe Tyr Val Leu Glu His Ser Ser Phe Glu Leu
               510                 515                 520
   TTG GGT ACA GGG GGA TCT GCC ACT ATG TCC TAC AAA TTT CCA GCT GTG      1392
   Leu Gly Thr Gly Gly Ser Ala Thr Met Ser Tyr Lys Phe Pro Ala Val
           525                 530                 535
   CCC CCA GAA AAC CTA GAA GGC TGC AGC CAA CAT TTT TAT GAA ATG TAC      1440
   Pro Pro Glu Asn Leu Glu Gly Cys Ser Gln His Phe Tyr Glu Met Tyr
       540                 545                 550
   AAC CCT TTG TAC GGT TCT CGT TTA GGG GTA CCT GAC ACA TTA GGA GGG      1488
   Asn Pro Leu Tyr Gly Ser Arg Leu Gly Val Pro Asp Thr Leu Gly Gly
   555                 560                 565                 570
   GAC CCT AAA TTT AGA TCA TTG ACA CAC GAA GAC CAC GCA ATT CAG CCA      1536
   Asp Pro Lys Phe Arg Ser Leu Thr His Glu Asp His Ala Ile Gln Pro
                   575                 580                 585
   CAA AAC TTT ATG CCT GGG CCA CTA ATA AAT TCA GTG TCT ACC AAA GAA      1584
   Gln Asn Phe Met Pro Gly Pro Leu Ile Asn Ser Val Ser Thr Lys Glu
               590                 595                 600
   GGA GAC AAT TCT AAT ACA GGT GCT GGA AAA GCC CTT ACG GGG CTT AGT      1632
   Gly Asp Asn Ser Asn Thr Gly Ala Gly Lys Ala Leu Thr Gly Leu Ser
           605                 610                 615
   ACT GGC ACT AGC CAA AAC ACC AGA ATT TCC CTA CGC CCC GGG CCA GTA      1680
   Thr Gly Thr Ser Gln Asn Thr Arg Ile Ser Leu Arg Pro Gly Pro Val
       620                 625                 630
   TCT CAG CCA TAC CAT CAC TGG GAC ACT GAT AAA TAT GTT ACA GGA ATA      1728
   Ser Gln Pro Tyr His His Trp Asp Thr Asp Lys Tyr Val Thr Gly Ile
   635                 640                 645                 650
   AAT GCC ATT TCA CAT GGA CAA ACC ACT TAT GGA AAT GCT GAG GAC AAA      1776
   Asn Ala Ile Ser His Gly Gln Thr Thr Tyr Gly Asn Ala Glu Asp Lys
                   655                 660                 665
   GAG TAT CAG CAA GGG GTA GGA AGA TTT CCA AAT GAA AAA GAA CAG CTT      1824
   Glu Tyr Gln Gln Gly Val Gly Arg Phe Pro Asn Glu Lys Glu Gln Leu
               670                 675                 680
   AAG CAG TTA CAA GGT CTT AAC ATG CAC ACA TAC TTC CCT AAT AAA GGA      1872
   Lys Gln Leu Gln Gly Leu Asn Met His Thr Tyr Phe Pro Asn Lys Gly
           685                 690                 695
   ACC CAA CAA TAC ACA GAC CAA ATT GAA CGC CCT CTT ATG GTG GGC TCT      1920
   Thr Gln Gln Tyr Thr Asp Gln Ile Glu Arg Pro Leu Met Val Gly Ser
       700                 705                 710
   GTT TGG AAC AGA AGA GCT CTT CAC TAT GAA AGT CAG CTG TGG AGT AAA      1968
   Val Trp Asn Arg Arg Ala Leu His Tyr Glu Ser Gln Leu Trp Ser Lys
   715                 720                 725                 730
   ATC CCT AAC TTA GAT GAC AGT TTT AAA ACT CAA TTT GCA GCC CTA GGC      2016
   Ile Pro Asn Leu Asp Asp Ser Phe Lys Thr Gln Phe Ala Ala Leu Gly
                   735                 740                 745
   GGG TGG GGT TTG CAT CAA CCA CCC CCT CAA ATA TTT TTA AAA ATA CTA      2064
   Gly Trp Gly Leu His Gln Pro Pro Pro Gln Ile Phe Leu Lys Ile Leu
               750                 755                 760
   CCA CAA AGT GGG CCA ATT GGA GGT ATT AAA TCC ATG GGA ATT ACT ACT      2112
   Pro Gln Ser Gly Pro Ile Gly Gly Ile Lys Ser Met Gly Ile Thr Thr
           765                 770                 775
   TTA GTT CAA TAT GCT GTG GGA ATA ATG ACA GTT ACC ATG ACC TTT AAA      2160
   Leu Val Gln Tyr Ala Val Gly Ile Met Thr Val Thr Met Thr Phe Lys
       780                 785                 790
   TTG GGA CCT CGA AAG GCT ACT GGA AGG TGG AAT CCC CAG CCT GGC GTT      2208
   Leu Gly Pro Arg Lys Ala Thr Gly Arg Trp Asn Pro Gln Pro Gly Val
   795                 800                 805                 810
   TAT CCT CCT CAT GCA GCT GGT CAT TTA CCA TAT GTA CTG TAT GAC CCC      2256
   Tyr Pro Pro His Ala Ala Gly His Leu Pro Tyr Val Leu Tyr Asp Pro
                   815                 820                 825
   ACA GCT ACA GAT GCA AAG CAA CAC CAC AGA CAC GGA TAT GAA AAG CCT      2304
   Thr Ala Thr Asp Ala Lys Gln His His Arg His Gly Tyr Glu Lys Pro
               830                 835                 840
   GAA GAA TTG TGG ACT GCC AAA AGC CGT GTG CAC CCA TTG                  2343
   Glu Glu Leu Trp Thr Ala Lys Ser Arg Val His Pro Leu
           845                 850                 855
<210> 86
   <211> 781
   <212> PRT
   <213> erythrovirus
<400> 86
   Met Ser Lys Thr Thr Asn Lys Trp Trp Glu Ser Ser Asp Lys Phe Ala
    1               5                  10                  15
   Gln Asp Val Tyr Lys Gln Phe Val Gln Phe Tyr Glu Lys Ala Thr Gly
               20                  25                  30
   Thr Asp Leu Glu Leu Ile Gln Ile Leu Lys Asp His Tyr Asn Ile Ser
           35                  40                  45
   Leu Asp Asn Pro Leu Glu Asn Pro Ser Ser Leu Phe Asp Leu Val Ala
       50                  55                  60
   Arg Ile Lys Ser Asn Leu Lys Asn Ser Pro Asp Leu Tyr Ser His His
   65                  70                  75                  80
   Phe Gln Ser His Gly Gln Leu Ser Asp His Pro His Ala Leu Ser Ser
                   85                  90                  95
   Ser Asn Ser Ser Ala Glu Pro Arg Gly Glu Asn Ala Val Leu Ser Ser
               100                 105                 110
   Glu Asp Leu His Lys Pro Gly Gln Val Ser Ile Gln Leu Pro Gly Thr
           115                 120                 125
   Asn Tyr Val Gly Pro Gly Asn Glu Leu Gln Ala Gly Pro Pro Gln Asn
       130                 135                 140
   Ala Val Asp Ser Ala Ala Arg Ile His Asp Phe Arg Tyr Ser Gln Leu
   145                 150                 155                 160
   Ala Lys Leu Gly Ile Asn Pro Tyr Thr His Trp Thr Val Ala Asp Glu
                   165                 170                 175
   Glu Leu Leu Lys Asn Ile Lys Asn Glu Thr Gly Phe Gln Ala Gln Ala
               180                 185                 190
   Val Lys Asp Tyr Phe Thr Leu Lys Gly Ala Ala Ala Pro Val Ala His
           195                 200                 205
   Phe Gln Gly Ser Leu Pro Glu Val Pro Ala Tyr Asn Ala Ser Glu Lys
       210                 215                 220
   Tyr Pro Ser Met Thr Ser Val Asn Ser Ala Glu Ala Ser Thr Gly Ala
   225                 230                 235                 240
   Gly Gly Gly Gly Ser Asn Pro Thr Lys Ser Met Trp Ser Glu Gly Ala
                   245                 250                 255
   Thr Phe Thr Ala Asn Ser Val Thr Cys Thr Phe Ser Arg Gln Phe Leu
               260                 265                 270
   Ile Pro Tyr Asp Pro Glu His His Tyr Lys Val Phe Ser Pro Ala Ala
           275                 280                 285
   Ser Ser Cys His Asn Ala Ser Gly Lys Glu Ala Lys Val Cys Thr Ile
       290                 295                 300
   Ser Pro Ile Met Gly Tyr Ser Thr Pro Trp Arg Tyr Leu Asp Phe Asn
   305                 310                 315                 320
   Ala Leu Asn Leu Phe Phe Ser Pro Leu Glu Phe Gln His Leu Ile Glu
                   325                 330                 335
   Asn Tyr Gly Ser Ile Ala Pro Asp Ala Leu Thr Val Thr Ile Ser Glu
               340                 345                 350
   Ile Ala Val Lys Asp Val Thr Asp Lys Thr Gly Gly Gly Val Gln Val
           355                 360                 365
   Thr Asp Ser Thr Thr Gly Arg Leu Cys Met Leu Val Asp His Glu Tyr
       370                 375                 380
   Lys Tyr Pro Tyr Val Leu Gly Gln Gly Gln Asp Thr Leu Ala Pro Glu
   385                 390                 395                 400
   Leu Pro Ile Trp Val Tyr Phe Pro Pro Gln Tyr Ala Tyr Leu Thr Val
                   405                 410                 415
   Gly Glu Val Asn Thr Gln Gly Ile Ser Gly Asp Ser Lys Lys Leu Ala
   420 425 430
   Ser Glu Glu Ser Ala Phe Tyr Val Leu Glu His Ser Ser Phe Glu Leu
   435 440 445
   Leu Gly Thr Gly Gly Ser Ala Thr Met Ser Tyr Lys Phe Pro Ala Val
   450 455 460
   Pro Pro Glu Asn Leu Glu Gly Cys Ser Gln His Phe Tyr Glu Met Tyr
   465 470 475 480
   Asn Pro Leu Tyr Gly Ser Arg Leu Gly Val Pro Asp Thr Leu Gly Gly
   485 490 495
   Asp Pro Lys Phe Arg Ser Leu Thr His Glu Asp His Ala Ile Gln Pro
   500 505 510
   Gln Asn Phe Met Pro Gly Pro Leu Ile Asn Ser Val Ser Thr Lys Glu
   515 520 525
   Gly Asp Asn Ser Asn Thr Gly Ala Gly Lys Ala Leu Thr Gly Leu Ser
   530 535 540
   Thr Gly Thr Ser Gln Asn Thr Arg Ile Ser Leu Arg Pro Gly Pro Val
   545 550 555 560
   Ser Gln Pro Tyr His His Trp Asp Thr Asp Lys Tyr Val Thr Gly Ile
   565 570 575
   Asn Ala Ile Ser His Gly Gln Thr Thr Tyr Gly Asn Ala Glu Asp Lys
   580 585 590
   Glu Tyr Gln Gln Gly Val Gly Arg Phe Pro Asn Glu Lys Glu Gln Leu
   595 600 605
   Lys Gln Leu Gln Gly Leu Asn Met His Thr Tyr Phe Pro Asn Lys Gly
   610 615 620
   Thr Gln Gln Tyr Thr Asp Gln Ile Glu Arg Pro Leu Met Val Gly Ser
   625 630 635 640
   Val Trp Asn Arg Arg Ala Leu His Tyr Glu Ser Gln Leu Trp Ser Lys
   645 650 655
   Ile Pro Asn Leu Asp Asp Ser Phe Lys Thr Gln Phe Ala Ala Leu Gly
   660 665 670
   Gly Trp Gly Leu His Gln Pro Pro Pro Gln Ile Phe Leu Lys Ile Leu
   675 680 685
   Pro Gln Ser Gly Pro Ile Gly Gly Ile Lys Ser Met Gly Ile Thr Thr
   690 695 700
   Leu Val Gln Tyr Ala Val Gly Ile Met Thr Val Thr Met Thr Phe Lys
   705 710 715 720
   Leu Gly Pro Arg Lys Ala Thr Gly Arg Trp Asn Pro Gln Pro Gly Val
   725 730 735
   Tyr Pro Pro His Ala Ala Gly His Leu Pro Tyr Val Leu Tyr Asp Pro
   740 745 750
   Thr Ala Thr Asp Ala Lys Gln His His Arg His Gly Tyr Glu Lys Pro
   755 760 765
   Glu Glu Leu Trp Thr Ala Lys Ser Arg Val His Pro Leu
   770 775 780
<210> 87
   <211> 681
   <212> ADN
   <213> erythrovirus
<400> 87
   ATG AGT AAA ACC ACT AAC AAA TGG TGG GAA AGC AGT GAC AAA TTT GCC        48
   Met Ser Lys Thr Thr Asn Lys Trp Trp Glu Ser Ser Asp Lys Phe Ala
               785                 790                 795
   CAG GAC GTG TAT AAG CAG TTT GTG CAA TTT TAT GAA AAA GCT ACT GGA        96
   Gln Asp Val Tyr Lys Gln Phe Val Gln Phe Tyr Glu Lys Ala Thr Gly
           800                 805                 810
   ACA GAC TTA GAG CTT ATT CAA ATT TTA AAA GAC CAT TAC AAC ATT TCT       144
   Thr Asp Leu Glu Leu Ile Gln Ile Leu Lys Asp His Tyr Asn Ile Ser
       815                 820                 825
   TTA GAT AAT CCT TTA GAA AAC CCC TCT TCT TTA TTT GAC TTA GTT GCT       192
   Leu Asp Asn Pro Leu Glu Asn Pro Ser Ser Leu Phe Asp Leu Val Ala
   830                 835                 840                 845
   CGC ATT AAA AGT AAT CTT AAA AAC TCT CCA GAC CTA TAT AGT CAT CAT       240
   Arg Ile Lys Ser Asn Leu Lys Asn Ser Pro Asp Leu Tyr Ser His His
                   850                 855                 860
   TTT CAG AGC CAT GGA CAG TTA TCT GAC CAC CCC CAT GCC TTA TCA TCC       288
   Phe Gln Ser His Gly Gln Leu Ser Asp His Pro His Ala Leu Ser Ser
               865                 870                 875
   AGT AAC AGT AGT GCA GAA CCT AGA GGA GAA AAT GCA GTA TTA TCT AGT       336
   Ser Asn Ser Ser Ala Glu Pro Arg Gly Glu Asn Ala Val Leu Ser Ser
           880                 885                 890
   GAA GAC TTA CAC AAG CCT GGG CAA GTT AGC ATA CAA TTA CCC GGT ACT       384
   Glu Asp Leu His Lys Pro Gly Gln Val Ser Ile Gln Leu Pro Gly Thr
       895                 900                 905
   AAC TAT GTT GGG CCT GGC AAT GAG CTA CAA GCT GGG CCT CCG CAG AAT       432
   Asn Tyr Val Gly Pro Gly Asn Glu Leu Gln Ala Gly Pro Pro Gln Asn
   910                 915                 920                 925
   GCT GTG GAC AGT GCT GCA AGG ATT CAT GAC TTT AGG TAT AGC CAA TTG       480
   Ala Val Asp Ser Ala Ala Arg Ile His Asp Phe Arg Tyr Ser Gln Leu
                   930                 935                 940
   GCT AAG TTG GGA ATA AAT CCT TAT ACA CAT TGG ACG GTA GCA GAT GAA       528
   Ala Lys Leu Gly Ile Asn Pro Tyr Thr His Trp Thr Val Ala Asp Glu
               945                 950                 955
   GAA TTG TTA AAA AAT ATA AAA AAT GAA ACA GGG TTT CAA GCA CAA GCA       576
   Glu Leu Leu Lys Asn Ile Lys Asn Glu Thr Gly Phe Gln Ala Gln Ala
           960                 965                 970
   GTA AAA GAT TAC TTT ACT TTA AAA GGT GCA GCT GCC CCT GTG GCC CAT       624
   Val Lys Asp Tyr Phe Thr Leu Lys Gly Ala Ala Ala Pro Val Ala His
       975                 980                 985
   TTT CAA GGA AGT TTA CCG GAA GTG CCC GCG TAC AAC GCC TCA GAA AAA       672
   Phe Gln Gly Ser Leu Pro Glu Val Pro Ala Tyr Asn Ala Ser Glu Lys
   990                 995                 1000                1005
   TAC CCC AGC                                                           681
   Tyr Pro Ser
<210> 88
   <211> 227
   <212> PRT
   <213> erythrovirus
<400> 88
   Met Ser Lys Thr Thr Asn Lys Trp Trp Glu Ser Ser Asp Lys Phe Ala
   1               5                   10                  15
   Gln Asp Val Tyr Lys Gln Phe Val Gln Phe Tyr Glu Lys Ala Thr Gly
               20                  25                  30
   Thr Asp Leu Glu Leu Ile Gln Ile Leu Lys Asp His Tyr Asn Ile Ser
           35                  40                  45
   Leu Asp Asn Pro Leu Glu Asn Pro Ser Ser Leu Phe Asp Leu Val Ala
       50                  55                  60
   Arg Ile Lys Ser Asn Leu Lys Asn Ser Pro Asp Leu Tyr Ser His His
   65                  70                  75                  80
   Phe Gln Ser His Gly Gln Leu Ser Asp His Pro His Ala Leu Ser Ser
                   85                  90                  95
   Ser Asn Ser Ser Ala Glu Pro Arg Gly Glu Asn Ala Val Leu Ser Ser
               100                 105                 110
   Glu Asp Leu His Lys Pro Gly Gln Val Ser Ile Gln Leu Pro Gly Thr
           115                 120                 125
   Asn Tyr Val Gly Pro Gly Asn Glu Leu Gln Ala Gly Pro Pro Gln Asn
       130                 135                 140
   Ala Val Asp Ser Ala Ala Arg Ile His Asp Phe Arg Tyr Ser Gln Leu
   145                 150                 155                 160
   Ala Lys Leu Gly Ile Asn Pro Tyr Thr His Trp Thr Val Ala Asp Glu
                   165                 170                 175
   Glu Leu Leu Lys Asn Ile Lys Asn Glu Thr Gly Phe Gln Ala Gln Ala
               180                 185                 190
   Val Lys Asp Tyr Phe Thr Leu Lys Gly Ala Ala Ala Pro Val Ala His
           195                 200                 205
   Phe Gln Gly Ser Leu Pro Glu Val Pro Ala Tyr Asn Ala Ser Glu Lys
       210                 215                 220
   Tyr Pro Ser
   225
<210> 89
   <211> 306
   <212> ADN
   <213> erythrovirus
<400> 89
   TTG CTC GCA TTA AAA GTA ATC TTA AAA ACT CTC CAG ACC TAT ATA GTC      48
   Leu Leu Ala Leu Lys Val Ile Leu Lys Thr Leu Gln Thr Tyr Ile Val
           230                 235                 240
   ATC ATT TTC AGA GCC ATG GAC AGT TAT CTG ACC ACC CCC ATG CCT TAT      96
   Ile Ile Phe Arg Ala Met Asp Ser Tyr Leu Thr Thr Pro Met Pro Tyr
       245                 250                 255
   CAT CCA GTA ACA GTA GTG CAG AAC CTA GAG GAG AAA ATG CAG TAT TAT        144
   His Pro Val Thr Val Val Gln Asn Leu Glu Glu Lys Met Gln Tyr Tyr
   260                 265                 270                 275
   CTA GTG AAG ACT TAC ACA AGC CTG GGC AAG TTA GCA TAC AAT TAC CCG        192
   Leu Val Lys Thr Tyr Thr Ser Leu Gly Lys Leu Ala Tyr Asn Tyr Pro
                   280                 285                 290
   GTA CTA ACT ATG TTG GGC CTG GCA ATG AGC TAC AAG CTG GGC CTC CGC        240
   Val Leu Thr Met Leu Gly Leu Ala Met Ser Tyr Lys Leu Gly Leu Arg
               295                 300                 305
   AGA ATG CTG TGG ACA GTG CTG CAA GGA TTC ATG ACT TTA GGT ATA GCC        288
   Arg Met Leu Trp Thr Val Leu Gln Gly Phe Met Thr Leu Gly Ile Ala
           310                 315                 320
   AAT TGG CTA AGT TGG GAA                                                306
   Asn Trp Leu Ser Trp Glu
       325
<210> 90
   <211> 102
   <212> PRT
   <213> erythrovirus
<400> 90
   Leu Leu Ala Leu Lys Val Ile Leu Lys Thr Leu Gln Thr Tyr Ile Val
   1               5                   10                  15
   Ile Ile Phe Arg Ala Met Asp Ser Tyr Leu Thr Thr Pro Met Pro Tyr
               20                  25                  30
   His Pro Val Thr Val Val Gln Asn Leu Glu Glu Lys Met Gln Tyr Tyr
           35                  40                  45
   Leu Val Lys Thr Tyr Thr Ser Leu Gly Lys Leu Ala Tyr Asn Tyr Pro
       50                  55                  60
   Val Leu Thr Met Leu Gly Leu Ala Met Ser Tyr Lys Leu Gly Leu Arg
   65                  70                  75                  80
   Arg Met Leu Trp Thr Val Leu Gln Gly Phe Met Thr Leu Gly Ile Ala
                   85                  90                  95
   Asn Trp Leu Ser Trp Glu
               100
<210> 91
   <211> 1662
   <212> ADN
   <213> erythrovirus
<400> 91
   ATG ACT TCA GTT AAC TCT GCA GAA GCC AGC ACT GGT GCA GGC GGG GGA        48
   Met Thr Ser Val Asn Ser Ala Glu Ala Ser Thr Gly Ala Gly Gly Gly
           105                 110                 115
   GGT AGC AAC CCT ACA AAA AGC ATG TGG AGT GAA GGG GCT ACA TTT ACT        96
   Gly Ser Asn Pro Thr Lys Ser Met Trp Ser Glu Gly Ala Thr Phe Thr
       120                 125                 130
   GCT AAT TCT GTA ACG TGT ACA TTC TCT AGG CAA TTT TTA ATT CCA TAT        144
   Ala Asn Ser Val Thr Cys Thr Phe Ser Arg Gln Phe Leu Ile Pro Tyr
   135                 140                 145                 150
   GAT CCA GAG CAT CAT TAT AAA GTG TTC TCT CCA GCA GCT AGT AGC TGC        192
   Asp Pro Glu His His Tyr Lys Val Phe Ser Pro Ala Ala Ser Ser Cys
                   155                 160                 165
   CAC AAT GCT AGT GGG AAA GAG GCA AAA GTG TGC ACT ATT AGT CCC ATT        240
   His Asn Ala Ser Gly Lys Glu Ala Lys Val Cys Thr Ile Ser Pro Ile
               170                 175                 180
   ATG GGG TAC TCT ACT CCG TGG AGA TAC TTA GAT TTT AAT GCT TTA AAT        288
   Met Gly Tyr Ser Thr Pro Trp Arg Tyr Leu Asp Phe Asn Ala Leu Asn
           185                 190                 195
   TTG TTT TTC TCA CCA TTA GAG TTT CAG CAC TTA ATT GAA AAT TAT GGT        336
   Leu Phe Phe Ser Pro Leu Glu Phe Gln His Leu Ile Glu Asn Tyr Gly
       200                 205                 210
   AGT ATA GCT CCA GAT GCT TTA ACT GTA ACT ATT TCA GAA ATT GCT GTA        384
   Ser Ile Ala Pro Asp Ala Leu Thr Val Thr Ile Ser Glu Ile Ala Val
   215                 220                 225                 230
   AAA GAT GTC ACA GAC AAA ACA GGA GGA GGT GTG CAA GTT ACT GAC AGC        432
   Lys Asp Val Thr Asp Lys Thr Gly Gly Gly Val Gln Val Thr Asp Ser
                   235                 240                 245
   ACC ACA GGA CGT TTG TGT ATG TTA GTG GAT CAT GAG TAT AAA TAC CCA        480
   Thr Thr Gly Arg Leu Cys Met Leu Val Asp His Glu Tyr Lys Tyr Pro
               250                 255                 260
   TAT GTG CTA GGT CAG GGA CAA GAC ACA CTA GCT CCA GAA CTG CCC ATT        528
   Tyr Val Leu Gly Gln Gly Gln Asp Thr Leu Ala Pro Glu Leu Pro Ile
           265                 270                 275
   TGG GTT TAC TTT CCC CCC CAG TAT GCT TAC TTA ACA GTA GGT GAA GTA        576
   Trp Val Tyr Phe Pro Pro Gln Tyr Ala Tyr Leu Thr Val Gly Glu Val
       280                 285                 290
   AAC ACA CAA GGA ATT TCA GGA GAC AGC AAA AAA TTG GCT AGT GAA GAA        624
   Asn Thr Gln Gly Ile Ser Gly Asp Ser Lys Lys Leu Ala Ser Glu Glu
   295                 300                 305                 310
   TCA GCT TTT TAT GTG TTA GAG CAC AGT TCA TTT GAA CTT TTG GGT ACA        672
   Ser Ala Phe Tyr Val Leu Glu His Ser Ser Phe Glu Leu Leu Gly Thr
                   315                 320                 325
   GGG GGA TCT GCC ACT ATG TCC TAC AAA TTT CCA GCT GTG CCC CCA GAA        720
   Gly Gly Ser Ala Thr Met Ser Tyr Lys Phe Pro Ala Val Pro Pro Glu
               330                 335                 340
   AAC CTA GAA GGC TGC AGC CAA CAT TTT TAT GAA ATG TAC AAC CCT TTG        768
   Asn Leu Glu Gly Cys Ser Gln His Phe Tyr Glu Met Tyr Asn Pro Leu
           345                 350                 355
   TAC GGT TCT CGT TTA GGG GTA CCT GAC ACA TTA GGA GGG GAC CCT AAA        816
   Tyr Gly Ser Arg Leu Gly Val Pro Asp Thr Leu Gly Gly Asp Pro Lys
       360                 365                 370
   TTT AGA TCA TTG ACA CAC GAA GAC CAC GCA ATT CAG CCA CAA AAC TTT        864
   Phe Arg Ser Leu Thr His Glu Asp His Ala Ile Gln Pro Gln Asn Phe
   375                 380                 385                 390
   ATG CCT GGG CCA CTA ATA AAT TCA GTG TCT ACC AAA GAA GGA GAC AAT        912
   Met Pro Gly Pro Leu Ile Asn Ser Val Ser Thr Lys Glu Gly Asp Asn
                    395                 400                 405
   TCT AAT ACA GGT GCT GGA AAA GCC CTT ACG GGG CTT AGT ACT GGC ACT      960
   Ser Asn Thr Gly Ala Gly Lys Ala Leu Thr Gly Leu Ser Thr Gly Thr
                410                 415                 420
   AGC CAA AAC ACC AGA ATT TCC CTA CGC CCC GGG CCA GTA TCT CAG CCA     1008
   Ser Gln Asn Thr Arg Ile Ser Leu Arg Pro Gly Pro Val Ser Gln Pro
           425                 430                 435
   TAC CAT CAC TGG GAC ACT GAT AAA TAT GTT ACA GGA ATA AAT GCC ATT     1056
   Tyr His His Trp Asp Thr Asp Lys Tyr Val Thr Gly Ile Asn Ala Ile
       440                 445                 450
   TCA CAT GGA CAA ACC ACT TAT GGA AAT GCT GAG GAC AAA GAG TAT CAG     1104
   Ser His Gly Gln Thr Thr Tyr Gly Asn Ala Glu Asp Lys Glu Tyr Gln
   455                 460                 465                 470
   CAA GGG GTA GGA AGA TTT CCA AAT GAA AAA GAA CAG CTT AAG CAG TTA     1152
   Gln Gly Val Gly Arg Phe Pro Asn Glu Lys Glu Gln Leu Lys Gln Leu
                   475                 480                 485
   CAA GGT CTT AAC ATG CAC ACA TAC TTC CCT AAT AAA GGA ACC CAA CAA     1200
   Gln Gly Leu Asn Met His Thr Tyr Phe Pro Asn Lys Gly Thr Gln Gln
               490                 495                 500
   TAC ACA GAC CAA ATT GAA CGC CCT CTT ATG GTG GGC TCT GTT TGG AAC     1248
   Tyr Thr Asp Gln Ile Glu Arg Pro Leu Met Val Gly Ser Val Trp Asn
           505                 510                 515
   AGA AGA GCT CTT CAC TAT GAA AGT CAG CTG TGG AGT AAA ATC CCT AAC     1296
   Arg Arg Ala Leu His Tyr Glu Ser Gln Leu Trp Ser Lys Ile Pro Asn
       520                 525                 530
   TTA GAT GAC AGT TTT AAA ACT CAA TTT GCA GCC CTA GGC GGG TGG GGT     1344
   Leu Asp Asp Ser Phe Lys Thr Gln Phe Ala Ala Leu Gly Gly Trp Gly
   535                 540                 545                 550
   TTG CAT CAA CCA CCC CCT CAA ATA TTT TTA AAA ATA CTA CCA CAA AGT     1392
   Leu His Gln Pro Pro Pro Gln Ile Phe Leu Lys Ile Leu Pro Gln Ser
                   555                 560                 565
   GGG CCA ATT GGA GGT ATT AAA TCC ATG GGA ATT ACT ACT TTA GTT CAA     1440
   Gly Pro Ile Gly Gly Ile Lys Ser Met Gly Ile Thr Thr Leu Val Gln
               570                 575                 580
   TAT GCT GTG GGA ATA ATG ACA GTT ACC ATG ACC TTT AAA TTG GGA CCT     1488
   Tyr Ala Val Gly Ile Met Thr Val Thr Met Thr Phe Lys Leu Gly Pro
           585                 590                 595
   CGA AAG GCT ACT GGA AGG TGG AAT CCC CAG CCT GGC GTT TAT CCT CCT     1536
   Arg Lys Ala Thr Gly Arg Trp Asn Pro Gln Pro Gly Val Tyr Pro Pro
       600                 605                 610
   CAT GCA GCT GGT CAT TTA CCA TAT GTA CTG TAT GAC CCC ACA GCT ACA     1584
   His Ala Ala Gly His Leu Pro Tyr Val Leu Tyr Asp Pro Thr Ala Thr
   615                 620                 625                 630
   GAT GCA AAG CAA CAC CAC AGA CAC GGA TAT GAA AAG CCT GAA GAA TTG     1632
   Asp Ala Lys Gln His His Arg His Gly Tyr Glu Lys Pro Glu Glu Leu
                   635                 640                 645
   TGG ACT GCC AAA AGC CGT GTG CAC CCA TTG                             1662
   Trp Thr Ala Lys Ser Arg Val His Pro Leu
               650                 655
<210> 92
   <211> 554
   <212> PRT
   <213> erythrovirus
<400> 92
   Met Thr Ser Val Asn Ser Ala Glu Ala Ser Thr Gly Ala Gly Gly Gly
    1               5                  10                  15
   Gly Ser Asn Pro Thr Lys Ser Met Trp Ser Glu Gly Ala Thr Phe Thr
               20                  25                  30
   Ala Asn Ser Val Thr Cys Thr Phe Ser Arg Gln Phe Leu Ile Pro Tyr
           35                  40                  45
   Asp Pro Glu His His Tyr Lys Val Phe Ser Pro Ala Ala Ser Ser Cys
       50                  55                  60
   His Asn Ala Ser Gly Lys Glu Ala Lys Val Cys Thr Ile Ser Pro Ile
   65                  70                  75                  80
   Met Gly Tyr Ser Thr Pro Trp Arg Tyr Leu Asp Phe Asn Ala Leu Asn
                   85                  90                  95
   Leu Phe Phe Ser Pro Leu Glu Phe Gln His Leu Ile Glu Asn Tyr Gly
               100                 105                 110
   Ser Ile Ala Pro Asp Ala Leu Thr Val Thr Ile Ser Glu Ile Ala Val
           115                 120                 125
   Lys Asp Val Thr Asp Lys Thr Gly Gly Gly Val Gln Val Thr Asp Ser
       130                 135                 140
   Thr Thr Gly Arg Leu Cys Met Leu Val Asp His Glu Tyr Lys Tyr Pro
   145                 150                 155                 160
   Tyr Val Leu Gly Gln Gly Gln Asp Thr Leu Ala Pro Glu Leu Pro Ile
                   165                 170                 175
   Trp Val Tyr Phe Pro Pro Gln Tyr Ala Tyr Leu Thr Val Gly Glu Val
               180                 185                 190
   Asn Thr Gln Gly Ile Ser Gly Asp Ser Lys Lys Leu Ala Ser Glu Glu
           195                 200                 205
   Ser Ala Phe Tyr Val Leu Glu His Ser Ser Phe Glu Leu Leu Gly Thr
       210                 215                 220
   Gly Gly Ser Ala Thr Met Ser Tyr Lys Phe Pro Ala Val Pro Pro Glu
   225                 230                 235                 240
   Asn Leu Glu Gly Cys Ser Gln His Phe Tyr Glu Met Tyr Asn Pro Leu
                   245                 250                 255
   Tyr Gly Ser Arg Leu Gly Val Pro Asp Thr Leu Gly Gly Asp Pro Lys
               260                 265                 270
   Phe Arg Ser Leu Thr His Glu Asp His Ala Ile Gln Pro Gln Asn Phe
           275                 280                 285
   Met Pro Gly Pro Leu Ile Asn Ser Val Ser Thr Lys Glu Gly Asp Asn
       290                 295                 300
   Ser Asn Thr Gly Ala Gly Lys Ala Leu Thr Gly Leu Ser Thr Gly Thr
   305                 310                 315                 320
   Ser Gln Asn Thr Arg Ile Ser Leu Arg Pro Gly Pro Val Ser Gln Pro
                   325                 330                 335
   Tyr His His Trp Asp Thr Asp Lys Tyr Val Thr Gly Ile Asn Ala Ile
               340                 345                 350
   Ser His Gly Gln Thr Thr Tyr Gly Asn Ala Glu Asp Lys Glu Tyr Gln
           355                 360                 365
   Gln Gly Val Gly Arg Phe Pro Asn Glu Lys Glu Gln Leu Lys Gln Leu
       370                 375                 380
   Gln Gly Leu Asn Met His Thr Tyr Phe Pro Asn Lys Gly Thr Gln Gln
   385                 390                 395                 400
   Tyr Thr Asp Gln Ile Glu Arg Pro Leu Met Val Gly Ser Val Trp Asn
                   405                 410                 415
   Arg Arg Ala Leu His Tyr Glu Ser Gln Leu Trp Ser Lys Ile Pro Asn
               420                 425                 430
   Leu Asp Asp Ser Phe Lys Thr Gln Phe Ala Ala Leu Gly Gly Trp Gly
           435                 440                 445
   Leu His Gln Pro Pro Pro Gln Ile Phe Leu Lys Ile Leu Pro Gln Ser
       450                 455                 460
   Gly Pro Ile Gly Gly Ile Lys Ser Met Gly Ile Thr Thr Leu Val Gln
   465                 470                 475                 480
   Tyr Ala Val Gly Ile Met Thr Val Thr Met Thr Phe Lys Leu Gly Pro
                   485                 490                 495
   Arg Lys Ala Thr Gly Arg Trp Asn Pro Gln Pro Gly Val Tyr Pro Pro
               500                 505                 510
   His Ala Ala Gly His Leu Pro Tyr Val Leu Tyr Asp Pro Thr Ala Thr
           515                 520                 525
   Asp Ala Lys Gln His His Arg His Gly Tyr Glu Lys Pro Glu Glu Leu
       530                 535                 540
   Trp Thr Ala Lys Ser Arg Val His Pro Leu
   545                 550
<210> 93
   <211> 396
   <212> ADN
   <213> erythrovirus
<400> 93
   CCT TTA AAT TGG GAC CTC GAA AGG CTA CTG GAA GGT GGA ATC CCC AGC     48
   Pro Leu Asn Trp Asp Leu Glu Arg Leu Leu Glu Gly Gly Ile Pro Ser
   555                 560                 565                 570
   CTG GCG TTT ATC CTC CTC ATG CAG CTG GTC ATT TAC CAT ATG TAC TGT     96
   Leu Ala Phe Ile Leu Leu Met Gln Leu Val Ile Tyr His Met Tyr Cys
                   575                 580                 585
   ATG ACC CCA CAG CTA CAG ATG CAA AGC AAC ACC ACA GAC ACG GAT ATG    144
   Met Thr Pro Gln Leu Gln Met Gln Ser Asn Thr Thr Asp Thr Asp Met
               590                 595                 600
   AAA AGC CTG AAG AAT TGT GGA CTG CCA AAA GCC GTG TGC ACC CAT TGT    192
   Lys Ser Leu Lys Asn Cys Gly Leu Pro Lys Ala Val Cys Thr His Cys
           605                 610                 615
   AAA CAT TCC CCA CCG TGT CCT CAG CCA GGA ACC GTC ACC CAC CGC CCA    240
   Lys His Ser Pro Pro Cys Pro Gln Pro Gly Thr Val Thr His Arg Pro
       620                 625                 630
   CCT GTG CCG CCC AGA TTA TAT GTG CCC CCT CCA ATA CCC CGT AGG CAA    288
   Pro Val Pro Pro Arg Leu Tyr Val Pro Pro Pro Ile Pro Arg Arg Gln
   635                 640                 645                 650
   CCA TCT ATA AAA GAT ACA GAC GCT GTA GAA TAT AAA TTA TTA ACT AGA    336
   Pro Ser Ile Lys Asp Thr Asp Ala Val Glu Tyr Lys Leu Leu Thr Arg
                   655                 660                 665
   TAT GAA CAA CAT GTA ATT AGA ATG CTA AGA TTA TGT AAT ATG TAC ACA    384
   Tyr Glu Gln His Val Ile Arg Met Leu Arg Leu Cys Asn Met Tyr Thr
               670                 675                 680
   AGT TTG GAA AAA                                                    396
   Ser Leu Glu Lys
           685
<210> 94
   <211> 132
   <212> PRT
   <213> erythrovirus
<400> 94
   Pro Leu Asn Trp Asp Leu Glu Arg Leu Leu Glu Gly Gly Ile Pro Ser
   1 5 10 15
   Leu Ala Phe Ile Leu Leu Met Gln Leu Val Ile Tyr His Met Tyr Cys
   20 25 30
   Met Thr Pro Gln Leu Gln Met Gln Ser Asn Thr Thr Asp Thr Asp Met
   35 40 45
   Lys Ser Leu Lys Asn Cys Gly Leu Pro Lys Ala Val Cys Thr His Cys
   50 55 60
   Lys His Ser Pro Pro Cys Pro Gln Pro Gly Thr Val Thr His Arg Pro
   65 70 75 80
   Pro Val Pro Pro Arg Leu Tyr Val Pro Pro Pro Ile Pro Arg Arg Gln
   85 90 95
   Pro Ser Ile Lys Asp Thr Asp Ala Val Glu Tyr Lys Leu Leu Thr Arg
   100 105 110
   Tyr Glu Gln His Val Ile Arg Met Leu Arg Leu Cys Asn Met Tyr Thr
   115 120 125
   Ser Leu Glu Lys
   130
<210> 95
   <211> 40
   <212> PRT
   <213> erythrovirus
<400> 95
   Met Ser Lys Thr Thr Asn Lys Trp Trp Glu Ser Ser Asp Lys Phe Ala
   1 5 10 15
   Gln Asp Val Tyr Lys Gln Phe Val Gln Phe Tyr Glu Lys Ala Thr Gly
   20 25 30
   Thr Asp Leu Glu Leu Ile Gln Ile
   35 40
<210> 96
   <211> 21
   <212> PRT
   <213> erythrovirus
<400> 96
   Ser Leu Phe Asp Leu Val Ala Arg Ile Lys Ser Asn Leu Lys Asn Ser
   1 5 10 15
   Pro Asp Leu Tyr Ser
   20
<210> 97
   <211> 24
   <212> PRT
   <213> erythrovirus
<400> 97
   Leu Ser Asp His Pro His Ala Leu Ser Ser Ser Asn Ser Ser Ala Glu
   1 5 10 15
   Pro Arg Gly Glu Asn Ala Val Leu
   20
<210> 98
   <211> 21
   <212> PRT
   <213> erythrovirus
<400> 98
   Glu Asp Leu His Lys Pro Gly Gln Val Ser Ile Gln Leu Pro Gly Thr
   1 5 10 15
   Asn Tyr Val Gly Pro
   20
<210> 99
   <211> 22
   <212> PRT
   <213> erythrovirus
<400> 99
   Gly Asn Glu Leu Gln Ala Gly Pro Pro Gln Asn Ala Val Asp Ser Ala
   1 5 10 15
   Ala Arg Ile His Asp Phe
   20
<210> 100
   <211> 21
   <212> PRT
   <213> erythrovirus
<400> 100
   Ile Lys Asn Glu Thr Gly Phe Gln Ala Gln Ala Val Lys Asp Tyr Phe
   1 5 10 15
   Thr Leu Lys Gly Ala
   20
<210> 101
   <211> 32
   <212> PRT
   <213> erythrovirus
<400> 101
   Ser Thr Gly Ala Gly Gly Gly Gly Ser Asn Pro Thr Lys Ser Met Trp
   1 5 10 15
   Ser Glu Gly Ala Thr Phe Thr Ala Asn Ser Val Thr Cys Thr Phe Ser
   20 25 30
<210> 102
   <211> 21
   <212> PRT
   <213> erythrovirus
<400> 102
   Pro Pro Gln Tyr Ala Tyr Leu Thr Val Gly Glu Val Asn Thr Gln Gly
   1 5 10 15
   Ile Ser Gly Asp Ser
   20
<210> 103
   <211> 37
   <212> PRT
   <213> erythrovirus
<400> 103
   Ala Phe Tyr Val Leu Glu His Ser Ser Phe Glu Leu Leu Gly Thr Gly
   1 5 10 15
   Gly Ser Ala Thr Met Ser Tyr Lys Phe Pro Ala Val Pro Pro Glu Asn
   20 25 30
   Leu Glu Gly Cys Ser
   35
<210> 104
   <211> 28
   <212> PRT
   <213> erythrovirus
<400> 104
   Gln Asn Phe Met Pro Gly Pro Leu Ile Asn Ser Val Ser Thr Lys Glu
   1 5 10 15
   Gly Asp Asn Ser Asn Thr Gly Ala Gly Lys Ala Leu
   20 25
<210> 105
   <211> 19
   <212> ADN
   <213> erythrovirus
<400> 105
   TGCAGATGCC CTCCACCCA 19
<210> 106
   <211> 21
   <212> ADN
   <213> erythrovirus
<400> 106
   GCTGCTTTCA CTGAGTTCTT C 21
<210> 107
   <211> 20
   <212> ADN
   <213> erythrovirus
<400> 107
   GACCAGTTCA GGAGAATCAT 20
<210> 108
   <211> 19
   <212> ADN
   <213> erythrovirus
<400> 108
   ATCGGCAAGC GGCGTGTAA 19
<210> 109
   <211> 20
   <212> ADN
   <213> erythrovirus
<400> 109
   ATCCAGACAG GTAAGCACAT 20
<210> 110
   <211> 21
   <212> ADN
   <213> erythrovirus
<400> 110 21
   ATCGGCAAGC GGCGTGTAAA A 21
<210> 111
   <211> 19
   <212> ADN
   <213> erythrovirus
<400> 111 19
   CATGCCTTAT CATCCAGTA 19
<210> 112
   <211> 20
   <212> ADN
   <213> erythrovirus
<400> 112 20
   TTGGCTATAC CTAAAGTCAT 20
<210> 113
   <211> 19
   <212> ADN
   <213> erythrovirus
<400> 113
   CACTATGAAA ACTGGGCAA 19
<210> 114
   <211> 19
   <212> ADN
   <213> erythrovirus
<400> 114 19
   ACAATTCTTC ATCTGCTAC 19
<210> 115
   <211> 21
   <212> ADN
   <213> erythrovirus
<400> 115
   AAACTGGGCA ATAAACTACA C 21
<210> 116
   <211> 20
   <212> ADN
   <213> erythrovirus
<400> 116
   CTTCATCTGC TACCGTCCAA 20
<210> 117
   <211> 33
   <212> ADN
   <213> erythrovirus
<400> 117
   AAAGGCCTAG ATCTTGTAGA TTATGAGTAA AAC 33
<210> 118
   <211> 26
   <212> ADN
   <213> erythrovirus
<400> 118
   CGGAATTCGG TGGGTGACGG TTCCTG 26
<210> 119
   <211> 29
   <212> ADN
   <213> erythrovirus
<400> 119
   CACGGATCCA TACCCCAGCA TGACTTCAG 29
<210> 120
   <211> 26
   <212> ADN
   <213> erythrovirus
<400> 120
   CACGGATCCG GTGGGTGACG GTTCCTG 26
<210> 121
   <211> 36
   <212> ADN
   <213> erythrovirus
<400> 121
   ACCAGTATCA GCAGCAGTGG TGGTGAAAGC TCTGAA 36